(19) Europäisches Patentamt
European Patent Office
Office européen des brevets

(11) Numéro de publication: **0 442 820 B1**

# (12) FASCICULE DE BREVET EUROPEEN

(45) Date de publication du fascicule du brevet: **13.09.95**

(21) Numéro de dépôt: **91400393.4**

(22) Date de dépôt: **15.02.91**

(51) Int. Cl.[6]: **C07D 235/16**, A61K 31/415, C07D 235/28, C07D 471/04, C07F 9/6506, C07F 9/6561, A61K 31/44

## (54) Nouveaux dérivés de benzimidazole et d'azabenzimidazole, antagonistes des récepteurs au thromboxane; leurs procédés de préparation, intermédiaires de synthèse, compositions les contenant.

(30) Priorité: **16.02.90 FR 9001925**

(43) Date de publication de la demande:
**21.08.91 Bulletin 91/34**

(45) Mention de la délivrance du brevet:
**13.09.95 Bulletin 95/37**

(84) Etats contractants désignés:
**AT BE CH DE DK ES FR GB GR IT LI LU NL SE**

(56) Documents cités:
**EP-A- 0 178 413**
**DE-B- 1 131 688**
**FR-A- 1 580 823**
**US-A- 4 880 804**

**CHEMICAL ABSTRACTS, vol. 76, no. 19, 8 mai 1972, page 10, colonne 1, résumé no. 107834w, Columbus, Ohio, US; W. JUNGSTAND et al.: "Effect of different benzimidazole nitrogen mustards upon transplantation-tumors"**

(73) Titulaire: **LABORATOIRES UPSA**
**1 bis, rue du Docteur Camille Bru**
**F-47000 Agen (FR)**

(72) Inventeur: **Bru-Magniez, Nicole**
**24-26, Avenue Raphael**
**F-75016 Paris (FR)**
Inventeur: **Nicolai, Eric**
**52, Rue St. Ouen,**
**Rce Le Hameau BtC**
**F-14000 Caen (FR)**
Inventeur: **Teulon, Jean-Marie**
**13, Avenue Guibert**
**F-78170 La Celle St Cloud (FR)**

(74) Mandataire: **Portal, Gérard et al**
**Cabinet Beau de Loménie**
**158, rue de l'Université**
**F-75340 Paris Cédex 07 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

**CHEMICAL ABSTRACTS**, vol. 76, no. 13, 27 mars 1972, page 377, colonne 1, résuméno. 72453s, Columbus, Ohio, US; W. OZEGOWSKI et al.: "Gamma-[1-methyl-5-[bis(beta-chloroethyl)-amino]-2-benzimidazolyl]butyricacid hydrochloride, a new cytostatic agent of the group of b.n. mustards"

**CHEMICAL ABSTRACTS**, vol. 69, no. 23, 2 décembre 1968, page 9039, colonne 2, résumé no. 96581p, Columbus, Ohio, US; A.M. SIMONOV et al.: "Cleavage of the furannucleus in some 2-furylbenzimidazoles"

## Description

La présente invention concerne en tant que produits nouveaux, les dérivés de benzimidazole et d'azabenzimidazole de formule générale (I) ci-dessous ainsi que leurs sels.

Les composés en question présentent un profil pharmacologique très intéressant dans la mesure où ils sont doués de propriétés antagonistes des récepteurs au thromboxane. Le thromboxane $A_2$ (ou $TXA_2$) intervient au niveau de divers tissus ou cellules. Une action constrictive est observée sur la musculature lisse vasculaire, bronchique et utérine. Les plaquettes sanguines sont agrégées par le $TXA_2$ tandis que les membranes des cellules circulantes sont modifiées et peuvent ainsi adhérer entre elles. Les différentes propriétés décrites pour le thromboxane $A_2$ permettent d'envisager un rôle favorable d'un antagoniste des récepteurs au $TXA_2$ dans les pathologies suivantes : infarctus du myocarde, angine de poitrine, ictus cérébral, migraine, hémorragie cérébrale, athérosclérose, embolie pulmonaire, asthme bronchique, bronchite, pneumonie, choc circulatoire d'origines diverses (hémorragie, septicémie, défaillance cardiaque, traumatique, pancréatite aigüe, brûlure, bactérienne), néphrite, rejet de greffe, métastases cancéreuses.

La présente invention concerne également le procédé de préparation des dits produits et leurs applications en thérapeutique. Elle concerne en outre les composés intermédiaires nouveaux permettant la synthèse des dits produits.

Ces dérivés de benzimidazole et d'azabenzimidazole sont caractérisés en ce qu'ils répondent à la formule générale (I) :

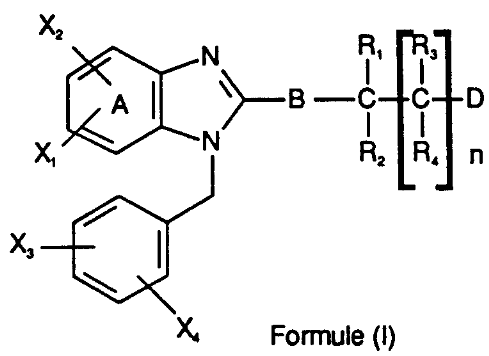

Formule (I)

dans laquelle :

A est un noyau aromatique ou un hétérocycle azoté,

$X_1$, $X_2$, $X_3$ et $X_4$ représentent indépendamment un atome d'hydrogène, un atome d'halogène, un radical alkyle inférieur, un radical cyclo alkyle inférieur en $C_3$-$C_7$, un radical alkoxy, un radical alkylthio, un groupe sulfone, $SO_2$ alkyle inférieur, un groupe sulfoxyde, SO alkyle inférieur, un groupe trifluorométhyle, un groupe nitro, un groupe hydroxyle, un radical méthylène alcool ou une fonction COOR' où R' est un hydrogène ou un alkyle inférieur, $X_3$ et $X_4$ peuvent également former avec le phényle un naphtalène,

B représente $CR_5R_6$, $R_5$ et $R_6$ étant un atome d'hydrogène ou un alkyle inférieur, ou un radical cycloalkyle en $C_3$-$C_7$, ou l'atome de soufre,

$R_1$, $R_2$, $R_3$ et $R_4$ représentent indépendamment un atome d'hydrogène ou un radical alkyle inférieur, ou un radical cyclo alkyle en $C_3$-$C_7$, $CR_1R_2$ ou $CR_3R_4$ peuvent former avec B lorsque ce dernier représente $CR_5R_6$ un cycloalkyle ou un cycloalkène de 3 à 7 atomes de carbone, $R_1R_2$, $R_3R_4$ peuvent également former un cycle de 3 à 7 atomes de carbones,

n est un nombre entier de 1 à 4 et peut être 0 si $R_1$ et $R_2$ sont différents de l'hydrogène,

D représente une fonction chimique qui peut être :

$COOR_7$, $R_7$ étant l'atome d'hydrogène ou un radical alkyle inférieur ou un radical cyclo alkyle en $C_3$-$C_7$
CONH-$R_8$, $R_8$ étant l'atome d'hydrogène ou un radical alkyle inférieur ou un radical cyclo alkyle en $C_3$-$C_7$
CN.

Dans la description et les revendications, on entend par radical alkyle inférieur une chaîne hydrocarbonée de 1 à 6 atomes de carbone linéaire ou ramifiée. Un radical alkyle inférieur est par exemple un radical méthyle, éthyle, propyle, isopropyle, butyle, isobutyle, tertiobutyle, pentyle, isopentyle, hexyle ou isohexyle.

Par radical cycloalkyle en $C_3$-$C_7$, on entend un radical cyclique saturé, il s'agit de préférence d'un radical cyclopropane, cyclobutane, cyclopentane, cyclohexane ou cycloheptane.

Par radical cycloalkène en $C_3$-$C_7$, on entend un radical cyclique possédant une insaturation, il s'agit de préférence d'un radical cyclobutène, cyclopentène, cyclohexène ou cycloheptène.

Par radical alkoxy on entend un groupement O-alkyle inférieur et par radical alkyl thio un groupement S-alkyle inférieur, alkyle inférieur étant défini comme ci-dessus.

Dans la description et les revendications, on entend par halogène un atome de chlore, de brome, d'iode ou de fluor.

Par cycle aromatique on entend tous cycles aromatiques, tels que benzène, naphtalène.

Par hétérocycle azoté on entend tous cycles aromatiques comportant dans le noyau de un à quatre azotes. Parmi les cycles azotés on préférera particulièrement la pyridine.

Les dérivés les plus proches de ces composés connus à ce jour dans la littérature sont décrits dans un brevet d'invention n° 1.580.823 déposé en France le 1er décembre 1967 sous priorité US du 2 décembre 1966 n° 598 607 au nom de T.Y. SHEN, A.R. MATZUK, et H. SHAM (MERCK et CO.). Ce brevet décrit des benzimidazoles substitués dans l'une des positions 1 ou 2 par un résidu d'acide alcanoïque inférieur et dans l'autre position par un groupe aromatique ou hétéroaromatique ayant moins de trois noyaux condensés.

Le résidu d'acide alcanoïque inférieur est limité à une longueur de chaîne de deux carbones et peut être : $-CH_2-$, $-CH_2-CH_2-$, $-CH(CH_3)-CH_2-$.

Ces dérivés sont décrits comme anti-inflammatoires et anti-pyrétiques.

La demanderesse a trouvé que ces composés ne possèdent pas ou à un degré faible une activité antagoniste des récepteurs au thromboxane.

En effet, la longueur de la chaîne qui porte la fonction acide à un ou deux atomes de carbone est insuffisante pour manifester une affinité convenable avec le récepteur.

Par contre, de façon surprenante, la demanderesse a mis en évidence que lorsque cette chaîne possède plus de trois atomes de carbone ou un hétéroatome comme le soufre et au moins un atome de carbone, et optimalement une chaîne ramifiée à cinq carbones l'affinité avec le récepteur était telle qu'elle permettait d'obtenir de très bons antagonistes au $TXA_2$.

Des variantes de réalisation de l'invention font l'objet des sous- revendications. En particulier, selon ces variantes, A peut être un cycle phényle, un cycle pyridine. $X_1$ peut être un atome de fluor, un atome de chlore. De même, $X_3$ peut être un atome de chlore ou un groupement méthoxy ou un groupement méthyl thio ; $X_4$ peut être un atome de chlore ; D peut être avantageusement une fonction acide.

Selon également une variante particulière, B peut être un groupement méthylène, $R_1$ et $R_2$ représentent chacun un méthyle, $R_3$ et $R_4$ représentent l'hydrogène et n est égal à 1.

Selon une autre variante avantageuse, B est l'atome de soufre.

Selon encore une autre variante, C et $R_1$ et $R_2$ représentent un cyclopentane.

Les composés de l'invention particulièrement préférés sont ceux qui sont choisis parmi les produits de formule :

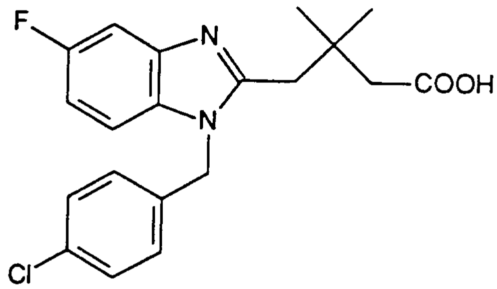

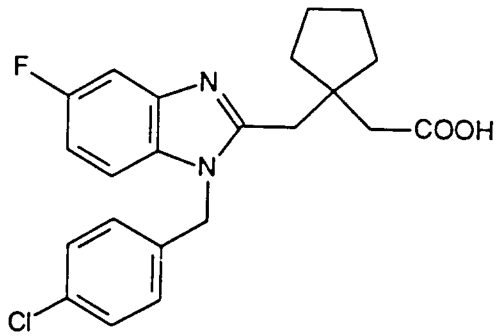

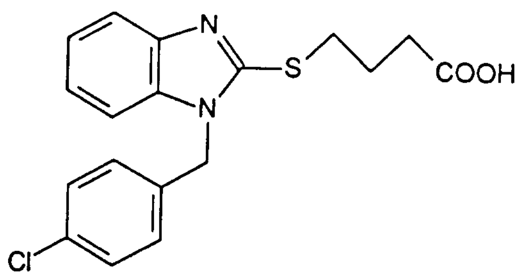

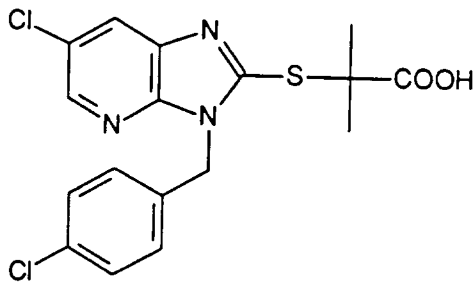

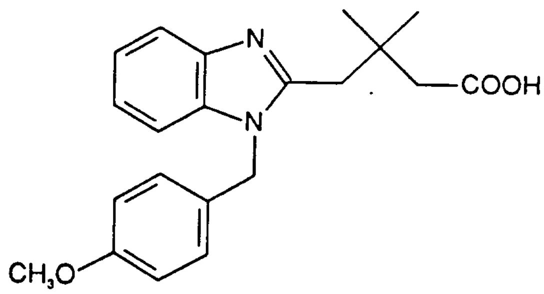

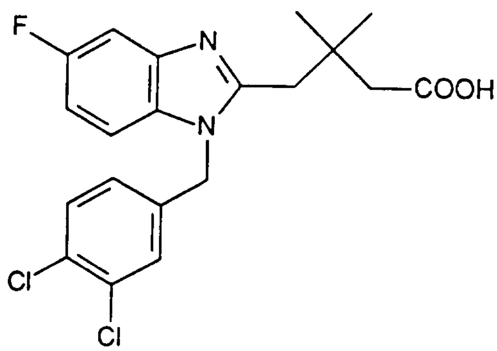

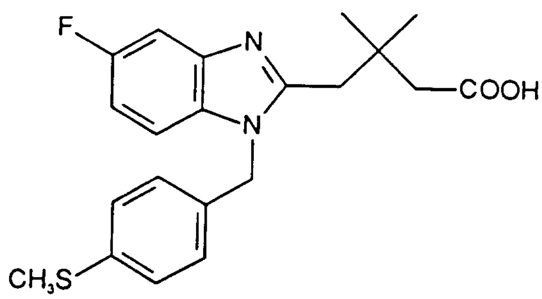

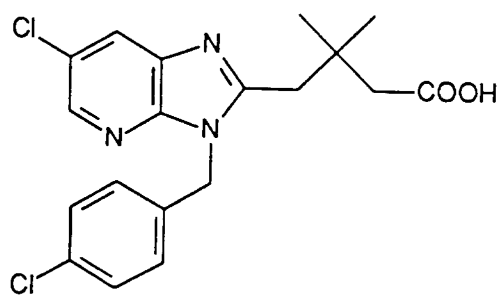

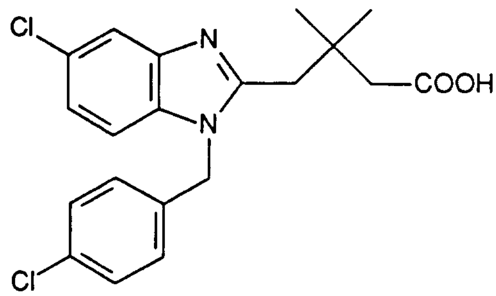

A - <u>Procédé de préparation des composés de formule (I) pour lesquels B est l'atome de soufre.</u>

Selon l'invention, les composés de formule (I), dans lesquels :

B est l'atome de soufre et D un groupement $COOR_7$, A, $X_1$, $X_2$, $X_3$, $X_4$, $R_1$, $R_2$, $R_3$, $R_4$ et n étant définis comme ci-dessus, $R_7$ étant un radical alkyle inférieur peuvent être synthétisés par réaction d'un halogéno alkanoate d'alkyle de formule (II).

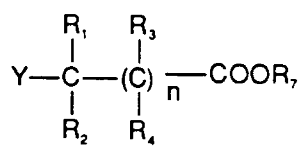

Formule (II)

dans laquelle $R_1$, $R_2$, $R_3$, $R_4$, n et $R_7$ sont définis comme ci-dessus et Y représente un halogène optimalement chlore ou brome sur un dérivé mercapto benzimidazole ou azabenzimidazole de formule (III)

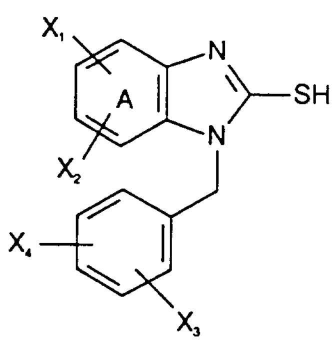

Formule (III)

dans laquelle A, $X_1$, $X_2$, $X_3$ et $X_4$ sont définis comme ci-dessus, en présence d'une base telle qu'un alcoolate de sodium ou de potassium dans un alcool, l'hydrure de sodium dans le diméthyl formamide ou le carbonate de potassium dans l'acétone ou la butanone-2.

Les composés de formule (III) sont synthétisés par action du sulfure de carbone ou du xanthogénate de potassium au reflux d'un solvant tel qu'un alcool sur des composés diamines de formule (IV).

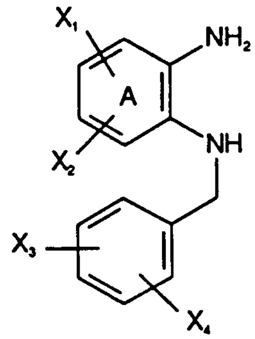

Formule (IV)

dans laquelle A, $X_1$, $X_2$, $X_3$ et $X_4$ sont définis comme ci-dessus.

Les composés de formule (IV) peuvent être obtenus par hydrogénation catalytique en présence de Nickel de Raney par exemple, dans des solvants tels qu'un alcool, le tétrahydrofurane ou le méthoxy-2 éthanol, sous pression ou à pression atmosphèrique et à une température comprise entre 20 et 120 ° C, des dérivés nitrés de formule (V) :

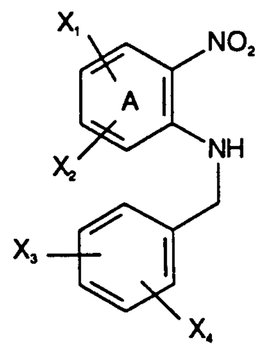

Formule (V)

dans laquelle A, $X_1$, $X_2$, $X_3$ et $X_4$ sont définis comme ci-dessus.

Ces dérivés nitrés de formule (V) peuvent être synthétisés par action d'une benzylamine substituée de formule (VI).

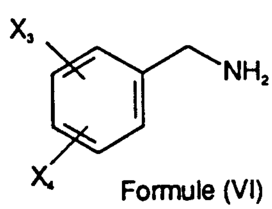

Formule (VI)

dans laquelle $X_3$ et $X_4$ sont définis comme ci-dessus, sur un dérivé nitrohalogéné de formule (VII).

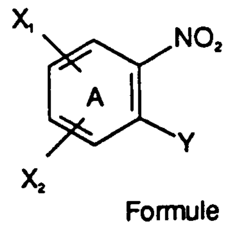

Formule (VII)

dans laquelle A, $X_1$ et $X_2$ sont définis comme ci-dessus et Y représente un atome d'halogène, optimalement chlore ou fluor.

Dans le cas où A représente un phényle, la réaction peut être effectuée dans un solvant tel qu'un alcool ou le tétrahydrofurane en présence de carbonate de potassium ou par simple chauffage à 135 °C des réactifs sans solvant ni base selon le procédé décrit dans le brevet belge 667,333 du 24 janvier 1966.

Dans le cas où A est un hétérocycle azoté, pyridine ou pyrimidine par exemple, on pourra effectuer la réaction dans des solvants comme le toluène ou le xylène en présence ou non de pyridine ou de méthyl-2 éthyl-5 pyridine.

Les benzylamines de formule (VI) sont commerciales ou peuvent être préparées par:

- hydrogénation de la base de Schiff correspondante, obtenue par action de l'ammoniac sur l'aldéhyde correspondant sous pression.
- hydrogénation de l'oxime correspondante, obtenue par action de l'hydroxylamine sur l'aldéhyde correspondant.
- dégradation d'Hoffmann, traitement par une solution d'hypohalite, par exemple l'hypobromite de sodium, du phényl acétamide correspondant.

Les dérivés ortho halogénonitrés de formule (VII) sont commerciaux ou peuvent être synthétisés selon des procédés décrits dans la littérature par exemple dans les références suivantes :

- dans le cas où A est un phényle :
- HOLLEMAN ; REIDING ; Rec.Trav.Chim. Pays Bas 1904, 23, 361
- SWARTS ; Rec.Trav.Chim. Pays Bas 1916, 35, 155

  dans la mesure où A est un hétérocycle azoté :
- BATKOWSKI, T. Rocz. Chem. 1968, 42 (12), 2079-88
- BEBENBURG, W ; STEIMMETZ, S ; THIELE, K : Chemiker Zeïtung ; 1979, 103 (12), 387-99
- BOON, W.R ; JONES, W.G.M. ; RAMAGE, G.R. J.Chem.Soc. 1951, 96
- KRUGER, S ; MANN, F.G. ; J.Chem.Soc. 1955, 2755
- FUJIMATO, M ; Pharm Bull (Tokyo) ; 1956, 4, 340.

Les composés nitrés de formule (V), dans lesquels A est un phényle, peuvent également être préparés en plusieurs étapes à partir de nitro anilines de formule (VII) dans laquelle A est un phényle et Y = $NH_2$. Ces nitroanilines sont traitées, dans un premier temps, par le chlorure de tosyle dans la pyridine. Le sulfonamide obtenu ainsi est ensuite alkylé en présence d'un agent métalant comme l'hydrure de sodium dans un solvant comme le diméthyl formamide par un chlorure de benzyle convenablement substitué. Le groupement tosylate (méthyl-4 benzène sulfonyle) étant ensuite hydrolysé dans l'acide propionique en présence d'acide sulfurique concentré pour donner les dérivés nitrés de formule (V) correspondants.

Une autre variante de procédé de synthèse de ces composés de formule (V) dans lesquels A est un phényle consiste à chauffer à une température comprise entre 100 et 130 °C sans solvant et en présence d'acétate de sodium sec et d'iode les mêmes nitro anilines avec des chlorures ou bromures de benzyle convenablement substitués.

B- Procédé de préparation des composés de formule (I) pour lesquels B est un groupement $CR_5R_6$.

Les dérivés de formule (I) dans lesquels B est un groupement $CR_5R_6$ et D un groupementCN ou $COOR_7$, A, $X_1$, $X_2$, $X_3$, $X_4$, $R_1$, $R_2$, $R_3$, $R_4$, $R_5$, $R_6$ et n étant définis comme ci-dessus et $R_7$ étant un groupement alkyle inférieur peuvent être synthétisés par réaction de dérivés de formule (IV) avec des dérivés de formule (VIII).

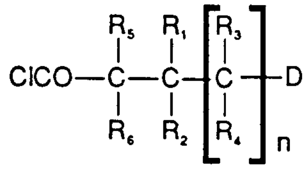

Formule (VIII)

dans laquelle D, $R_1$, $R_2$, $R_3$, $R_4$, $R_5$, $R_6$ et n sont définis comme ci-dessus et $R_7$ est un radical alkyle inférieur.

Ces dérivés de formule (VIII) peuvent être préparés selon un procédé classique de formation des chlorures d'acides par traitement avec par exemple le chlorure de thionyle ou l'oxychlorure de phosphore dans un solvant tel que le toluène par exemple ou sans solvant des dérivés acide esters ou acide cyanés correspondants de formule (IX) :

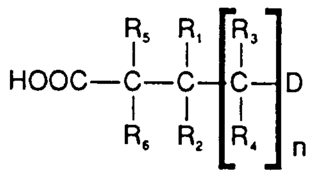

Formule (IX)

dans laquelle D, $R_1$, $R_2$, $R_3$, $R_4$, $R_5$, $R_6$ et n sont définis comme ci-dessus et $R_7$ est un radical alkyle inférieur.

Ces composés de formule (IX) sont obtenus selon différentes voies :

- par monosaponification des diesters ou hydrolyse des cyano esters correspondants en présence d'un équivalent de soude, selon un mode opératoire décrit par exemple dans la référence suivante :
  LE MOAL H ; FOUCAUD A. ; CARRIE R. ; DANION D. et FAYAT C.Bull.Soc.Chim.Fr. 1964, 828.
- par traitement avec un alcool des dérivés anhydrides d'acide correspondants, on aura une autre voie de préparation des dérivés acide esters. Les anhydrides d'acide sont obtenus par déshydratation des diacides correspondants à l'aide de l'anhydride acétique au reflux ou par traitement avec un demi équivalent de dicyclohexylcarbodiimide. La préparation des dérivés diacides utilisés et non commerciaux pourra être trouvée dans les références qui suivent :
  HOWARD E. ZIMMERMAN ; DAVID N. SCHISSEL ; J.Org.Chem. 1986, 51, 196-207
  H. NAJER ; R. GIUDICELLI ; J. SETTE ; Bull.Soc.Chim.Fr. 1964, 2572-2581
  J. SEYDEN PENNE ; M.C. ROUX-SCHMITT ; Bull.Soc.Chim.Fr. 1968, 3812
  N.L. ALLINGER ; M. NAKAZAKI ; V. ZALKOW ; J.Am.Chem.Soc. 1959, 81, 4074-4080
  J. MEINWALD ; J.J TUFARIELLO ; J.J HURST ; J.Am.Chem.Soc. 1964, 86, 2914-2920.

Cette réaction entre les chlorures d'acide esters ou les chlorures d'acide cyanés de formule (VIII) et les dérivés diaminés de formule (IV) est menée en deux étapes.

Dans un premier temps, dans un solvant tel que le chloroforme ou le tétrahydrofurane par exemple, en présence de triéthylamine ou de pyridine, elle conduit à un mélange de composés amides de formules (X) et (X bis).

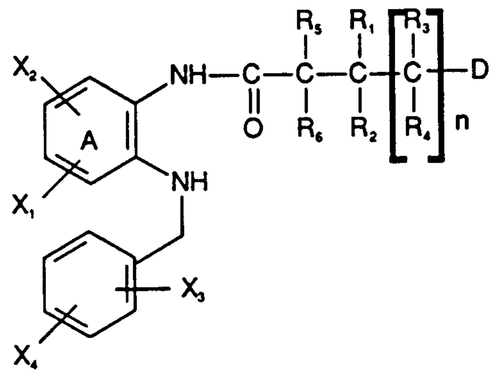

Formule (X)

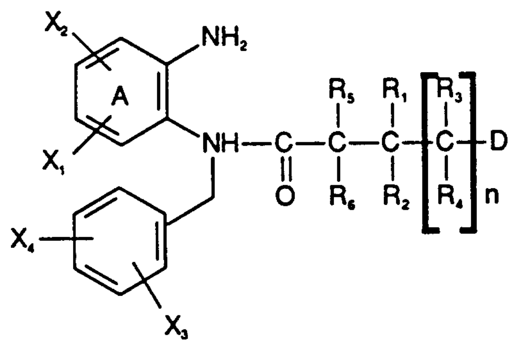

Formule (X bis)

dans lesquelles A, D, $X_1$, $X_2$, $X_3$, $X_4$, $R_1$, $R_2$, $R_3$, $R_4$, $R_5$, $R_6$ et n sont définis comme ci-dessus.

Ce mélange de composés amides de formules (X) et (X bis) est alors traité en milieu acide, soit par l'acide chlorhydrique concentré dans un alcool au reflux, soit par l'acide sulfurique concentré, soit par l'acide polyphosphorique, pour donner les composés de formule (I) dans lesquels B est un groupement $CR_5R_6$ et D est un groupement CN ou $CO_2R_7$, $R_7$ étant un radical alkyle inférieur.

Les composés de formule (I), dans lesquels B est un groupement $CR_5R_6$ et D est un groupement $CO_2R_7$ ou CN, $R_7$ étant un radical alkyle inférieur, $R_5$ et $R_6$ étant définis comme précédemment, peuvent également être préparés de la façon suivante :

Par action sur les composés de formule (IV) dans un mélange d'acide acétique et d'éthanol, d'aldéhydes de formule (VIII'),

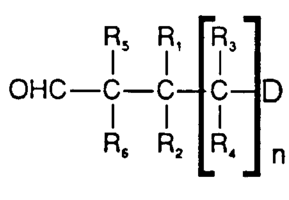

Formule (VIII')

dans laquelle $R_1$, $R_2$, $R_3$, $R_4$, $R_5$, $R_6$, n et D sont définis comme ci-dessus, on obtient des dérivés de formule (X')

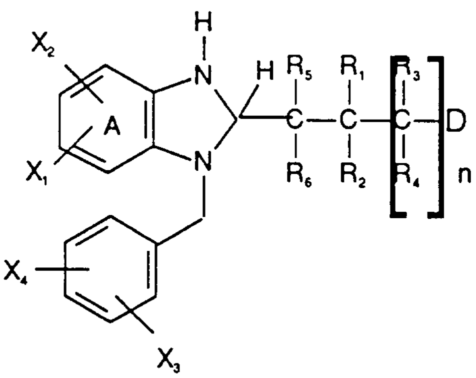

Formule (X')

dans laquelle $X_1$, $X_2$, $X_3$, $X_4$, $R_1$, $R_2$, $R_3$, $R_4$, $R_5$, $R_6$, n et D sont définis comme ci-dessus.

Par traitement de ces composés de formule (X') avec un agent oxydant comme l'iode ou le manganate de Barium, par exemple, à une température allant de l'ambiante à 100°C, on obtiendra les composés de formule (I) dans lesquels B est un groupement $CR_5R_6$ et D est un groupement $CO_2R_7$ou CN, $R_7$ étant un radical alkyle inférieur et $R_5$ et $R_6$ étant définis comme ci-dessus. Des exemples de ce genre de réactions pourront être trouvés dans les références suivantes :

- Srivastava. R.G. ; Venkataramani. P.S ; Synt. Comm. 1988, 18 (13), 1537-1544
- Perumattam. G ; Synt. Comm. 1989, 19 (19), 3367-3370.

Les aldéhydes de formule (VIII') peuvent être préparés par hydrogénation catalytique, en présence de palladium sur charbon et de lutidine dans le tétrahydrofurane, des dérivés chlorures d'acides de formule (VIII) dans laquelle D est une fonction ester.

Ce procédé est particulièrement avantageux lorsque le cycle A est un cycle pyridinique.

Les composés de formule (I) dans lesquels A est un phényle et D le groupement CN ou $CO_2R_7$, $R_7$ étant un radical alkyle inférieur peuvent également être synthétisés par action d'un chlorure de benzyle convenablement substitué sur des dérivés de formule (XI).

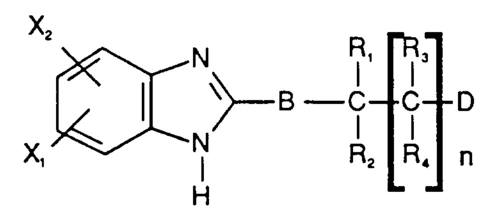

Formule (XI)

dans laquelle D, $X_1$, $X_2$, $R_1$, $R_2$, $R_3$, $R_4$, B et n sont définis comme ci-dessus, dans un solvant comme le diméthyl formamide en présence d'un agent métallant comme l'hydrure de sodium.

Les composés de formule (XI) dans lesquels B est l'atome de soufre peuvent être synthétisés à partir des composés de formule (XII).

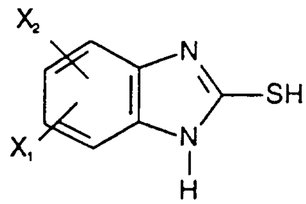

Formule (XII)

dans laquelle $X_1$ et $X_2$ sont définis comme ci-dessus, avec des composés halogéno alkanoate d'alkyle de formule (II) en présence d'une base telle que l'éthylate de sodium dans l'alcool ou le carbonate de potassium dans l'acétone ou le tétrahydrofurane.

Les composés de formule (XII) sont commerciaux ou synthétisés selon des procédés décrit dans le BEILSTEIN 24, 119 et 24, complément (3), 293.

Les composés de formule (XI) dans lesquels B est un groupement $CR_5$ $R_6$, $R_5$ et $R_6$ définis comme ci-dessus, peuvent être synthétisés par action sur un composé orthophénylène diamine de formule (XIII).

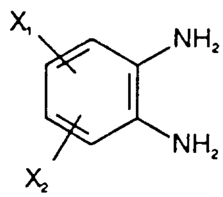

Formule (XIII)

dans laquelle $X_1$ et $X_2$ sont définis comme ci-dessus, d'un composé chlorure d'acide ester de formule (VIII), dans des conditions identiques à celles décrites précédemment pour la réaction des composés de formule (IV) avec les dérivés de formule (VIII).

Les composés de formule (XIII) sont commerciaux.

Les composés de formule (I) dans lesquels D est le groupement $COOR_7$ et $R_7$ l'atome d'hydrogène, sont obtenus par l'hydrolyse classique, soit en milieu acide, soit en milieu basique des composés de formule (I) dans lesquels D est le groupement $COOR_7$ et $R_7$ un radical alkyle inférieur ou encore D est le groupement CN.

Les composés de formule (I) dans lesquels D est un groupement $CONH\text{-}R_8$ sont obtenus par action d'amines de formule $R_8\text{-}NH_2$, $R_8$ étant défini comme ci-dessus sur des chlorures d'acide de formule (XIV).

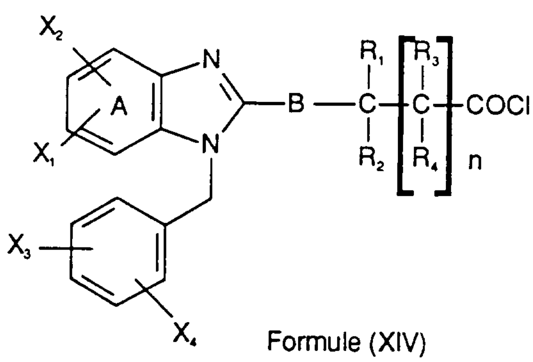

Formule (XIV)

dans laquelle A, $X_1$, $X_2$, $X_3$, $X_4$, $R_1$, $R_2$, $R_3$, $R_4$, B et n sont définis comme ci-dessus dans un solvant tel que le chloroforme ou le tétrahydrofurane en présence d'un excès d'amine ou alors de triéthylamine ou de pyridine.

Dans le cas où $R_8$ est l'hydrogène, on pourra tout simplement faire réagir le chlorure d'acide de formule

(XIV) sur une solution d'ammoniaque.

Les composés de formule (XIV) peuvent être préparés selon une méthode classique pour les chlorures d'acide, action du chlorure de thionyle ou du chlorure d'oxalyle ou de l'oxychlorure de phosphore sur les composés de formule (I) correspondants dans lesquels D est le groupement $CO_2H$.

Les composés de formule (I) dans lesquels D est le groupement CN peuvent être également préparés par déshydratation des composés amides, où D représente $CONH_2$, correspondants par traitement avec par exemple l'oxychlorure de phosphore dans un solvant comme le diméthylformamide ou sans solvant.

Des sels d'addition de certains composés de formule (I) peuvent s'obtenir, en particulier des sels d'addition pharmaceutiquement acceptables. On peut citer en particulier lorsque D représente une fonction acide, les sels de sodium, potassium, calcium.

Les nouveaux composés selon l'invention possèdent des propriétés pharmacologiques remarquables comme antagonistes des récepteurs au thromboxane et peuvent être utilisés en thérapeutique pour le traitement de l'infarctus du myocarde, de l'angine de poitrine, l'ictus cérébral, la migraine, les hémorragies cérébrales, l'athérosclérose, l'embolie pulmonaire, l'asthme bronchique, la bronchite, la pneumonie, les chocs circulatoires d'origines diverses, la néphrite, le rejet de greffe, les métastases cancéreuses.

Ainsi, l'invention couvre également une composition pharmaceutique, caractérisée en ce qu'elle comprend une quantité pharmaceutiquement efficace d'au moins un composé de formule (I), tel que précédemment défini, ainsi qu'éventuellement ses sels d'addition pharmaceutiquement acceptables, éventuellement incorporé dans un excipient, véhicule ou support pharmaceutiquement acceptable.

L'invention couvre encore une composition pharmaceutique à activité antagoniste des récepteurs au thromboxane permettant notamment de traiter favorablement l'infarctus du myocarde, l'angine de poitrine, l'ictus cérébral, la migraine, l'hémorragie cérébrale, l'arthérosclérose, l'embolie pulmonaire, l'asthme bronchique, la bronchite, une pneumonie, les chocs circulatoires d'origine diverses telles qu'hémorragie, septicémie, défaillance cardiaque, traumatique, pancréatique aigûe, brûlure, bactérienne, les néphrites, le rejet de greffe, les métastases cancéreuses, caractérisée en ce qu'elle comprend une quantité pharmaceutiquement efficace d'au moins un composé de formule (I) précité ou un de ses sels d'addition pharmaceutiquement acceptable, éventuellement incorporé dans un excipient, véhicule ou support pharmaceutiquement acceptable.

L'invention couvre encore un procédé de préparation d'une composition pharmaceutique, caractérisée en ce qu'on incorpore une quantité pharmaceutiquement efficace d'au moins un composé de formule (I) telle que précédemment définie, ainsi qu'éventuellement ses sels d'addition pharmaceutiquement acceptable, dans un excipient, véhicule ou support pharmaceutiquement acceptable.

Selon un autre mode de réalisation, on prépare une composition pharmaceutique à activité antagoniste des récepteurs au thromboxane permettant notamment de traiter favorablement l'infarctus du myocarde, l'angine de poitrine, l'ictus cérébral, la migraine, l'hémorragie cérébrale, l'arthérosclérose, l'embolie pulmonaire, l'asthme bronchique, la bronchite, une pneumonie les chocs circulatoires d'origine diverses telles qu'hémorragie, septicémie, défaillance cardiaque , traumatique, pancréatique aigûe, brûlure, bactérienne, les néphrites, le rejet de greffe, les métastases cancéreuses.

Selon une autre variante de réalisation, on prépare une composition pharmaceutique formulée sous forme de gélules ou de comprimés dosés de 1 à 200 mg ou sous forme de préparations injectables dosées de 0,01 à 10 mg.

L'invention couvre encore un procédé de traitement thérapeutique des mammifères, caractérisé en ce qu'on administre à ce mammifère une quantité thérapeutiquement efficace d'au moins un composé de formule (I) telle que précédemment définie, ou un de ses sels d'addition pharmaceutiquement acceptable, éventuellement incorporé dans un excipient, véhicule ou support pharmaceutiquement acceptable. Selon une variante de réalisation de ce procédé de traitement, le composé de formule (I) soit seul soit en association avec un excipient pharmaceutiquement acceptable, est formulé sous forme de gélules ou de comprimés dosés de 1 à 200 mg pour l'administration par voie orale, ou sous forme préparations injectables dosées de 0,01 à 10 mg pour une administration par voie parentérale.

En thérapeutique humaine et animale, les composés de formule (I) et leurs sels peuvent être administrés seuls ou en association avec un excipient physiologiquement acceptable sous une forme quelconque, en particulier sous forme de gélules et de comprimés par voie orale ou sous forme de soluté injectable par voie parentérale.

Comme il ressortira clairement des essais de pharmacologie donnés en fin de description les composés selon l'invention peuvent être administrés en thérapeutique humaine dans les indications précitées par voie orale sous forme de comprimés ou de gélules dosés de 1 à 200 mg ou par voie parentérale sous forme de préparations injectables dosées de 0,01 à 10 mg en une ou plusieurs prises journalières pour un adulte de poids moyen 60 à 70 kg.

En thérapeutique animale, la dose journalière utilisable devrait habituellement se situer de 1 à 100 mg par kg.

D'autres caractéristiques et avantages de l'invention seront mieux compris à la lecture qui va suivre de quelques exemples de préparation nullement limitatifs, mais donnés à titre d'illustration.

Exemple 1 :
**[(chloro-4 phényl) méthyl amino]-2 fluoro-5 nitrobenzène**

**Formule (V)** : $X_1$ = 5-F, $X_2$ = $X_3$ = H, $X_4$ = 4-Cl, A = phényl

30 g de difluoro-2,5 nitrobenzène et 26,7 g de chloro-4 benzylamine sont dissous dans 300 ml de tétrahydrofurane. 40 g de carbonate de potassium sont additionnés à cette solution et le mélange est porté au reflux durant 8 heures. Après refroidissement, le mélange réactionnel est additionné dans 1,7 l d'eau et 50 ml d'acide chlorhydrique concentré. Les cristaux obtenus sont essorés et lavés a l'eau puis à l'éther isopropylique pour donner 41,9 g de [(chloro-4 phényl) méthyl amino]-2 fluoro-5 nitrobenzène sous forme de cristaux de point de fusion 160 °C.

Selon ce mode opératoire l'exemple suivant a été synthétisé.

Exemple 2 : **[(dichloro-3,4-phényl) méthyl amino]-2 fluoro-5 nitrobenzène**

**Formule (V)** : $X_1$ = 5-F, $X_2$ = H, $X_3$ = 3-Cl, $X_4$ = 4-Cl, A = phényl

Cristaux de point de fusion 110 °C.

Exemple 3 :
**[(chloro-4 phényl) méthyl amino]-2 chloro-5 nitrobenzène**

**Formule (V)** : $X_1$ = 5-Cl, $X_2$ = H, $X_3$ = 4-Cl, $X_4$ = H, A = phényl

25 g de dichloro-2,5-nitrobenzène et 36,9 g de chloro-4 benzylamine sont chauffés deux heures à 135 °C, la température devant toujours être maintenue en dessous de 140 °C. Après refroidissement le mélange est repris à l'eau et extrait à l'acétate d'éthyle. Après séchage sur sulfate de magnésium et évaporation sous vide, le résidu est repris à l'éther et les cristaux obtenus sont essorés et lavés à l'éther pour donner 22,3 g de [(chloro-4 phényl) méthyl amino]-2 chloro-5 nitrobenzène sous forme de cristaux de point de fusion 120 °C.

Exemple 4 :
**[(chloro-4 phényl) méthyl amino]-2 methoxy-5 nitrobenzène**

**Formule (V)** : $X_1$ = 5-OMe, $X_2$ = H, $X_3$ = 4-Cl, $X_4$ = H, A = phényl

A) [(méthyl-4 phényl sulfonyl)amino]-2 methoxy-5 nitrobenzène :

50g de méthoxy-4 nitro-2 aniline sont agités à 0 °c dans 300 ml de pyridine. 56,7g de chlorure de tosyle sont ajoutés par portions à 0 °C et le mélange est ensuite agité deux heures à température ambiante, laissé une nuit au repos et versé dans un mélange glace/eau. Les cristaux obtenus sont essorés et lavés à l'eau puis à l'éther isopropylique pour donner 72,8g de [(méthyl-4 benzène sulfonyl)amino]-2 methoxy-5 nitrobenzène sous forme de cristaux de point de fusion 99 °C.

B) N-(chloro-4 benzyl) N-(méthyl-4 benzène sulfonyl) nitro-2 méthoxy-4 aniline

72,8g de [(méthyl-4 benzène sulfonyl)amino]-2 methoxy-5 nitrobenzène préparés en A) sont ajoutés à 56,5 ml de soude 4N et 29,2g de chlorure de chloro-4 benzyle. Le mélange est porté au reflux 4 heures puis 43,7g de chlorure de chloro-4 benzyle sont rajoutés et le tout est chauffé de nouveau 45 minutes au reflux. Après refroidissement, 12,2 ml de soude à 35% sont ajoutés au mélange réactionnel et celui-ci est chauffé au reflux durant trois heures et 45 minutes, puis refroidi et additionné d'eau et d'ether. L'insoluble est essoré et lavé à l'eau et à l'éther pour donner 98g de N-(chloro-4 benzyl) N-(méthyl-4 benzène sulfonyl) nitro-2 methoxy-4 aniline sous forme de cristaux de point de fusion 124 °C. L'évaporation de la phase éthérée permet d'obtenir 10g supplémentaires de cristaux de point de fusion 124 °C.

C) [(chloro-4 phényl) méthyl amino]-2 methoxy-5 nitrobenzène

108g de N-(chloro-4 benzyl) N-(méthyl-4 benzène sulfonyl) nitro-2 méthoxy-4 aniline préparés en B) sont ajoutés à 940 ml d'acide propionique et 102 ml d'acide sulfurique concentré. Le mélange est chauffé à 95 °C pendant 1h30 et la solution est concentrée au demi par évaporation sous vide, puis versée sur de la glace et neutralisée à l'hydroxyde d'ammonium.
Les cristaux obtenus sont essorés et lavés à l'eau puis à l'éther isopropylique pour donner 60g de [(chloro-4 phényl) méthyl amino]-2 methoxy-5 nitrobenzène sous forme de cristaux de point de fusion 135 °C.

Exemple 5 :
**[(chloro-4 phényl) méthyl amino]-2 nitro-3 pyridine**

**Formule (V)** : $X_3$ = 4-Cl, $X_1$ = $X_2$ = $X_4$ = H,A = pyridine-2
On porte au reflux durant 12 heures une solution de 26,5g de chloro-2 nitro-3 pyridine, 23,7g de chloro-4 benzylamine et 25 ml de méthyl-2 éthyl-5 pyridine dans 200 ml de xylène. Le mélange réactionnel est ensuite refroidi, additionné d'eau et d'acide acétique puis extrait à l'éther. La phase organique est séchée sur sulfate de magnésium, filtrée puis évaporée sous vide pour donner une huile qui cristallise dans l'éther isopropylique. Les cristaux sont essorés puis séchés. On obtient 27g de [(chloro-4 phényl) méthyl amino]-2 nitro-3 pyridine sous forme de cristaux de point de fusion 100 °C.

Exemple 6 :
**[(chloro-4 phényl) méthyl amino]-2 nitro-3 chloro-5 pyridine**

**Formule (V)** : $X_1$ = 5-Cl, $X_2$ = H, $X_3$ = 4-Cl, $X_4$ = H,A = pyridine-2
Une solution de 20,9g de chloro-4 benzylamine et 15,7g de dichloro-2,5 nitro-3 pyridine dans 250 ml de xylène et 20 ml de méthyl-2 éthyl-5 pyridine est chauffée 30 heures au reflux. Après refroidissement le mélange réactionnel est additionné d'eau puis extrait à l'acétate d'éthyle, la phase organique est lavée avec une solution diluée d'acide chlorhydrique et séchée sur sulfate de magnésium. Le solvant est évaporé sous vide et le résidu obtenu cristallise dans l'éther isopropylique pour donner 21,1 g de [(chloro-4 phényl) méthyl amino]-2 nitro-3 chloro-5 pyridine sous forme de cristaux de point de fusion 120 °C.

Exemple 7 :
**[(fluoro-2 bromo-4 phényl) méthyl amino]-2 nitro-3 chloro-5 pyridine**

**Formule (V)** : $X_1$ = 5-Cl, $X_2$ = H, $X_3$ = 2-F, $X_4$ = 4-Br, A = pyridine-2
Préparé selon le mode opératoire de l'exemple 6.
Cristaux de point de fusion 75-77 °C.

Exemple 8 :
**[(chloro-4 phényl) méthyl amino]-2 fluoro-5 aniline**

**Formule (IV) :** $X_1$ = 5-F, $X_2$ = H, $X_3$ = 4-Cl, $X_4$ = H, A = phényl
41,7 g de [(chloro-4 phényl) méthyl amino]-2 fluoro-5 nitrobenzène préparé à l'exemple 1 sont dissous dans 1 l de tétrahydrofurane et hydrogénés à pression et température ordinaires en présence de 5 g de Nickel de Raney. Quand la quantité théorique d'hydrogène a été absorbée, le catalyseur est éliminé par filtration et le solvant évaporé sous vide. On obtient ainsi 34,1 g de [(chloro-4 phényl) méthyl amino]-2 fluoro-5 aniline sous forme de cristaux de point de fusion 99 °C.
Selon le même mode opératoire, les exemples suivants ont été préparés.

Exemple 9 :
**[(chloro-4 phényl) méthyl amino] méthoxy-5 aniline**

**Formule (IV)** : $X_1$ = 5-MeO, $X_2$ = H, $X_3$ = 4-Cl, $X_4$ = H, A = phényl
Cristaux de point de fusion 90 °C.

Exemple 10 :
**[(dichloro-3,4 phényl) méthyl amino]-2 fluoro-5 aniline**

**Formule (IV)** : $X_1$ = 5-F, $X_2$ = H, $X_3$ = 3-Cl, $X_4$ = 4-Cl, A = phényl
Cristaux de point de fusion 104 ° C.

Exemple 11 :
**[(chloro-4 phényl) méthyl amino]-2 chloro-5 aniline**

**Formule (IV)** : $X_1$ = 5-Cl, $X_2$ = H, $X_3$ = 4-Cl, $X_4$ = H, A = phényl
Cristaux de point de fusion 138 ° C.

Exemple 12 :
**[(fluoro-2 bromo-4 phényl) méthyl amino]-2 amino-3 chloro-5 pyridine**

**Formule (IV)** : $X_1$ = 5-Cl, $X_2$ = H, $X_3$ = 2-F, $X_4$ = 4-Br, A = pyridine-2
Cristaux de point de fusion 97 ° C.

Exemple 13 :
**[(chloro-4 phényl) méthyl amino]-2 amino-3 pyridine**

**Formule (IV)** : $X_1$ = H, $X_2$ = H, $X_3$ = 4-Cl, $X_4$ = H, A = pyridine-2
Cristaux de point de fusion 132 ° C.

Exemple 14 :
**[(chloro-4 phényl) méthyl amino]-2 amino-3 chloro-5 pyridine**

**Formule (IV)** : $X_1$ = 5-Cl, $X_2$ = H, $X_3$ = 4-Cl, $X_4$ = H, A = pyridine-2
Huile utilisée telle quelle pour la suite.

Exemple 15 : **(chloro-4 phényl méthyl)-1 mercapto-2 fluoro-5 benzimidazole**

**Formule (III)** : $X_1$ = 5-F, $X_2$ = H, $X_3$ = 4-Cl, $X_4$ = H,A = phényl
A 35,2 g de [(chloro-4 phényl) méthyl amino]-2 fluoro-5 aniline préparée à l'exemple 8 dissous dans 500 ml d'éthanol, sont ajoutés 25 ml de sulfure de carbone. Le mélange est porté au reflux 12 heures et laissé revenir à température ambiante. Après quelques heures de repos les cristaux sont essorés et lavés à l'éthanol puis à l'isopropanol et à l'éther pour donner 33 g de (chloro-4 phényl méthyl)-1 mercapto-2 fluoro-5 benzimidazole sous forme de cristaux de point de fusion 215 ° C.
Selon le même mode opératoire nous avons préparé les exemples suivants :

Exemple 16 :
**(chloro-4 phényl méthyl)-1 mercapto-2 imidazo [4,5-b] pyridine**

**Formule (III)** : $X_3$ = 4-Cl, $X_1$ = $X_2$ = $X_4$ = H,A = pyridine-2
Cristaux de point de fusion 216 ° C.

Exemple 17 :
**(chloro-4 phényl méthyl)-1 mercapto-2 chloro-5 imidazo [4,5-b] pyridine**

**Formule (III)** : $X_1$ = 5-Cl, $X_2$ = H, $X_3$ = 4-Cl, $X_4$ = H,A = pyridine-2
Cristaux de point de fusion 260 ° C.

Exemple 18 :
**(Fluoro-2 bromo-4 phényl méthyl)-1 mercapto-2 chloro-5 pyridine**

**Formule (III)** : $X_1$ = 5-Cl, $X_2$ = H, $X_3$ = 2-F, $X_4$ = 4-Br, A = pyridine-2
Cristaux de point de fusion 240 ° C.

Exemple 19 :
**[(Chloro-4 benzyl)-1 fluoro-5 benzimidazolyl-2] mercapto-4 butanoate d'éthyle**

**Formule (I)** : $X_1$ = 5-F, $X_2$ = H, $X_3$ = 4-Cl, $X_4$ = H, A = phényl, B = S,
D = $CO_2Et$, $R_1$ = $R_2$ = $R_3$ = $R_4$ = H, n = 2

9 g de (chloro-4 phényl méthyl)-1 mercapto-2 fluoro-5 benzimidazole préparé à l'exemple 15 et 4,4 ml de bromo-4 butyrate d'éthyle sont chauffés 5 heures au reflux dans 100 ml d'acétone en présence de 6,3 g de carbonate de potassium. Le solvant est évaporé sous vide et le résidu repris à l'eau puis extrait à l'acétate d'éthyle est lavé avec une solution de soude diluée. La phase organique est séchée sur sulfate de magnésium et évaporée sous vide pour donner 11,9 g de [(Chloro-4 benzyl)-1 fluoro-5 benzimidazolyl-2] mercapto-4 butanoate d'éthyle sous forme d'huile utilisée telle quelle pour la suite.

Selon le même mode opératoire les exemples suivants ont été préparés :

Exemple 20 : **[(chloro-4 benzyl)-1 fluoro-5 benzimidazolyl-2] mercapto-5 pentanoate d'éthyle**

**Formule (I)** : $X_1$ = 5-F, $X_2$ = H, $X_3$ = 4-Cl, $X_4$ = H, A = phényl, B = S,
D = $CO_2Et$, $R_1$ = $R_2$ = $R_3$ = $R_4$ = H,n = 3
Huile utilisée telle quelle pour le suite.

Exemple 21 :
**[(Fluoro-2 bromo-4 benzyl)-1 chloro-5 imidazo [4,5-b] pyridine yl-2] mercapto-4 butanoate d'éthyle**

**Formule (I)** : $X_1$ = 5-Cl, $X_2$ = H, $X_3$ = 2-F, $X_4$ = 4-Br, A = pyridine-2,
D = $CO_2Et$, B = S, $R_1$ = $R_2$ = $R_3$ = $R_4$ = H, n = 2
Cristaux de point de fusion 94 °C.

Exemple 22 :
**[(Chloro-4 benzyl)-1 chloro-5 imidazo [4,5-b] pyridine yl-2] mercapto-2 méthyl-2 propionate d'éthyle**

**Formule (I)** : $X_1$ = 5-Cl, $X_2$ = H, $X_3$ = 4-Cl, $X_4$ = H, A = pyridine-2,
D = $CO_2Et$, B = S, $R_1$ = $R_2$ = $CH_3$, n = O
Huile utilisée telle quelle pour la suite.

Exemple 23 :
**[(Chloro-4 benzyl)-1 imidazo [4,5-b] pyridine yl-2] mercapto-4 butanoate d'éthyle**

**Formule (I)** : $X_1$ = H, $X_2$ = $X_3$ = H, $X_4$ = 4-Cl, A = pyridine-2,
D = $CO_2Et$, B = S, $R_1$ = $R_2$ = $R_3$ = $R_4$ = H, n = 2
Huile utilisée telle quelle pour la suite.

Exemple 24:
**(benzimidazolyl-2) mercapto-4 butanoate d'éthyle**

**Formule (XI)** : $X_1$ = $X_2$ = H, B = S, D = $CO_2Et$
$R_1$ = $R_2$ = $R_3$ = $R_4$ = H, n = 2

50g de mercapto-2 benzimidazole sont dissous dans 300 ml d'éthanol et une solution de 7,65g de sodium dans 150 ml d'éthanol est ajoutée à température ambiante et sous agitation. Le mélange est agité quelques minutes à température ambiante et 64,3g de bromo-4 butyrate d'éthyle sont ajoutés rapidement. Le mélange réactionnel est chauffé 6 heures au reflux, puis refroidi. Les solvants sont évaporés à sec sous vide et le résidu est repris à l'eau, les cristaux obtenus sont essorés et lavés à l'eau puis à l'éther et séchés pour donner 85g de (benzimidazolyl-2) mercapto-4 butanoate d'éthyle sous forme de cristaux de point de fusion 70-72 °C.

Exemple 25:
**(benzimidazolyl-2) mercapto-5 pentanoate d'éthyle**

**Formule (XI)** : $X_1$ = $X_2$ = H, B = S, D = $CO_2Et$
$R_1$ = $R_2$ = $R_3$ = $R_4$ = H, n = 3

Préparé selon le mode opératoire de l'exemple 24.
Cristaux de point de fusion 100 ° C.

Exemple 26 :
**[(Chloro-4 benzyl)-1 benzimidazolyl - 2] mercapto-4 butanoate d'éthyle**

**Formule (I)** : $X_1$ = H, $X_2$ = $X_3$ = H, $X_4$ = 4-Cl, A = phényl,
D = $CO_2$Et, B = S, $R_1$ = $R_2$ = $R_3$ = $R_4$ = H, n = 2

20g de (benzimidazolyl-2) mercapto-4 butanoate d'éthyle préparé à l'exemple 24 sont ajoutés à une suspension de 2,9g d'hydrure de sodium à 60% dans 150 ml de dimethylformamide anhydre. Le mélange est agité 30 minutes à 80 ° C puis refroidi à température ambiante et une solution de 12,7g de chloro-4 chloromethyl benzène dans 20 ml de dimethyl formamide anhydre est ajoutée goutte à goutte.

Le mélange est chauffé au reflux pendant 5 heures et le solvant est évaporé à sec. Le résidu est repris à l'eau et extrait à l'acétate d'éthyle, la phase organique est séchée puis évaporée sous vide pour donner 27g de [(Chloro-4 benzyl)-1 benzimidazolyl - 2] mercapto-4 butanoate d'éthyle sous forme d'une huile utilisée telle quelle pour la suite. Selon le même mode opératoire l'exemple suivant à été préparé.

Exemple 27 :
**[(dichloro -2,4- benzyl)-1 imidazolyl- 2] mercapto-4 butanoate d'éthyle**

**Formule (I)** : $X_1$ = H, $X_2$ = H, $X_3$ = 2-Cl, $X_4$ = 4-Cl, A = phényl,
D = $CO_2$Et, B = S, $R_1$ = $R_2$ = $R_3$ = $R_4$ = H, n = 2
Cristaux de point de fusion 92 ° C.

Exemple 28 :
**Chlorure d'acide, ester éthylique de l'acide diméthyl-3,3-glutarique**

**Formule (VIII)**: $R_5$ = $R_6$ = H, $R_1$ = $R_2$ = $CH_3$,$R_3$ = $R_4$ = H, n = 1, D = $CO_2$Et

50g d'anhydride dimethyl-3,3-glutarique sont dissous dans 500 ml d'éthanol absolu et le mélange est chauffé au reflux 12 heures. L'alcool est évaporé à sec sous vide et 250 ml de toluène sont rajoutés au résidu, puis 45 ml de chlorure de thionyle sont coulés goutte à goutte et sous agitation à température ambiante.

Le mélange est chauffé à 80 ° C pendant deux heures puis les solvants sont évaporés, le résidu est distillé entre 115-125 ° C sous 20 mm de mercure pour donner 58,2g de chlorure d'acide-ester ethylique de l'acide diméthyl- 3,3-glutarique.
Selon le même mode opératoire les exemples suivants ont été préparés :

Exemple 29 :
**Chlorure d'acide, ester éthylique de l'acide méthyl-3- glutarique**

**Formule (VIII)** : $R_5$ = $R_6$ = H, $R_1$ = $CH_3$, $R_2$ = $R_3$ = $R_4$ = H, n = 1, D = $CO_2$Et
Huile utilisée telle quelle pour la suite.

Exemple 30 :
**Chlorure d'acide, ester éthylique de l'acide diéthyl-3,3-glutarique**

**Formule (VIII)** : $R_5$ = $R_6$ = H, $R_1$ = $R_2$ = $C_2H_5$, $R_3$ = $R_4$ = H, n = 1, D = $CO_2$Et
Huile utilisée telle quelle pour la suite.

Exemple 31 :
**Chlorure d'acide, ester éthylique de l'acide méthyl-3-ethyl-3-glutarique**

**Formule (VIII)** : $R_5$ = $R_6$ = H, $R_1$ = $CH_3$, $R_2$ = $C_2H_5$, $R_3$ = $R_4$ = H, n = 1, D = $CO_2$Et
Huile utilisée telle quelle pour la suite.

Exemple 32 :
**Chlorure d'acide, ester éthylique de l'acide cyclohexane-1,1-diacétique**

**Formule (VIII)** : $R_5 = R_6 = H$, $R_1 + R_2 = CH_2-CH_2-CH_2-CH_2-CH_2$.
$R_3 = R_4 = H$, n = 1, D = $CO_2Et$
Huile de point d'ébulition 170-175 ° C sous 25 mm de mercure.

Exemple 33 :
**Chlorure d'acide, ester éthylique de l'acide cyclopentane-1,1-diacétique**

**Formule (VIII)** : $R_5 = R_6 = H$, $R_1 + R_2 = CH_2-CH_2-CH_2-CH_2$, $R_3 = R_4 = H$, n = 1,
D = $CO_2Et$
Huile de point d'ébulition 165-170 ° C sous 25 mm de mercure.

Exemple 34:
**[(Chloro-4 benzyl)-1 fluoro-5 benzimidazolyl - 2] -4dimethyl-3,3-butanoate d'éthyle**

**Formule (I)** : $R_1 = R_2 = CH_3$, $R_3 = R_4 = H$, n = 1, D = $CO_2$ Et, A = phényl
B = $CH_2$, $X_1$ = 5-F, $X_2$ = H, $X_3$ = 4-Cl, $X_4$ = H

10g de (chloro-4 benzyl amino) -2 fluoro-5 aniline préparés à l'exemple 8 sont dissous dans 100 ml de chloroforme stabilisé à l'amylène et 6 ml de triéthylamine. On rajoute goutte à goutte une solution de 8,25g de chlorure d'acide-ester éthylique de l'acide diméthyl-3,3-glutarique, préparés à l'exemple 28, dans 20 ml de chloroforme stabilisé à l'amylène. Le mélange est agité deux heures à température ambiante, les cristaux formés sont éliminés par filtration et les solvants évaporés sous vide. Le résidu obtenu est dissous dans 200 ml d'éthanol et 30 ml d'acide chlorhydrique concentré et le mélange est porté 10 heures au reflux. Les solvants sont évaporés à sec, et le résidu est repris à l'eau puis extrait à l'acétate d'éthyle. La phase organique est séchée et évaporée sous vide pour donner 14g de [(Chloro-4 benzyl)-1 fluoro-5 benzimidazolyl - 2]-4 dimethyl-3,3-butanoate d'éthyle sous forme d'huile utilisée telle quelle pour la suite.

Selon le même mode opératoire les exemples suivants ont été synthétisés:

Exemple 35:
**[[[(Chloro-4 benzyl)-1 fluoro-5 benzimidazolyl - 2] methyl]-1 cyclopentyl-1] acétate d'éthyle**

**Formule (I)** : $R_1 + R_2 = -CH_2-CH_2-CH_2-CH_2$ , $R_3 = R_4 = H$, n = 1,
D = $CO_2$ Et, A = phényl,
B = $CH_2$, $X_1$ = 5-F, $X_2$ = H, $X_3$ = 4-Cl, $X_4$ = H
Huile utilisée telle quelle pour la suite.

Exemple 36:
**[[[(Chloro-4 benzyl)-1 fluoro-5 benzimidazolyl - 2] methyl]-1 cyclohexyl-1] acétate d'éthyle**

**Formule (I)** : $R_1 + R_2 = CH_2-CH_2-CH_2-CH_2-CH_2$, $R_3 = R_4 = H$, n = 1,
D = $CO_2$ Et, A = phényl
B = $CH_2$, $X_1$ = 5-F, $X_2$ = H, $X_3$ = 4-Cl, $X_4$ = H
Huile utilisé telle quelle pour la suite.

Exemple 37:
**[(dichloro-3,4-benzyl)-1 fluoro-5 benzimidazolyl - 2]-4 diméthyl -3,3-butanoate d'éthyle**

**Formule (I)** : $R_1 = R_2 = CH_3$, $R_3 = R_4 = H$, n = 1, D = $CO_2$ Et, A = phényl B = $CH_2$,
$X_1$ = 5-F, $X_2$ = H, $X_3$ = 3-Cl, $X_4$ = 4-Cl
Huile utilisée telle quelle pour la suite.

Exemple 38:
**[(chloro-4-benzyl)-1 chloro-5 benzimidazolyl - 2]-4 diméthyl -3,3-butanoate d'éthyle**

**Formule (I)** : $R_1 = R_2 = CH_3$, $R_3 = R_4 = H$, n = 1, D = $CO_2$ Et, A = phényl B = $CH_2$,
$X_1$ = 5-Cl, $X_2$ = H, $X_3$ = 4-Cl, $X_4$ = H

Huile utilisée telle quelle pour la suite.

Exemple 39:
**[(chloro-4-benzyl)-1 méthoxy-5 benzimidazolyl-2]-4 diméthyl -3,3-butanoate d'éthyle**

**Formule (I)** : $R_1 = R_2 = CH_3$, $R_3 = R_4 = H$, n = 1, D = $CO_2$ Et, B = $CH_2$, A = phényl
$X_1$ = 5-MeO, $X_2$ = H, $X_3$ = 4-Cl, $X_4$ = H
Huile utilisée telle quelle pour la suite.

Exemple 40:
**(benzimidazolyl-2)-4 diméthyl-3,3- butanoate d'éthyle**

**Formule (XI)** : B = $CH_2$, $R_1 = R_2 = CH_3$, $R_3 = R_4 = H$, n = 1, D = $CO_2$Et, $X_1 = X_2 = H$

Une solution de 139,2g de chlorure d'acide-ester éthylique de l'acide diméthyl-3,3-glutarique, préparé à l'exemple 28, dans 125 ml de chloroforme stabilisé à l'amylène, est coulée goutte à goutte à une température comprise entre 5 °C et 10 °C sur une solution de 72,8 g d'ortho phénylène diamine et 112 ml de triéthylamine dans 1 l de tétrahydrofurane anhydre. Le mélange est agité à 0 °C pendant deux heures puis à 50 °C pendant une heure ; les cristaux sont éliminés par filtration et les solvants sont évaporés sous vide. Le résidu est repris dans 4,4 l d'éthanol et 444 ml d'acide chlorhydrique concentré et le mélange est porté au reflux pendant 12 heures. Les solvants sont évaporés et le résidu est repris à l'eau puis neutralisé par une solution 1N de soude et extrait à l'éther. La phase éthérée est séchée puis évaporée sous vide pour donner 99 g de (benzimidazolyl-2)-4 diméthyl-3,3-butanoate d'éthyle sous forme de cristaux de point de fusion 123 °C.
Selon le même mode opératoire les exemples suivants ont été préparés.

Exemple 41 :
**(dichloro-5,6-benzimidazolyl-2)-4 diméthyl-3,3- butanoate d'éthyle**

**Formule (XI)** : B = $CH_2$, $R_1 = R_2 = CH_3$, $R_3 = R_4 = H$, n = 1, D = $CO_2$Et, $X_1$ = 5-Cl, $X_2$ = 6-Cl
Cristaux de point de fusion 128 °C.

Exemple 42:
**(benzimidazolyl-2)-4 méthyl-3 éthyl-3 butanoate d'éthyle**

**Formule (XI)** : B = $CH_2$, $R_1 = CH_3$, $R_2 = C_2H_5$, $R_3 = R_4 = H$, n = 1, D = $CO_2$Et, $X_1 - X_2 = H$
Huile utilisée telle quelle pour la suite.

Exemple 43:
**(benzimidazolyl-2)-4 diéthyl-3,3- butanoate d'éthyle**

**Formule (XI)** : B = $CH_2$, $R_1 = R_2 = C_2H_5$, $R_3 = R_4 = H$, n = 1, D = $CO_2$Et, $X_1 = X_2 = H$
Cristaux de point de fusion 81 °C.

Exemple 44 :
**(benzimidazolyl-2)-4 méthyl-3 butanoate d'éthyle**

**Formule (XI)** : B = $CH_2$, $R_1 = CH_3$, $R_2 = R_3 = R_4 = H$, n = 1, D = $CO_2$Et, $X_1 = X_2 = H$
Cristaux de point de fusion 105 °C.

Exemple 45 :
**[(chloro-4 benzyl)-1 benzimidazolyl-2]-4 diméthyl-3,3 butanoate d'éthyle**

**Formule (I)** : $R_1 = R_2 = CH_3$, $R_3 = R_4 = H$, n = 1, D = $CO_2$ Et, A = phényl, B = $CH_2$ $X_1$ = H,
$X_2 = X_3 = H$, $X_4$ = 4-Cl

9g de (benzimidazolyl-2)-4 diméthyl-3,3- butanoate d'éthyle préparé à l'exemple 40 sont ajoutés à une suspension de 21,5 g d'hydrure de sodium à 60% dans 50 ml de diméthylformamide anhydre. Le mélange est agité 1 heure a 50 °C puis 5,6g de chloro-4 chlorure de benzyle sont rajoutés et la solution obtenue est chauffée 5 heures à 90 °C. Les solvants sont concentrés sous vide et le résidu est repris à l'eau puis extrait

à l'éther. La phase éthérée est lavée à l'eau puis séchée sur sulfate de magnésium et l'éther est évaporé à sec pour donner 12,9g de [(chloro-4 benzyl)-1 benzimidazolyl-2]-4 diméthyl-3,3-butanoate d'éthyle sous forme d'huile utilisée telle quelle pour la suite.
Selon le même mode opératoire les exemples suivants ont été préparés :

Exemple 46 :
**[(fluoro-2 bromo-4 benzyl)-1 benzimidazolyl-2]-4 diméthyl-3,3- butanoate d'éthyle**

**Formule (I)** : $R_1 = R_2 = CH_3$, $R_3 = R_4 = H$, $n = 1$, $D = CO_2$ Et, A = phényl, $B = CH_2$, $X_1 = H$, $X_2 = H$, $X_3 =$ 2-F, $X_4 =$ 4-Br
Huile utilisée telle quelle pour la suite.

Exemple 47 :
**[ benzyl -1 benzimidazolyl-2]-4 diméthyl-3,3- butanoate d'éthyle**

**Formule (I)** : $R_1 = R_2 = CH_3$, $R_3 = R_4 = H$, $n = 1$, $D = CO_2$ Et,
A = phényl, $B = CH_2$, $X_1 = X_2 = X_3 = X_4 = H$
Huile utilisée telle quelle pour la suite.

Exemple 48 :
**[( méthyl-4 benzyl) -1 benzimidazolyl-2]-4 diméthyl-3,3- butanoate d'éthyle**

**Formule (I)** : $R_1 = R_2 = CH_3$, $R_3 = R_4 = H$, $n = 1$, $D = CO_2$ Et, $B = CH_2$,
A = phényl, $X_1 = X_2 = X_3 = H$, $X_4 =$ 4-Me
Huile utilisée telle quelle dur la suite.

Exemple 49 :
**[( fluoro-4 benzyl) -1 benzimidazolyl-2]-4 diméthyl-3,3- butanoate d'éthyle**

**Formule (I)** : $R_1 = R_2 = CH_3$, $R_3 = R_4 = H$, $n = 1$, $D = CO_2$ Et,
A = phényl, $B = CH_2$, $X_1 = X_2 = X_3 = H$, $X_4 =$ 4-F
Huile utilisée telle quelle dur la suite.

Exemple 50 :
**[( méthoxy-4 benzyl) -1 benzimidazolyl-2]-4 dlméthyl-3,3- butanoate d'éthyle**

**Formule (I)** : $R_1 = R_2 = CH_3$, $R_3 = R_4 = H$, $n = 1$, $D = CO_2$ Et,
$B = CH_2$, A = phényl, $X_3 =$ 4-MeO, $X_1 = X_2 = X_4 = H$
Huile utilisée telle quelle dur la suite.

Exemple 51 :
**[( bromo-4 benzyl) -1 benzimidazolyl-2]-4 diméthyl-3,3- butanoate d'éthyle**

**Formule (I)** : $R_1 = R_2 = CH_3$, $R_3 = R_4 = H$, $n = 1$, $D = CO_2$ Et, $B = CH_2$,
A = phényl, $X_3 =$ 4-Br, $X_1 = X_2 = X_4 = H$
Huile utilisée telle quelle dur la suite.

Exemple 52 :
**[( chloro-4 benzyl) -1 dichloro-5,6- benzimidazolyl-2]-4 diméthyl-3,3-butanoate d'éthyle**

**Formule (I)** : $R_1 = R_2 = CH_3$, $R_3 = R_4 = H$, $n = 1$,
$D = CO_2$ Et, $B = CH_2$, A = phényl, $X_1 =$ 5-Cl, $X_2 =$ 6-Cl, $X_3 =$ 4 Cl, $X_4 = H$
Huile utilisée telle quelle pour la suite.

Exemple 53 :
**[( trifluorométhyl-3 benzyl) -1 benzimidazolyl-2]-4 diméthyl-3,3- butanoate d'éthyle**

**Formule (I)** : $R_1 = R_2 = CH_3$, $R_3 = R_4 = H$, n = 1, D = $CO_2$ Et, B = $CH_2$, A = phényl, $X_1$ = H, $X_2 = X_3$ = H, $X_4$ = 3-$CF_3$
Huile utilisée telle quelle dur la suite.

Exemple 54 ;
**[( chloro-4 benzyl) -1 benzimidazolyl-2]-4 méthyl-3 éthyl-3 butanoate d'éthyle**

**Formule (I)** : $R_1 = CH_3$, $R_2 = C_2H_5$, $R_3 = R_4 = H$, n = 1, D = $CO_2$ Et, B = $CH_2$, A = phényl, $X_1$ = H, $X_2 = X_3$ = H, $X_4$ = 4-Cl
Huile utilisée telle quelle pur la suite.

Exemple 55 :
**[( chloro-4 benzyl) -1 benzimidazolyl-2]-4 diéthyl-3,3- butanoate d'éthyle**

**Formule (I)** : $R_1 = R_2 = C_2H_5$, $R_3 = R_4 = H$, n = 1, D = $CO_2$ Et, B = $CH_2$, A = phényl, $X_1$ = H, $X_2$ = H, $X_3$ = 4-Cl, $X_4$ = H
Huile utilisée telle quelle pur la suite.

Exemple 56 :
**[( chloro-4 benzyl) -1 benzimidazolyl-2]-4 méthyl-3- butanoate d'éthyle**

**Formule (I)** : $R_1 = CH_3$, $R_2$ = H, $R_3 = R_4 = H$, n = 1, D = $CO_2$ Et, B = $CH_2$, A = phényl, $X_1 = X_2 = X_3$ = H, $X_4$ = 4-Cl
Huile utilisée telle quelle pour la suite.

Exemple 57 :
**[( naphthyl -2 méthyl) -1 benzimidazolyl-2]-4 diméthyl-3,3- butanoate d'éthyle**

**Formule (I)** : $R_1 = R_2 = CH_3$, $R_3 = R_4 = H$, n = 1, D = $CO_2$ Et, B = $CH_2$, A = phényl, $X_3 + X_4$ forment un noyau phényl en position -3,4-, $X_1 = X_2$ = H
Huile utilisée telle quelle pour la suite.

Exemple 58 :
**acide ( benzyl -1 benzimidazolyl-2)-4 diméthyl-3,3- butanoïque**

**Formule (I)** : $R_1 = R_2 = CH_3$, $R_3 = R_4 = H$, n = 1, D = $CO_2$ H, B = $CH_2$, A = phényl, $X_1 = X_2 = X_3 = X_4$ = H

9g de (benzyl -1 benzimidazolyl-2)-4 diméthyl-3,3- butanoate d'éthyle préparé à l'exemple 47 sont dissous dans un mélange composé de 90 ml d'acide chlorhydrique concentré, 270 ml d'eau et 250 ml d'acide acétique. Le mélange est porté au reflux pendant 4 heures et les solvants sont concentrés sous vide. Le résidu est repris avec une solution de soude 1N et lavé à l'éther ; la phase aqueuse est acidifiée par barbottage de dioxyde de soufre jusqu'à pH = 5-6, les cristaux formés sont essorés et lavés à l'eau et à l'éther isopropylique pour donner 5,3g d'acide (benzyl-1 benzimidazolyl-2)-4 diméthyl-3,3 butanoïque sous forme de cristaux de point de fusion 160-1 °C.
Selon le même mode opératoire les composés suivants ont été préparés.

Exemple 59 :
**acide [( chloro-4 benzyl)-1 fluoro-5 benzimidazolyl-2] mercapto-5 pentanoïque**

**Formule (I)** : $R_1 = R_2 = R_3 = R_4$ = H, n = 3, D = $CO_2$ H, B = S, A = phényl, $X_1$ = 5-F, $X_2 = X_3$ = H, $X_4$ = Cl
Cristaux de point de fusion 184-186 °C.

Exemple 60 :
**acide [( fluoro-2 bromo-4 benzyl)-1 chloro-5 imidazo[4,5-b] pyridine yl-2] mercapto-4 butanoïque**

**Formule (I)** : $R_1 = R_2 = R_3 = R_4 = H$, $n = 2$, $D = CO_2H$, $B = S$, A = pyridine, $X_1$ = 5-Cl, $X_2 = H$, $X_3$ = 2-F, $X_4$ = 4-Br
Cristaux de point de fusion 156-158 ° C.

Exemple 61 :
**acide [( chloro-4 benzyl)-1 chloro-5 imidazo[4,5-b] pyridine yl-2] mercapto-2 méthyl-2 propanoïque**

**Formule (I)** : $R_1 = R_2 = CH_3$, $n = 0$, $D = CO_2H$, $B = S$, A = pyridine, $X_1$ = 5-Cl, $X_2 = H$, $X_3$ = 4-Cl, $X_4 = H$
Cristaux de point de fusion 188-189 ° C.

Exemple 62 :
**acide [( chloro-4 benzyl)-1 imidazo[4,5-b] pyridine yl-2] mercapto-4 butanoïque**

**Formule (I)** : $R_1 = R_2 = R_3 = R_4 = H$, $n = 2$, $D = CO_2H$, $B = S$, A = pyridine, $X_1 = X_2 = H$, $X_3$ = 4-Cl, $X_4 = H$
Cristaux de point de fusion 121-122 ° C.

Exemple 63 :
**acide [( chloro-4 benzyl)-1 benzimidazolyl-2] mercapto-4 butanoïque**

**Formule (I)** : $R_1 = R_2 = R_3 = R_4 = H$, $n = 2$, $D = CO_2H$, $B = S$, A = phényl, $X_1 = X_2 = X_3 = H$, $X_4$ = 4-Cl
Cristaux de point de fusion 187-190 ° C.

Exemple 64 :
**acide [( dichloro-2,4- benzyl)-1 benzimidazolyl-2] mercapto-4 butanoïque**

**Formule (I)** : $R_1 = R_2 = R_3 = R_4 = H$, $n = 2$, $D = CO_2H$, $B = S$, A = phényl, $X_1 = X_2 = H$, $X_3$ = 2-Cl, $X_4$ = 4-Cl
Cristaux de point de fusion 117-120 ° C.

Exemple 65 :
**acide [( chloro-4 benzyl)-1 fluoro-5 benzimidazolyl-2] mercapto-4 butanoïque**

**Formule (I)** : $R_1 = R_2 = R_3 = R_4 = H$, $n = 2$, $D = CO_2H$, $B = S$, A = phényl, $X_1$ = 5-F, $X_2 = X_3 = H$, $X_4$ = 4-Cl
Cristaux de point de fusion 176-178 ° C.

Exemple 66 :
**acide [( méthyl-4 benzyl) -1 benzimidazolyl-2]-4 diméthyl-3,3- butanoïque**

**Formule (I)** : $R_1 = R_2 = CH_3$, $R_3 = R_4 = H$, $n = 1$, $D = CO_2H$, $B = CH_2$, A = phényl, $X_1 = X_2 = X_3 = H$, $X_4$ = 4-Me
Cristaux de point de fusion 147-148 ° C.

Exemple 67 :
**acide [( fluoro-4 benzyl) -1 benzimidazolyl-2]-4 diméthyl-3,3- butanoïque**

**Formule (I)**: $R_1 = R_2 = CH_3$, $R_3 = R_4 = H$, $n = 1$, $D = CO_2H$, $B = CH_2$, A = phényl $X_1 = X_2 = X_3 = H$, $X_4$ = 4-F
Cristaux de point de fusion 180-181 ° C.

Exemple 68 :
**acide [( méthoxy-4 benzyl) -1 benzimidazolyl-2]-4 diméthyl-3,3-butanoïque**

**Formule (I)** : $R_1 = R_2 = CH_3$, $R_3 = R_4 = H$, n = 1, D = $CO_2H$, B = $CH_2$, A = phényl
$X_1 = X_2 = X_3 = H$, $X_4$ = 4-MeO
Cristaux de point de fusion 149-150 °C.

Exemple 69 :
**acide [( bromo-4 benzyl) -1 benzimidazolyl-2]-4 diméthyl-3,3- butanoïque**

**Formule (I)** : $R_1 = R_2 = CH_3$, $R_3 = R_4 = H$, n = 1, D = $CO_2H$, B = $CH_2$, A = phényl,
$X_1 = X_2 = X_3 = H$, $X_4$ = 4-Br
Cristaux de point de fusion 171-172 °C.

Exemple 70 :
**acide [( chloro-4 benzyl) -1 dichloro-5,6- benzimidazolyl-2]-4 diméthyl-3,3-butanoïque**

**Formule (I)** : $R_1 = R_2 = CH_3$, $R_3 = R_4 = H$, n = 1, D = $CO_2H$, B = $CH_2$, A = phényl, $X_1$ = 5-Cl,
$X_2$ = 6-Cl, $X_3$ = H, $X_4$ = 4-Cl
Cristaux de point de fusion 197-199 °C.

Exemple 71 :
**acide [( trifluorométhyl-3 benzyl) -1 benzimidazolyl-2]-4 diméthyl-3,3-butanoïque**

**Formule (I)** : $R_1 = R_2 = CH_3$, $R_3 = R_4 = H$, n = 1, D = $CO_2H$, B = $CH_2$, A = phényl,
$X_1 = X_2 = X_3 = H$, $X_4$ = 3-$CF_3$
Cristaux de point de fusion 163-164 °C.

Exemple 72 :
**acide [[( chloro-4 benzyl) -1 fluoro-5 benzimidazolyl-2]méthyl-1 cyclopentyl-1] acétique**

**Formule (I)** : $R_1 + R_2 = CH_2\ CH_2\ CH_2\ CH_2$, $R_3 = R_4 = H$, n = 1, D = $CO_2H$, B = $CH_2$,
A = phényl, $X_1$ = 5-F, $X_2 = X_3 = H$, $X_4$ = 4-Cl
Cristaux de point de fusion 164-165 °C.

Exemple 73 :
**acide [[( chloro-4 benzyl) -1 fluoro-5 benzimidazolyl-2]méthyl-1 cyclohexyl-1] acétique**

**Formule (I)** : $R_1 + R_2 = CH_2\ CH_2\ CH_2\ CH_2\ CH_2$, $R_3 = R_4 = H$, n = 1, D = $CO_2H$, B = $CH_2$,
A = phényl, $X_1$ = 5-F, $X_2 = X_3 = H$, $X_4$ = 4-Cl
Cristaux de point de fusion 182-184 °C.

Exemple 74 :
**acide [( chloro-4 benzyl) -1 fluoro-5 benzimidazolyl-2]-4 diméthyl-3,3-butanoïque**

**Formule (I)** : $R_1 = R_2 = CH_3$, $R_3 = R_4 = H$, n = 1, D = $CO_2H$, B = $CH_2$, A = phényl, $X_1$ = 5-F,
$X_2 = X_3 = H$, $X_4$ = 4-Cl
Cristaux de point de fusion 164-165 °C.

Exemple 75 :
**acide [( chloro-4 benzyl) -1 benzimidazolyl-2]-4 méthyl-3- éthyl-3 butanoïque**

**Formule (I)** : $R_1 = C_2H_5$, $R_2 = CH_3$, $R_3 = R_4 = H$, n = 1, D = $CO_2H$, B = $CH_2$, A = phényl,
$X_1 = X_2 = X_3 = H$, $X_4$ = 4-Cl
Cristaux de point de fusion 120-123 °C.

Exemple 76 :
**acide [( fluoro-2 bromo-4 benzyl) -1 benzimidazolyl-2]-4 diméthyl-3,3-butanoïque**

**Formule (I)** : $R_1 = R_2 = CH_3$, $R_3 = R_4 = H$, n = 1, D = $CO_2$ H, B = $CH_2$, A = phényl, $X_1 = X_2 = H$, $X_3$ = 2-F, $X_4$ = 4-Br
Cristaux de point de fusion 147-148 °C.

Exemple 77 :
**acide [( chloro-4 benzyl) -1 benzimidazolyl-2]-4 diméthyl-3,3- butanoïque**

**Formule (I)** : $R_1 = R_2 = CH_3$, $R_3 = R_4 = H$, n = 1, D = $CO_2$ H, B = $CH_2$, A = phényl, $X_1 = X_2 = X_3 = H$, $X_4$ = 4-Cl
Cristaux de point de fusion 170-171 °C.

Exemple 78 :
**acide [( chloro-4 benzyl) -1 méthoxy-5 benzimidazolyl-2]-4 diméthyl-3,3-butanoïque**

**Formule (I)** : $R_1 = R_2 = CH_3$, $R_3 = R_4 = H$, n = 1, D = $CO_2$ H, B = $CH_2$, A = phényl, $X_1$ = 5-MeO, $X_2 = X_3 = H$, $X_4$ = 4-Cl
Cristaux de point de fusion 174-176 °C.

Exemple 79 :
**acide [( chloro-4 benzyl) -1 chloro-5 benzimidazolyl-2]-4 diméthyl-3,3-butanoïque**

**Formule (I)** : $R_1 = R_2 = CH_3$, $R_3 = R_4 = H$, n = 1, D = $CO_2$ H, B = $CH_2$, A = phényl, $X_1$ = 5-Cl, $X_2 = X_3 = H$, $X_4$ = 4-Cl
Cristaux de point de fusion 205-207 °C.

Exemple 80 :
**acide [( dichloro-3,4- benzyl) -1 fluoro-5 benzimidazolyl-2]-4 diméthyl-3,3-butanoïque**

**Formule (I)** : $R_1 = R_2 = CH_3$, $R_3 = R_4 = H$, n = 1, D = $CO_2$ H, B = $CH_2$, A = phényl, $X_1$ = 5-F, $X_2$ = H, $X_3$ = 3-Cl, $X_4$ - 4-Cl
Cristaux de point de fusion 177-180 °C.

Exemple 81 :
**acide [( nitro-4 benzyl) -1 benzimidazolyl-2]-4 diméthyl-3,3- butanoïque**

**Formule (I)** : $R_1 = R_2 = CH_3$, $R_3 = R_4 = H$, n = 1, D = $CO_2$ H, B = $CH_2$, A = phényl $X_1 = X_2 = X_3 = H$, $X_4$ = 4-$NO_2$
Cristaux de point de fusion 192-194 °C.

Exemple 82 :
**acide [( chloro-4 benzyl) -1 benzimidazolyl-2]-4 diéthyl-3,3- butanoïque**

**Formule (I)** : $R_1 = R_2 = C_2H_5$, $R_3 = R_4 = H$, n = 1, D = $CO_2$ H, B = $CH_2$, A = phényl, $X_1 = X_2 = X_3 = H$, $X_4$ = 4-Cl
Cristaux de point de fusion 139-140 °C.

Exemple 83 :
**acide [( chloro-4 benzyl) -1 benzimidazolyl-2]-4 méthyl-3 butanoïque**

**Formule (I)** : $R_1$ = H, $R_2 = CH_3$, $R_3 = R_4 = H$, n = 1, D = $CO_2$ H, B = $Ch_2$, A = phényl, $X_1 = X_2 = X_3 = H$, $X_4$ = 4-Cl
Cristaux de point de fusion 201-202 °C.

Exemple 84 :

**acide [( naphthyl-2 méthyl)-1 benzimidazolyl-2]-4 diméthyl-3,3-butanoïque**

**Formule (I)** : A = phényl, B = $CH_2$, D = $CO_2$ H, $R_1$ = $R_2$ = $CH_3$, $R_3$ = $R_4$ = H, n = 1, $X_1$ = $X_2$ = H, $X_3$ + $X_4$ forment un noyau phényl en position 3 et 4.
Cristaux de point de fusion 147-149 ° C.

Exemple 85 :

**[(chloro-4 benzyl)-1 benzimidazolyl-2]-4 diméthyl-3,3- butanamide.**

**Formule (I)**: $R_1$ = $R_2$ = $CH_3$, $R_3$ = $R_4$ = H, n = 1, B = $CH_2$, D = $CONH_2$ A = phényl, $X_3$ = 4-Cl, $X_1$ = $X_2$ = $X_4$ = H

11,7g d'acide [(chloro-4 benzyl)-1 benzimidazolyl-2]-4 diméthyl-3,3- butanoïque préparé a l'exemple 77 sont ajoutés dans 100 ml de toluene anhydre et 3 ml de chlorure de thionyle. Le mélange est chauffé à 80 ° C pendant 4 heures et les solvants sont évaporés sous vide. Le résidu est repris dans 50 ml de chloroforme stabilisé à l'amylène et ajouté goutte à goutte dans 50 ml d'hydroxyde d'ammonium à 28%. Après la fin de l'addition, le mélange est agité à température ambiante durant 1 heure 30 puis décanté. La phase organique est séchée sur sulfate de magnésium et le solvant est évaporé à sec sous vide. Le résidu cristallise dans l'éther isopropylique et est recristallisé dans l'acétonitrile pour donner 5,1g de [(chloro-4 benzyl)-1 benzimidazolyl-2]-4 diméthyl-3,3-butanamide sous forme de cristaux de point de fusion 163-165 ° C.

Exemple 86 :

**[(chloro-4 benzyl)-1 benzimidazolyl-2]-4 diméthyl-3,3- butyronitrile**

**Formule (I)** : $R_1$ = $R_2$ = $CH_3$, $R_3$ = $R_4$ = H, n = 1,B = $CH_2$, D = CN A = phényl, $X_3$ = 4-Cl, $X_1$ = $X_2$ = $X_4$ = H

2,7g de [(chloro-4 benzyl)-1 benzimidazolyl-2]-4 diméthyl-3,3- butanamide sont dissous dans 50 ml de chloroforme. On ajoute 2,3 ml d'oxychlorure de phosphore et le mélange est chauffé 5 heures au reflux. Après refroidissement les solvants sont évaporés sous vide et le résidu, repris à l'eau, est extrait à l'acétate d'éthyle. La phase organique est séchée sur sulfate de magnésium et évaporée à sec sous vide pour donner une huile qui cristallise dans l'éther. Les cristaux sont essorés et lavés à l'éther, puis séchés pour donner 2,5g de [(chloro-4 benzyl)-1 benzimidazolyl-2]-4 diméthyl-3,3- butyronitrile sous forme de cristaux de point de fusion 110 ° C.

Exemple 87 :

**acide [(hydroxy-4 benzyl)-1 benzimidazolyl-2]-4 diméthyl-3,3- butanoïque**

**Formule (I)**: $R_1$ = $R_2$ = $CH_3$, $R_3$ = $R_4$ = H, n = 1, B = $CH_2$, D = $CO_2H$, A=phényl, $X_4$ = 4-OH, $X_1$ = $X_2$ = $X_3$ = H

2g d'acide [(méthoxy-4 benzyl)-1 benzimidazolyl-2]-4 diméthyl-3,3 butanoïque, préparé à l'exemple 68, sont dissous dans 40 ml d'acide acétique et 40 ml d'acide bromhydrique à 48%. Le mélange est chauffé au reflux 3 heures et les solvants sont évaporés sous vide. Le résidu est repris avec une solution de soude 1N de façon à ajuster le pH à 9-10 et la phase aqueuse ainsi obtenue est lavée à l'éther puis acidifiée par le dioxyde de soufre à pH = 5,5. Les cristaux obtenus sont essorés, lavés à l'eau puis à l'éther, puis chromatographiés sur gel de silice dans un éluant chloroforme 9/méthanol-1 pour donner 0,4g d'acide [-(hydroxy-4 benzyl)-1 benzimidazolyl-2]-4 diméthyl-3,3-butanoïque sous forme de cristaux de point de fusion 215-216 ° C.

Exemple 88 :

**chlorure d'acide-ester éthylique de l'acide trans-cyclobutane -1,2-dicarboxylique**

**Formule (VIII)** : $R_1$ + $R_5$ = $CH_2$-$CH_2$, $R_2$ = $R_6$ = H, D = $CO_2Et$, n = O

14,2g de trans-cyclobutane -1,2- dicarboxylate d'éthyle sont dissous dans 100 ml d'éthanol et 2,8g de soude en pastille sont ajoutés ainsi que 30 ml d'eau. Le mélange est chauffé au reflux 1 heure et les solvants sont évaporés sous vide. Le résidu est repris à l'eau et lavé à l'éther. La phase aqueuse est acidifiée avec de l'acide chlorhydrique dilué et extraite à l'éther. La phase éthérée est séchée sur sulfate de magnésium et évaporée sous vide pour donner 7,5g d'acide-ester éthylique de l'acide trans-cyclobutane

-1,2-dicarboxylique. A ces 7,5g sont rajoutés 6 ml de chlorure de thionyle et 50 ml de toluène et le mélange est porté deux heures au reflux. Les solvants sont évaporés à sec pour donner 9g de chlorure d'acide-ester éthylique de l'acide trans-cyclobutane -1,2- dicarboxylique sous forme d'huile utilisée telle quelle pour la suite.

Exemple 89 :
**trans-[(chloro-4 benzyl)-1 fluoro-5 benzimidazolyl-2]-2 cyclobutane-1 carboxylate d'éthyle**

**Formule (I)** : B = $CR_5R_6$, $R_1$ + $R_5$ = $CH_2$ $CH_2$, $R_2$ = $R_6$ = H, n = O, D = $CO_2Et$,
A = phényl, $X_1$ = 5-F, $X_2$ = H, $X_3$ = 4-Cl, $X_4$ = H
Préparé selon le mode opératoire de l'exemple 34.
Huile utilisée telle quelle pour la suite.

Exemple 90 :
**Acide trans-[(chloro-4 benzyl)-1 fluoro-5 benzimidazolyl-2]-2 cyclobutane-1 carboxylique.**

**Formule (I)** : B = $CR_5R_6$, $R_1$ + $R_5$ = $CH_2$ $CH_2$, $R_2$ = $R_6$ = H, n = O, D = $CO_2H$,
A = phényl, $X_1$ = 5-F, $X_2$ = H, $X_3$ = 4-Cl, $X_4$ = H
Préparé selon le mode opératoire de l'exemple 58.
Cristaux de point de fusion 173-175 ° C.

Exemple 91 :
**[(chloro-4phényl méthyl)-1 chloro-5 imidazo [4,5-b] pyridine yl-2]-4 diméthyl-3,3-butanoate d'éthyle**

**Formule (I)** : $R_1$ = $R_2$ = $CH_3$, $R_3$ = $R_4$ = H, n = 1, D = COOEt, A = pyridine-2, B = $CH_2$,
$X_1$ = 5-Cl, $X_2$ = $X_3$ = H, $X_4$ = 4-Cl

16,5 g de [(chloro-4 benzyl) amino]-2 amino-3 chloro-5 pyridine, préparée à l'exemple 14, sont dissous dans un mélange constitué de 25 ml d'éthanol et 25 ml d'acide acétique. 12,1 g de formyl-4 diméthyl-3,3-butanoate d'éthyle sont ajoutés et le mélange est agité 4 heures à température ambiante. Les solvants sont évaporés à sec sous vide et le résidu est dissous dans 200 ml de diméthoxy-1,2-éthane. 20 g d'iode sont ajoutés et la solution est chauffée 16 heures à 50 ° C. Le solvant est ensuite évaporé à sec sous vide, le résidu est repris à l'eau et extrait à l'éther. La phase éthérée est lavée à l'eau, séchée sur sulfate de magnésium et évaporée. L'huile obtenue est chromatographiée sur gel de silice dans un éluant cyclohexane 7 / Acétate d'éthyle 3 pour donner 10 g de [(chloro-4phényl méthyl)-1 chloro-5 imidazo [4,5-b] pyridine yl-2]-4 diméthyl-3,3-butanoate d'éthyle sous forme d'une huile utilisée telle quelle pour la suite.

Préparation du formyl-4 diméthyl-3,3 butanoate d'éthyle :

40 g du chlorure d'acide-ester éthylique de l'acide diméthyl-3,3-glutarique préparé à l'exemple 28, sont dissous dans 400 ml de tétrahydrofurane. 2 g de palladium sur charbon à 5 % et 22,8 ml de lutidine-2,6-sont ajoutés et le mélange est hydrogéné à pression normale et température ambiante. Quand la prise d'hydrogène a cessé, le catalyseur est filtré, le solvant est évaporé sous vide. Le résidu est repris à l'eau et extrait à l'éther. La phase éthérée est lavée avec une solution d'acide chlorhydrique dilué à froid, lavée avec une solution de bicarbonate de soude à froid, séchée sur sulfate de magnésium et évaporée sous vide pour donner, après distillation du résidu, 18 g de formyl-4 diméthyl-3,3 butanoate d'éthyle sous forme d'un liquide de point d'ébullition $Eb^{20}$ = 112-118 ° C.

Exemple 92 :
**Acide [(chloro-4 phénylméthyl)-1 chloro-5 imidazo [4,5-b] pyridine yl-2]-4 diméthyl-3,3 butanoïque**

**Formule (I)** : $R_1$ = $R_2$ = $CH_3$, $R_3$ = $R_4$ = H, n = 1, D = COOH, A = pyridine-2, B = $CH_2$,
$X_1$ = 5-Cl, $X_2$ = $X_3$ = H, $X_4$ = 4-Cl

Suivant le mode opératoire de l'exemple 58, mais à partir du [(chloro-4 phényl méthyl)-1 chloro-5 imidazo [4,5-b] pyridine yl-2]-4 diméthyl-3,3-butanoate d'éthyle préparé à l'exemple 91, on obtient l'acide [-(chloro-4 phénylméthyl)-1 chloro-5 imidazo [4,5-b] pyridine yl-2]-4 diméthyl-3,3-butanoïque sous forme de cristaux de point de fusion 120-122 ° C.

Exemple 93 :
**(méthylthio-4 benzyl) amino-2 fluoro-5 nitrobenzène**

**Formule (V)** : $X_1$ = 5-F, $X_2$ = $X_3$ = H, $X_4$ = 4-$SCH_3$, A = phényl

24,8 g d'amino-2 fluoro-5 nitrobenzène et 27,6 g de chlorure de (méthylthio-4) benzyle sont mélangés et 14,4 g d'acétate de sodium anhydre et 0,3 g d'iode sont ajoutés. Le mélange est chauffé, sous agitation, à 120°C pendant 12 heures, puis refroidi et repris avec une solution d'acide chlorhydrique dilué et extrait à l'acétate d'éthyle. La phase organique est lavée à l'acide chlorhydrique dilué, puis à l'eau et séchée sur sulfate de magnésium et évaporée à sec. L'huile obtenue cristallise dans l'éther isopropylique pour donner 23,8 g de (méthylthio-4 benzyl) amino-2 fluoro-5 nitrobenzène sous forme de cristaux de point de fusion 117°C.

Exemple 94 :
**(méthylthio-4 benzyl) amino-2 fluoro-5 aniline**

**Formule (IV)** : $X_1$ = 5-F, $X_2$ = $X_3$ = H, $X_4$ = 4-$SCH_3$, A = phényl

Préparé selon le mode opératoire de l'exemple 8.

Cristaux de point de fusion 112°C.

Exemple 95 :
**[(méthylthio-4 benzyl)-1 fluoro-5 benzimidazolyl-2]-4 diméthyl-3,3-butanoate d'éthyle**

**Formule (I)** : $R_1$ = $R_2$ = $CH_3$, $R_3$ = $R_4$ = H, n = 1, D = $CO_2Et$, A = phényl, B = $CH_2$, $X_1$ = 5-F, $X_2$ = $X_3$ = H, $X_4$ = 4-$SCH_3$

Préparé selon le mode opératoire de l'exemple 34.

Huile utilisée telle quelle pour la suite.

Exemple 96 :
**Acide [(méthylthio-4 benzyl)-1 fluoro-5 benzimidazolyl-2]-4 diméthyl-3,3-butanoïque**

**Formule (I)** : $R_1$ = $R_2$ = $CH_3$, $R_3$ = $R_4$ = H, n = 1, D = $CO_2H$, A = phényl, B = $CH_2$, $X_1$ = 5-F, $X_2$ = $X_3$ = H, $X_4$ = 4-$SCH_3$

Préparé selon le mode opératoire de l'exemple 58.

Cristaux de point de fusion 154-155°C.

Exemple 97 :
**Acide [(méthylsulfonyl-4) benzyl-1 fluoro-5 benzimidazolyl-2]-4 diméthyl-3,3-butanoïque**

**Formule (I)** : $R_1$ = $R_2$ = $CH_3$, $R_3$ = $R_4$ = H, n = 1, D = $CO_2H$, A = phényl, B = $CH_2$, $X_1$ = 5-F, $X_2$ = $X_3$ = H, $X_4$ = 4-$SO_2CH_3$

3 g d'acide [(méthylthio-4 benzyl)-1 fluoro-5 benzimidazolyl-2]-4 diméthyl 3,3-butanoïque préparé à l'exemple 96, sont dissous dans 100 ml de méthanol. Le mélange est refroidi à 0°C et 3,8 g d'acide méta chloro perbenzoïque à 70 % sont ajoutés. A la fin de l'addition, le mélange est agité à température ambiante pendant 10 heures. Les cristaux formés sont essorés et lavés au méthanol puis séchés pour donner 2,4 g d'acide [(méthylsulfonyl-4) benzyl-1 fluoro-5 benzimidazolyl-2]-4 diméthyl-3,3-butanoïque sous forme de cristaux de point de fusion 221-222°C.

Exemple 98 :
**(3,4-dichloro benzyl)amino-2 chloro-5 nitrobenzène**

**Formule (V)** : $X_1$ = 5-Cl, $X_2$ = H, $X_3$ = 3-Cl, $X_4$ = 4-Cl, A = phényl

Préparé selon le mode opératoire de l'exemple 3.

Cristaux de point de fusion 129°C.

Exemple 99:
**(3,4-dichloro benzyl)amino-2 chloro-5 aniline**

**Formule (IV)** :$X_1$ = 5-Cl, $X_2$ = H, $X_3$ =3-Cl, $X_4$ = 4-Cl, A = phényl
Préparé selon le mode opératoire de l'exemple 8.
Cristaux de point de fusion 80 ° C.

Exemple 100:
**[(3,4-dichloro benzyl)-1 chloro-5 benzimidazolyl-2]-4 diméthyl-3,3-butanoate d'éthyle**

**Formule (I)** : $R_1$ = $R_2$ = $CH_3$, $R_3$ = $R_4$ = H, n = 1, D = $CO_2Et$, A = phényl, B = $CH_2$, $X_1$ = 5-Cl, $X_2$ = H, $X_3$ = 3-Cl, $X_4$ = 4-Cl
Préparé selon le mode opératoire de l'exemple 34.
Huile utilisée telle quelle pour la suite.

Exemple 101:
**Acide [(3,4-dichloro benzyl)-1 chloro-5 benzimidazolyl-2]-4 diméthyl-3,3-butanoïque**

**Formule (I)** : $R_1$ = $R_2$ = $CH_3$, $R_3$ = $R_4$ = H, n = 1, D = $CO_2H$, A = phényl, B = $CH_2$, $X_1$ = 5-Cl, $X_2$ = H, $X_3$ = 4-Cl, $X_5$ = 3-Cl
Préparé selon le mode opératoire de l'exemple 58.
Cristaux de point de fusion 183-184 ° C.

Exemple 102 :
**(fluoro2-bromo-4benzyl) amino-2 fluoro-5 nitrobenzène**

**Formule (V)** : $X_1$ = 5-F, $X_2$ = H, $X_3$ = 2-F, $X_4$ = 4-Br, A = phényl
Préparé selon le mode opératoire de l'exemple 1.
Cristaux de point de fusion 130 ° C.

Exemple 103 :
**(fluoro2-bromo-4benzyl) amino-2 fluoro-5 aniline**

**Formule (IV)** : $X_1$ = 5-F, $X_2$ = H, $X_3$ = 2-F, $X_4$ = 4-Br, A = phényl
Préparé selon le mode opératoire de l'exemple 8.
Huile utilisée telle quelle pour la suite.

Exemple 104 :
**[(fluoro2- bromo-4 benzyl)-1- fluoro-5 benzimidazolyl-2]-4 diméthyl-3,3-butanoate d'éthyle**

**Formule (I)** : $R_1$ = $R_2$ = $CH_3$, $R_3$ = $R_4$ = H, n = 1, D = $CO_2Et$, A = phényl, B = $CH_2$, $X_1$ = 5-F, $X_2$ = H, $X_3$ = 2-F, $X_4$ = 4-Br
Préparé selon le mode opératoire de l'exemple 34.
Huile utilisée telle quelle pour la suite.

Exemple 105:
**Acide [(fluoro2- bromo-4 benzyl)-1 fluoro-5 benzimidazolyl-2]-4 diméthyl-3,3-butanoïque**

**Formule (I)** : $R_1$ = $R_2$ = $CH_3$, $R_3$ = $R_4$ = H, n = 1, D = $CO_2H$, A = phényl, B = $CH_2$, $X_1$ = 5-F, $X_2$ = H, $X_3$ = 2-F, $X_4$ = 4-Br
Préparé selon le mode opératoire de l'exemple 58.
Cristaux de point de fusion 145-7 ° C.

Exemple 106:
**(bromo-4 benzyl) amino-2 fluoro-5 nitrobenzène**

**Formule (V)** : $X_1$ = 5-F, $X_2$ = H, $X_3$ = 4-Br, $X_4$ - H, A = phényl
Préparé selon le mode opératoire de l'exemple 1.

Cristaux de point de fusion 163 ° C.

Exemple 107:
**(bromo-4 benzyl) amino-2 fluoro-5 aniline**

**Formule (IV)** : $X_1$ = 5-F, $X_2$ = H, $X_3$ = 4-Br, $X_4$ = H, A = phényl
Préparé selon le mode opératoire de l'exemple 8.
Cristaux de point de fusion 97 ° C.

Exemple 108:
**[(bromo-4 benzyl)-1 fluoro-5 benzimidazolyl-2]-4 diméthyl-3,3-butanoate d'éthyle**

**Formule (I)** : $R_1$ = $R_2$ = $CH_3$, $R_3$ = $R_4$ = H, n = 1, D = $CO_2Et$, A = phényl, B = $CH_2$, $X_1$ = 5-F, $X_2$ = $X_3$ = H, $X_4$ = 4-Br
Préparé selon le mode opératoire de l'exemple 34.
Huile utilisée telle quelle pour la suite.

Exemple 109:
**Acide [(bromo-4 benzyl)-1 fluoro-5 benzimidazolyl-2]-4 diméthyl-3,3-butanoïque**

**Formule (I)** : $R_1$ = $R_2$ = $CH_3$, $R_3$ = $R_4$ = H, n = 1, D = $CO_2H$, A = phényl, B = $CH_2$, $X_1$ = 5-F, $X_2$ = $X_3$ = H, $X_4$ = 4-Br
Préparé selon le mode opératoire de l'exemple 58.
Cristaux de point de fusion 172-174 ° C.

Exemple 110:
**(Méthoxy-4 benzyl) amino-2 chloro-5 nitrobenzène**

**Formule (V)** : $X_1$ = 5-Cl, $X_2$ = $X_3$ = H, $X_4$ = 4-$OCH_3$, A = phényl
Préparé selon le mode opératoire de l'exemple 3.
Cristaux de point de fusion 114 ° C.

Exemple 111:
**(Méthoxy-4 benzyl) amino-2 chloro-5 aniline**

**Formule (IV)** : $X_1$ = 5-Cl, $X_2$ = $X_3$ = H, $X_4$ = 4-$OCH_3$, A = phényl
Préparé selon le mode opératoire de l'exemple 8.
Cristaux de point de fusion 108 ° C.

Exemple 112:
**[(Méthoxy-4 benzyl)-1chloro-5 benzimidazolyl-2]-4 diméthyl-3,3-butanoate d'éthyle**

**Formule (I)** : $R_1$ = $R_2$ = $CH_3$, $R_3$ = $R_4$ = H, n = 1, D = $CO_2Et$, A = phényl, B = $CH_2$, $X_1$ =5-Cl , $X_2$ = $X_3$ = H, $X_4$ = 4-$OCH_3$
Préparé selon le mode opératoire de l'exemple 34.
Huile utilisée telle quelle pour la suite.

Exemple 113:
**Acide [(Méthoxy-4 benzyl)-1 chloro-5 benzimidazolyl-2]-4 diméthyl-3,3-butanoïque**

**Formule (I)** : $R_1$ = $R_2$ = $CH_3$, $R_3$ = $R_4$ = H, n = 1, D = $CO_2H$, A = phényl, B = $CH_2$, $X_1$ = 5-Cl, $X_2$ = $X_3$ = H, $X_4$ = 4-$OCH_3$
Préparé selon le mode opératoire de l'exemple 58.
Cristaux de point de fusion 144-145 ° C.

Exemple 114:
**(Méthylthio-4 benzyl) amino-2 chloro-5 nitrobenzène**

**Formule (V)** : $X_1$ = 5-Cl, $X_2$ = $X_3$ = H, $X_4$ = 4-$SCH_3$, A = phényl
Préparé selon le mode opératoire de l'exemple 93.
Cristaux de point de fusion 74 °C.

Exemple 115:
**(Méthylthio-4 benzyl) amino-2 chloro-5 aniline**

**Formule (IV)** : $X_1$ = 5-Cl, $X_2$ = $X_3$ = H, $X_4$ = 4-$SCH_3$, A = phényl
Préparé selon le mode opératoire de l'exemple 8.
Cristaux de point de fusion 131 °C.

Exemple 116:
**[(Méthylthio-4 benzyl)-1 chloro-5 benzimidazolyl-2]-4 diméthyl-3,3 butanoate d'éthyle**

**Formule (I)**: $R_1$ = $R_2$ = $CH_3$, $R_3$ = $R_4$ = H, n = 1, D = $CO_2Et$, A = phényl, B = $CH_2$, $X_1$ = 5-Cl, $X_2$ = $X_3$ = H, $X_4$ = 4-$SCH_3$
Préparé selon le mode opératoire de l'exemple 34.
Huile utilisée telle quelle pour la suite.

Exemple 117:
**Acide [(Méthylthio-4 benzyl)-1 chloro-5 benzimidazolyl-2]-4 diméthyl-3,3-butanoïque**

**Formule (I)**: $R_1$ = $R_2$ = $CH_3$, $R_3$ = $R_4$ = H, n = 1, D = $CO_2H$, A = phényl, B = $CH_2$, $X_1$ = 5-Cl, $X_2$ = $X_3$ = H, $X_4$ = 4-$SCH_3$
Préparé selon le mode opératoire de l'exemple 58.
Cristaux de point de fusion 138-139 °C.

Exemple 118:
**Acide [(Méthylsulfoxy-4 benzyl)-1 chloro-5 benzimidazolyl-2]-4 diméthyl-3,3-butanoïque**

**Formule (I)** : $R_1$ = $R_2$ = $CH_3$, $R_3$ = $R_4$ = H, n = 1, D = $CO_2H$, A = phényl, B = $CH_2$, $X_1$ = 5-Cl, $X_2$ = $X_3$ = H, $X_4$ = 4-$SOCH_3$

5 g d'acide [(méthylthio-4 benzyl)-1 chloro-5 benzimidazolyl-2]-4 diméthyl-3,3-butanoïque préparés à l'exemple 117, sont dissous dans 350 ml de méthanol. La solution est agitée à 0 °C, puis 2,9 g d'acide méta chloro perbenzoïque à 75 % sont ajoutés. Le mélange est agité à froid 30 minutes puis 4 heures à température ambiante. La solution est concentrée à sec et reprise à l'eau et à l'éther, les cristaux formés sont essorés et lavés à l'éther puis chromatographiés sur gel de silice dans un éluant éther 90 / méthanol 10 / acide acétique 0,5 pour donner 2,5 g d'acide [(méthylsulfoxy-4 benzyl)-1 chloro-5 benzimidazolyl-2]-4 diméthyl-3,3 butanoïque sous forme de cristaux de point de fusion 161-2 °C.

Exemple 119:
**Acide [(Méthylsulfonyl-4 benzyl)-1 chloro-5 benzimidazolyl-2]-4 diméthyl-3,3-butanoïque**

**Formule (I)** : $R_1$ = $R_2$ = $CH_3$, $R_3$ = $R_4$ = H, n = 1, D = $CO_2H$, A = phényl, B = $CH_2$, $X_1$ = 5-Cl, $X_2$ = $X_3$ = H, $X_4$ = 4-$SO_2CH_3$
Préparé selon le mode opératoire de l'exemple 97.
Cristaux de point de fusion 228-30 °C.

Exemple 120:
**(Fluoro-2 chloro-4 benzyl) amino-2 chloro-5 nitrobenzène**

**Formule (V)** : $X_1$ = 5-Cl, $X_2$ = H, $X_3$ = 2-F, $X_4$ = 4-Cl, A = phényl
Préparé selon le mode opératoire de l'exemple 3.
Cristaux de point de fusion 130 °C.

Exemple 121:
**(Fluoro-2 chloro-4 benzyl) amino-2 chloro-5 aniline**

**Formule (IV)**: $X_1$ = 5-Cl, $X_2$ = H, $X_3$ = 2-F, $X_4$ = 4-Cl, A = phényl
Préparé selon le mode opératoire de l'exemple 8.
Huile utilisée telle quelle pour la suite.

Exemple 122:
**[(Fluoro-2 chloro-4 benzyl)-1 chloro-5 benzimidazolyl-2]-4 diméthyl-3,3-butanoate d'éthyle**

**Formule (I)**: $R_1$ = $R_2$ = $CH_3$, $R_3$ = $R_4$ = H, n = 1, D = $CO_2Et$, A = phényl, B = $CH_2$, $X_1$ = 5-Cl, $X_2$ = H, $X_3$ = 2-F, $X_4$ = 4-Cl
Préparé selon le mode opératoire de l'exemple 34.
Huile utilisée telle quelle pour la suite.

Exemple 123:
**Acide [(Fluoro-2 chloro-4 benzyl)-1 chloro-5 benzimidazolyl-2]-4 diméthyl-3,3-butanoïque**

**Formule (I)** : $R_1$ = $R_2$ = $CH_3$, $R_3$ = $R_4$ = H, n = 1, D = $CO_2H$, B = $CH_2$ $X_1$ = 5-Cl, $X_2$ = H, $X_3$ = 2-F, $X_4$ = 4-Cl
Préparé selon le mode opératoire de l'exemple 58.
Cristaux de point de fusion 186-188 ° C.

Exemple 124:
**(chloro-4 benzyl) amino-2 bromo-5 nitrobenzène**

**Formule (V)** : $X_1$ = 5-Br, $X_2$ = $X_3$ = H, $X_4$ = 4-Cl, A = phényl
Préparé selon le mode opératoire de l'exemple 3.
Cristaux de point de fusion 118 ° C.

Exemple 125:
**(chloro-4 benzyl) amino-2 bromo-5 aniline**

**Formule (IV)** : $X_1$ = 5-Br, $X_2$ = $X_3$ = H, $X_4$ = 4-Cl, A = phényl
Préparé selon le mode opératoire de l'exemple 8.
Cristaux de point de fusion 149 ° C.

Exemple 126:
**[(chloro-4 benzyl)-1 bromo-5 benzimidazolyl-2]-4 diméthyl-3,3-butanoate d'éthyle**

**Formule (I)**: $R_1$ = $R_2$ = $CH_3$, $R_3$ = $R_4$ = H, n = 1, D = $CO_2Et$, A = phényl, B = $CH_2$, $X_1$ = 5-Br, $X_2$ = $X_3$ = H, $X_4$ = 4-Cl
Préparé selon le mode opératoire de l'exemple 34.
Huile utilisée telle quelle pour la suite.

Exemple 127:
**Acide [(chloro-4 benzyl)-1 bromo-5 benzimidazolyl-2]-4 diméthyl-3,3-butanoïque**

**Formule (I)**: $R_1$ = $R_2$ = $CH_3$, $R_3$ = $R_4$ = H, n = 1, D = $CO_2H$, A = phényl, B = $CH_2$, $X_1$ = 5-Br, $X_2$ = $X_3$ = H, $X_4$ = 4-Cl
Préparé selon le mode opératoire de l'exemple 58.
Cristaux de point de fusion 219-221 ° C.

Exemple 128:
**(méthoxy-4 benzyl) amino-2 fluoro-5 nitrobenzène**

**Formule (V)** : $X_1$ = 5-F, $X_2$ = $X_3$ = H, $X_4$ = 4-OMe, A = phényl
Préparé selon le mode opératoire de l'exemple 1.

Cristaux de point de fusion 106 ° C.

Exemple 129:
**(méthoxy-4 benzyl) amino-2 fluoro-5 aniline**

**Formule (IV)**: $X_1$ = 5-F, $X_2$ = $X_3$ = H, $X_4$ = 4-OMe, A = phényl
Préparé selon le mode opératoire de l'exemple 8.
Cristaux de point de fusion 123 ° C.

Exemple 130:
**[(méthoxy-4 benzyl)-1 fluoro-5 benzimidazolyl-2]-4dimethyl-3,3-butanoate d'éthyle**

**Formule (I)** : $R_1$ = $R_2$ = $CH_3$, $R_3$ = $R_4$ = H, n = 1, D = $CO_2Et$, A = phényl, $X_1$ = 5-F, $X_2$ = $X_3$ = H, $X_4$ = 4-OMe
Préparé selon le mode opératoire de l'exemple 34.
Huile utilisée telle quelle pour la suite.

Exemple 131:
**Acide [(méthoxy-4 benzyl)-1 fluoro-5 benzimidazolyl-2]-4diméthyl-3,3-butanoïque**

**Formule (I)**: $R_1$ = $R_2$ = $CH_3$, $R_3$ = $R_4$ = H, n = 1, D = $CO_2H$, A = phényl, B = $CH_2$, $X_1$ = 5-F, $X_2$ = $X_3$ = H, $X_4$ = 4-OMe
Préparé selon le mode opératoire de l'exemple 58.
Cristaux de point de fusion 137-138 ° C.

Exemple 132:
**(Fluoro-2 bromo-4 benzyl) amino-2 chloro-5 nitrobenzène**

**Formule (V)** : $X_1$ = 6-Cl, $X_2$ = H, $X_3$ = 2-F, $X_4$ = 4-Br, A = phényl
Préparé selon le mode opératoire de l'exemple 3.
Cristaux de point de fusion 130 ° C.

Exemple 133:
**(Fluoro-2 bromo-4 benzyl) amlno-2 chloro-5 aniline**

**Formule (IV)**: $X_1$ = 5-Cl, $X_2$ = H, $X_3$ = 2-F, $X_4$ = 4-Br, A = phényl
Préparé selon le mode opératoire de l'exemple 8.
Cristaux de point de fusion 89 ° C.

Exemple 134:
**[(Fluoro-2 bromo-4 benzyl)-1 chloro-5 benzimidazolyl-2]-4 diméthyl-3,3-butanoate d'éthyle**

**Formule (I)**: $R_1$ = $R_2$ = $CH_3$, $R_3$ = $R_4$ = H, n = 1, D = $CO_2Et$, A = phényl, B = $CH_2$, $X_1$ = 5-Cl, $X_2$ = H, $X_3$ = 2-F, $X_4$ = 4-Br
Préparé selon le mode opératoire de l'exemple 34.
Huile utilisée telle quelle pour la suite.

Exemple 135:
**Acide [(Fluoro-2 bromo-4 benzyl)-1 chloro-5 benzimidazolyl-2]-4 diméthyl-3,3-butanoïque**

**Formule (I)**: $R_1$ = $R_2$ = $CH_3$, $R_3$ = $R_4$ = H, n = 1, D = $CO_2H$, A = phényl, B = $CH_2$, $X_1$ = 5-Cl, $X_2$ = H, $X_3$ = 2-F, $X_4$ = 4-Br
Préparé selon le mode opératoire de l'exemple 58.
Cristaux de point de fusion 176-7 ° C.

Exemple 136:
**(Bromo-4 benzyl) amino-2 chloro-5 nitrobenzène**

**Formule (V)** : $X_1$ = 5-Cl, $X_2$ = $X_3$ = H, $X_4$ = 4-Br, A = phényl
Préparé selon le mode opératoire de l'exemple 3.
Cristaux de point de fusion 136 ° C.

Exemple 137:
**(Bromo-4 benzyl) amino-2 chloro-5 aniline**

**Formule (IV)**: $X_1$ = 5-Cl, $X_2$ = $X_3$ = H, $X_4$ = 4-Br, A = phényl
Préparé selon le mode opératoire de l'exemple 8.
Cristaux de point de fusion 152 ° C.

Exemple 138:
**[(bromo-4 benzyl)-1 chloro-5 benzimidazolyl-2]-4diméthyl-3,3-butanoate d'éthyle**

**Formule (I)**: $R_1$ = $R_2$ = $CH_3$, $R_3$ = $R_4$ = H, n = 1, D = $CO_2Et$, A = phényl, B = $CH_2$, $X_1$ = 5-Cl, $X_2$ = $X_3$ = H, $X_4$ = 4-Br
Préparé selon le mode opératoire de l'exemple 34.
Huile utilisée telle quelle pour la suite.

Exemple 139:
**Acide [(bromo-4 benzyl)-1 chloro-5 benzimidazolyl-2]-4-diméthyl-3,3-butanoïque**

**Formule (I)**: $R_1$ = $R_2$ = $CH_3$, $R_3$ = $R_4$ = H, n = 1, D = $CO_2H$, A = phényl, B = $CH_2$, $X_1$ = 5-Cl, $X_2$ = $X_3$ = H, $X_4$ = 4-Br
Préparé selon le mode opératoire de l'exemple 34.
Huile utilisée telle quelle pour la suite.

Exemple 140:
**(naphthyl-2) méthyl amino-2 chloro-5 nitrobenzène**

**Formule (V)**: $X_1$ = 5-Cl, $X_2$ = H, A = phényl, $X_3$ + $X_4$ forment un noyau phényl en position 3 et 4
Préparé selon le mode opératoire de l'exemple 93.
Cristaux de point de fusion 143 ° C.

Exemple 141:
**(naphthyl-2) méthyl amino-2 chloro-5 aniline**

**Formule (IV)**: $X_1$ = 5-Cl, $X_2$ = H, A = phényl, $X_3$ + $X_4$ forment un noyau phényl en position 3 et 4
Préparé selon le mode opératoire de l'exemple 8.
Cristaux de point de fusion 124 ° C.

Exemple 142:
**[(naphthyl-2) méthyl-1 chloro-5 benzimidazolyl-2]-4 diméthyl-3,3 butanoate d'éthyle**

**Formule (I)**: $R_1$ = $R_2$ = $CH_3$, $R_3$ = $R_4$ = H, n = 1, D = $CO_2Et$, A = phényl, B = $CH_2$, $X_1$ = 5-Cl, $X_2$ = H, $X_3$ et $X_4$ forment un noyau phényl en position 3 et 4.
Préparé selon le mode opératoire de l'exemple 34.
Huile utilisée telle quelle pour la suite.

Exemple 143:
**Acide [(naphthyl-2) méthyl-1 chloro-5 benzimidazolyl-2]-4 diméthyl-3,3 butanoïque**

**Formule (I)**: $R_1$ = $R_2$ = $CH_3$, $R_3$ = $R_4$ = H, n = 1, D = $CO_2H$, A = phényl, B = $CH_2$, $X_1$ = 5-Cl, $X_2$ = H, $X_3$ et $X_4$ forment un noyau phényl en position 3 et 4.
Préparé selon le mode opératoire de l'exemple 58.

Cristaux de point de fusion 168-169 ° C.

Exemple 144:
**(naphthyl-2) méthyl amino-1 fluoro-5 nitrobenzène**

**Formule (V)** : $X_1$ = 5-F, $X_2$ = H, A = phényl, $X_3$ et $X_4$ forment un noyau phényl en position 3 et 4.
Préparé selon le mode opératoire de l'exemple 93.
Cristaux de point de fusion 170 ° C.

Exemple 145:
**(naphthyl-2) méthylamino-1 fluoro-5 aniline**

**Formule (IV)**: $X_1$ = 5-F, $X_2$ = H, A = phényl, $X_3$ et $X_4$ forment un noyau phényl en position 3 et 4.
Préparé selon le mode opératoire de l'exemple 8.
Cristaux de point de fusion 118 ° C.

Exemple 146:
**[(naphthyl-2) méthyl-1 fluoro-5 benzimidazolyl-2]-4diméthyl-3,3-butanoate d'éthyle**

**Formule (I)**: $R_1$ = $R_2$ = $CH_3$, $R_3$ = $R_4$ = H, n = 1, D = $CO_2Et$, A = phényl, B = $CH_2$, $X_1$ = 5-F, $X_2$ = H, $X_3$ et $X_4$ forment un noyau phényl en position 3 et 4.
Préparé selon le mode opératoire de l'exemple 34.
Huile utilisée telle quelle pour la suite.

Exemple 147:
**Acide [(naphthyl-2) méthyl-1 fluoro-5 benzimidazolyl-2]-4-diméthyl-3,3-butanoïque**

**Formule (I)**: $R_1$ = $R_2$ = $CH_3$, $R_3$ = $R_4$ = H, n = 1, D = $CO_2H$, A = phényl, B = $CH_2$, $X_1$ = 5-F, $X_2$ = H, $X_3$ et $X_4$ forment un noyau phényl en position 3 et 4.
Préparé selon le mode opératoire de l'exemple 58.
Cristaux de point de fusion 157-158 ° C.

Exemple 148:
**Chlorure d'acide, ester éthylique de l'acide cyclopropane-1,1-diacétique**

**Formule (VIII)**: $R_5$ = $R_6$ = H, $R_1$ + $R_2$ = $CH_2CH_2$, $R_3$ = $R_4$ = H, n = 1, D = $CO_2Et$,
Préparé selon le mode opératoire de l'exemple 28 à partir de l'anhydride de l'acide cyclopropane-1,1-diacétique.
Huile utilisée telle quelle pour la suite.

Préparation de l'anhydride cyclopropane-1,1 diacétique :

11 g de cyclopropane-1,1-diacétonitrile (dont on peut trouver la préparation dans les références : SEYDEN. PENNE. J ; ROUX. SCHMITT. M.C.; Bull. Soc. Chim. Fr. 1968, 9, 3810-3812 et CHAMBOUX. B ; ETIENNE. Y ; PALLAUD. R ; C.R. Acad. Science. Paris. 1962 ; 255 ; p 536-538) sont additionnés à 150 ml de potasse à 20 % et le mélange est porté au reflux pendant 12 heures. Après refroidissement le mélange est lavé à l'éther et la phase aqueuse est acidifiée par l'acide chlorhydrique puis saturée par du chlorure de sodium et extraite à l'acétate d'éthyle. La phase organique est séchée sur sulfate de magnésium puis évaporée à sec pour donner 10,5 g d'acide cyclopentane-1,1-diacétique de point de fusion 105 ° C. Cet acide est dissous dans 50 ml d'anhydride acétique et le mélange est porté au reflux pendant 5 heures. Le solvant est évaporé à sec pour donner 10,2 g d'anhydride de l'acide cyclopropane-1,1-diacétique de point de fusion 102 ° C.

Exemple 149:
**[ [(chloro-4 benzyl)-1 fluoro-5 benzimidazolyl-2] méthyl-1 cyclopropyl-1] acétate d'éthyle**

**Formule (I)**: $R_1$ + $R_2$ = $CH_2$ $CH_2$, $R_3$ = $R_4$ = H, n = 1, D = $CO_2Et$, A = phényl, B = $CH_2$, $X_1$ = 5-F, $X_2$ = $X_3$ = H, $X_4$ = 4-Cl

Préparé selon le mode opératoire de l'exemple 34, à partir du chlorure d'acide ester de l'acide cyclopropane-1,1-diacétique préparé à l'exemple 148.

Huile utilisée telle quelle pour la suite.

Exemple 150:

**Acide [[chloro-4 benzyl)-1 fluoro-5 benzimidazolyl-2] méthyl-1 cyclopropyl-1] acétique**

**Formule (I)**: $R_1 + R_2 = CH_2\ CH_2$, $R_3 = R_4 = H$, $n = 1$, $D = CO_2H$, $B = CH_2$, A = phényl, $X_1$ = 5-F, $X_2 = X_3 = H$, $X_4$ = 4-Cl

Préparé selon le mode opératoire de l'exemple 58.

Cristaux de point de fusion 181-3 °C.

Exemple 151:

**Chlorure d'acide ester éthylique de l'acide trans-cyclopentane-1,2 dicarboxylique**

**Formule (VIII)**: $R_1 + R_5 = CH_2\ CH_2\ CH_2$, $R_2 = R_6 = H$, $D = CO_2Et$, n = O

Préparé selon le mode opératoire de l'exemple 88.

Huile utilisée telle quelle pour la suite.

Exemple 152:

**trans-[(chloro-4 benzyl)-1 fluoro-5 benzimidazolyl-2]-2 cyclopentane-1 carboxylate d'éthyle**

**Formule (I)**: $B = CR_5R_6$, $R_1 + R_5 = CH_2\ CH_2CH_2$, $R_2 = R_6 = H$, n = O, $D = CO_2Et$, A = phényl, $X_1$ = 5-F, $X_2$ = H, $X_3$ = 4-Cl, $X_4$ = H

Préparé selon le mode opératoire de l'exemple 34 à partir du chlorure d'acide ester éthylique de l'acide trans-cyclopentane-1,2-dicarboxylique préparé à l'exemple 151.

Huile utilisée telle quelle pour la suite.

Exemple 153:

**Acide trans-[(chloro-4 benzyl)-1 fluoro-5 benzimidazolyl-2]-2 cyclopentane-1 carboxylique**

**Formule (I)**: $B = CR_5R_6$, $R_1 + R_5 = CH_2\ CH_2CH_2$, $R_2 = R_6 = H$, n = O, $D = CO_2H$, A = phényl, $X_1$ = 5-F, $X_2$ = H, $X_3$ = 4-Cl, $X_4$ = H

Préparé selon le mode opératoire de l'exemple 58.

Cristaux de point de fusion 210-211 °C.

Exemple 154 :

**[(chloro-4 benzyl)-1 imidazo [4,5-b] pyridine-yl-2]-4 diméthyl-3,3-butanoate d'éthyle**

**Formule (I)**: $R_1 = R_2 = CH_3$, $R_3 = R_4 = H$, $n = 1$, $D = CO_2Et$, A = pyridine-2, $B = CH_2$, $X_1 = X_2 = H$, $X_3$ = H, $X_4$ = 4-Cl

Préparé selon le mode opératoire de l'exemple 91.

Huile utilisée telle quelle pour la suite.

Exemple 155 :

**Acide [(chloro-4 benzyl)-1 imidazo [4,5-b] pyridine-yl-2]-4 diméthyl-3,3-butanoïque**

**Formule (I)**: $R_1 = R_2 = CH_3$, $R_3 = R_4 = H$, $n = 1$, $D = CO_2H$, A = pyridine-2, $B = CH_2$, $X_1 = X_2 = H$, $X_3$ = H, $X_4$ = 4-Cl

Préparé selon le mode opératoire de l'exemple 58.

Cristaux de point de fusion 138-140 °C.

Exemple 156 :

**Formyl-5 diméthyl-4,4-valéronitrile**

**Formule (VIII') :** $R_1 = R_2 = CH_3$, $R_3 = R_4 = R_5 = R_6 = H$, $n = 2$, D = CN

A] (dioxolane-yl-2)-4 diméthyl-3,3-butanoate d'éthyle :

74 g de formyl-4 diméthyl-3,3-butanoate d'éthyle, préparé à l'exemple 91, sont dissous dans 450 ml de toluène anhydre en présence de 0,5 g d'acide para toluène sulfonique et de 26,7 g d'éthylène glycol. Le mélange est porté au reflux et l'eau formée au cours de la réaction est éliminée par l'intermédiaire d'un Dean Stark. Au bout de deux heures de reflux, le solvant est évaporé sous vide et le résidu est distillé pour donner 86,7 g de (dioxolane-yl-2)-4 diméthyl-3,3-butanoate d'éthyle sous forme d'un liquide de point d'ébullition $Eb^{10}$ = 138-140 °C.

B] (dioxolane-yl-2)-4 diméthyl-3,3-butanol

86,7 g de (dioxolane-yl-2)-4 diméthyl-3,3-butanoate d'éthyle préparé ci-dessus sont dissous dans 720 ml d'éther. Cette solution est ajoutée goutte à goutte à 10 °C sur une suspension de 9 g d'hydrure double de lithium et d'aluminium dans 700 ml d'éther. Après la fin de l'addition le mélange est agité 3 heures à température ambiante puis refroidi à 10 °C. Une solution saturée de sulfate de sodium est ajoutée goutte à goutte à cette température jusqu'à l'obtention d'un précipité granuleux que l'on essore. Le filtrat éthéré est évaporé à sec à une température inférieure à 30 °C pour donner 81,9 g de (dioxolane-yl-2)-4 diméthyl-3,3-butanol sous forme d'une huile utilisée telle quelle pour la suite.

C] Mésylate du (dioxolane-yl-2)-4 diméthyl-3,3-butanol

81,9 g du (dioxolane-yl-2)-4 diméthyl-3,3-butanol, préparé ci-dessus, sont dissous dans 600 ml de chloroforme stabilisé à l'amylène en présence de 62 ml de triéthylamine. Le mélange est refroidi à 5 °C et 81 ml de chlorure de mésyle sont ajoutés goutte à goutte. Le mélange est ensuite agité 4 heures à 5 °C, puis laissé une nuit à cette température et lavé avec de l'eau froide et évaporé à 30 °C sous vide. Le résidu obtenu est repris à l'éther et lavé au bicarbonate de soude. La phase éthérée est séchée sur sulfate de magnésium, puis évaporée à 30 °C sous vide pour donner 93,5 g de mésylate du (dioxolane-yl-2)-4 diméthyl-3,3-butanol.

D] (Dioxolane-yl-2)-5 diméthyl-4,4-valéronitrile

93,5 g de mésylate préparé en C] sont dissous dans 500 ml d'acétonitrile. 50 g de cyanure de potassium et 4,8 g d'éther couronne 18-6 sont ajoutés. Le mélange est chauffé au reflux pendant 8 heures puis repris à l'eau et extrait à l'acétate d'éthyle. La phase organique est lavée plusieurs fois à l'eau, puis séchée sur suffate de magnésium et évaporée sous vide. Le résidu obtenu est distillé sous pression réduite pour donner 50 g de (dioxolane-yl-2)-5 diméthyl-4,4-valéronitrile de point d'ébullition $Eb^{0,5}$ = 90-8 °C.

E] Formyl-5 diméthyl-4,4'valéronitrile

50 g de (dioxolane-yl-2)-5 diméthyl-4,4-valéronitrile préparé en D] sont dissous dans 1,3 l d'acétone en présence de 140 ml d'acide chlorhydrique concentré et 700 ml d'eau. Le mélange est agité à température ambiante pendant 5 heures, l'acétone est concentrée sous vide et le résidu repris à l'éther et lavé à l'eau puis évaporé à sec pour donner 35 g de formyl-5 diméthyl-4,4-valéronitrile.

Exemple 157 :
**[(chloro-4 benzyl)-1 fluoro-5 benzimidazolyl-2]-5 dimethyl-4,4-valéronitrile**

**Formule (I)**: $R_1 = R_2 = CH_3$, $R_3 = R_4 = H$, n = 2, D = CN, A = phényl, B = $CH_2$, $X_1$ = 5-F, $X_2 = X_3 = H$, $X_4$ = 4-Cl

Préparé selon le mode opératoire de l'exemple 91 à partir du formyl-5 diméthyl-4,4-valéronitrile préparé à l'exemple 156.

Huile utilisée telle quelle pour la suite.

Exemple 158 :
**Acide [(chloro-4 benzyl)-1 fluoro-5 benzimidazolyl-2]-5 diméthyl-4,4-pentanoïque**

**Formule (I)**: $R_1 = R_2 = CH_3$, $R_3 = R_4 = H$, n = 2, D = $CO_2H$, $X_1$ = 5-F, $X_2$ = H, $X_3$ = H, $X_4$ = 4-Cl, B = $CH_2$, A = phényl

3 g de [(chloro-4 benzyl)-1 fluoro-5 benzimidazolyl-2]-5 diméthyl-4,4-valéronitrile, préparé à l'exemple 157 sont dissous dans un mélange composé de 30 ml d'eau et 30 ml d'éthanol. 3 g de soude en pastilles sont ajoutés et le mélange est chauffé au reflux pendant 15 heures. On additionne après refroidissement, 100 ml d'eau et la solution obtenue est lavée à l'éther. La phase aqueuse est acidifiée par barbottage de dioxyde de soufre et les cristaux obtenus sont essorés, lavés à l'eau puis à l'éther isopropylique et séchés pour donner 2,3 g d'acide [(chloro-4 benzyl)-1 fluoro-5 benzimidazolyl-2]-5 diméthyl-4,4-pentanoïque sous forme de cristaux de point de fusion 184-6-° C.

Exemple 159 :
**Chlorure d'acide ester éthylique de l'acide cyclohexène cis-4,5-dicarboxylique**

**Formule (VIII)** : n = O, D = $CO_2Et$, $R_1$ = $R_5$ = H, $R_6$ + $R_2$ = $CH_2$-CH=CH-$CH_2$

Préparé selon le mode opératoire de l'exemple 28 à partir de l'anhydride de l'acide cyclohexène cis-4,5-dicarboxylique.

Huile utilisée telle quelle pour la suite.

Exemple 160 :
**Cis-[(chloro-4 benzyl)-1 fluoro-5 benzimidazolyl-2]-5 cyclohexène-4 carboxylate d'éthyle**

**Formule (I)**: B = $CR_5R_6$, $R_1$ = $R_5$ = H, $R_2$ + $R_6$ = $CH_2$-CH=CH-$CH_2$, n = O, D = $CO_2Et$, A = phényl, $X_1$ = 5-F, $X_2$ = $X_3$ = H, $X_4$ = 4-Cl

Préparé selon le mode opératoire de l'exemple 34.

Cristaux de point de fusion 136° C.

Exemple 161:
**Acide cis-[(chloro-4 benzyl)-1 fluoro-5 benzimidazolyl-2]-5 cyclohexéne-4 carboxylique**

**Formule (I)**: B = $CR_5R_6$, $R_1$ = $R_5$ = H, $R_2$ + $R_6$ = $CH_2$-CH=CH-$CH_2$, n = O, D = $CO_2H$, A = phényl, $X_1$ = 5-F, $X_2$ = $X_3$ = H, $X_4$ = 4-Cl

Préparé selon le mode opératoire de l'exemple 58.

Cristaux de point de fusion 185-186° C.

Exemple 162 :
**(Méthylthio-4 benzyl) amino-2 nitro-3 chloro-5 pyridine**

**Formule (V)**: $X_1$ = 5-Cl, $X_2$ = H, $X_3$ = 4-$SCH_3$, $X_4$ = H, A = pyridine-2

Préparé selon le mode opératoire de l'exemple 6.

Cristaux de point de fusion 88° C.

Exemple 163 :
**(Méthylthio-4 benzyl) amino-2 amino-3 chloro-5 pyridine**

**Formule (IV)**: $X_1$ = 5-Cl, $X_2$ = H, $X_3$ = 4-$SCH_3$, $X_4$ = H, A = pyridine-2

Préparé selon le mode opératoire de l'exemple 14.

Cristaux de point de fusion 116° C.

Exemple 164 :
**[(Méthylthio-4 benzyl)-1 chloro-5 imidazo [4,5-b] pyridine-yl-2]-4diméthyl-3,3-butanoate d'éthyle**

**Formule (I)**: $R_1$ = $R_2$ = $CH_3$, $R_3$ = $R_4$ = H, n = 1, D = $CO_2Et$, A = pyridine-2, B = $CH_2$, $X_1$ = 5-Cl, $X_2$ = $X_3$ = H, $X_4$ = 4-$SCH_3$

Préparé selon le mode opératoire de l'exemple 91.

Huile utilisée telle quelle pour la suite.

Exemple 165 :

**Acide [(méthylthio-4 benzyl)-1 chloro-5 imidazo [4,5-b] pyridine yl-2]-4 diméthyl-3,3-butanoïque**

**Formule (I)**: $R_1 = R_2 = CH_3$, $R_3 = R_4 = H$, n = 1, D = $CO_2H$, A = pyridine-2, B = $CH_2$
$X_1$ = 5-Cl, $X_2 = X_3 = H$, $X_4$ = 4-$SCH_3$
Préparé selon le mode opératoire de l'exemple 92.

Cristaux de point de fusion 125-126 ° C.

TABLEAU

| | | |
|---|---|---|
| Exemple 58 | 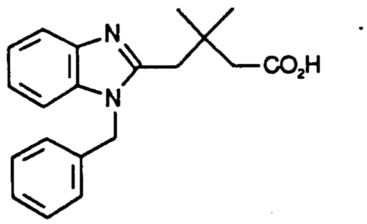 | UP 116-52 |
| Exemple 59 | 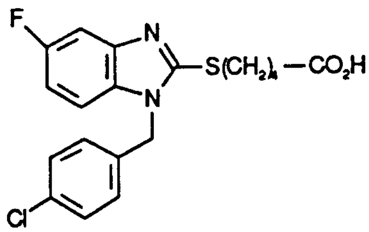 | UP 116-11 |
| Exemple 60 | 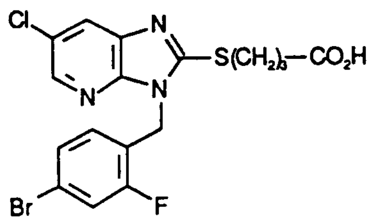 | UP 116-17 |
| Exemple 61 | 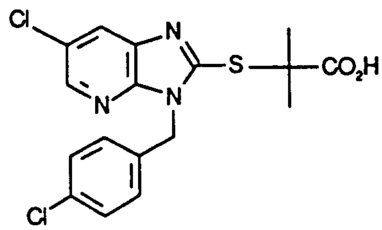 | UP 116-21 |
| Exemple 62 | 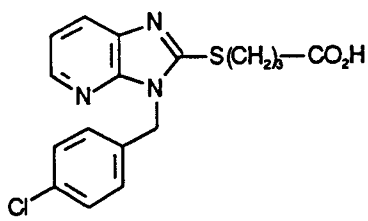 | UP 116-13 |
| Exemple 63 | 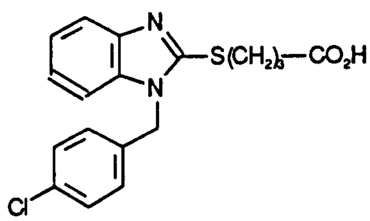 | UP 116-16 |

Exemple 64

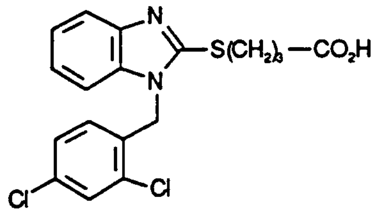

UP 116-18

Exemple 65

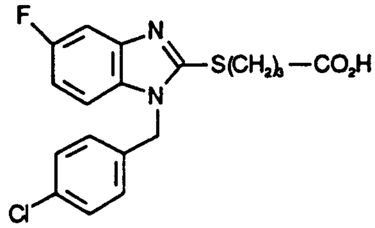

UP 116-1

Exemple 66

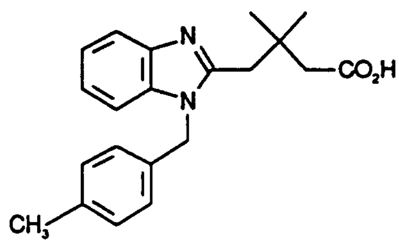

UP 116-57

Exemple 67

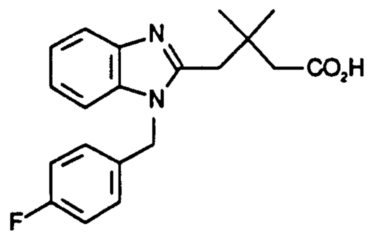

UP 116-56

Exemple 68

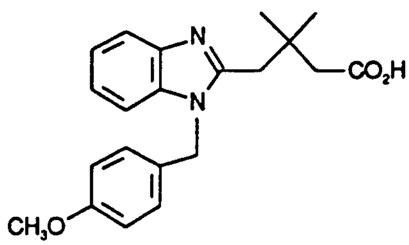

UP 116-55

Exemple 69

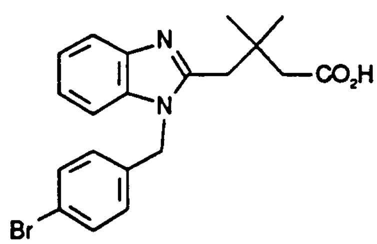

UP 116-59

Exemple 70

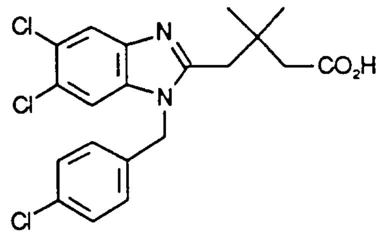

UP 116-63

Exemple 71

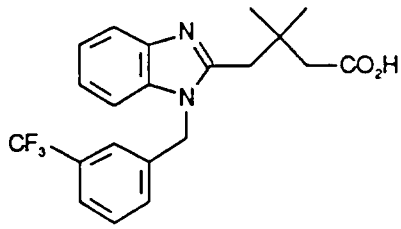

UP 116-67

Exemple 72

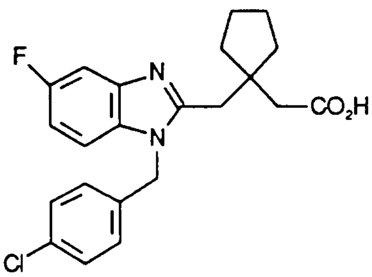

UP 116-53

Exemple 73

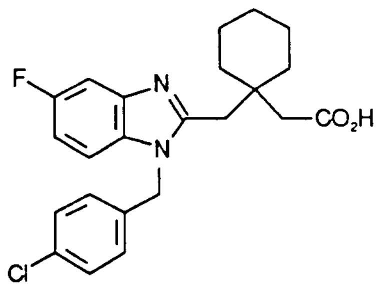

UP 116-54

Exemple 74

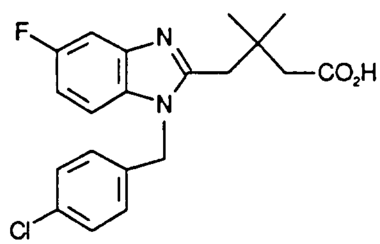

UP 116-47

Exemple 75

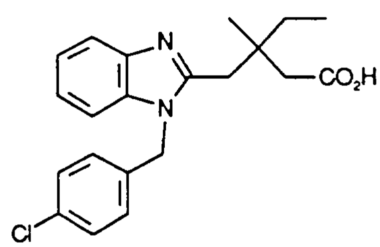

UP 116-65

Exemple 76

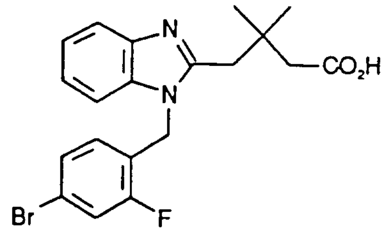

UP 116-51

Exemple 77

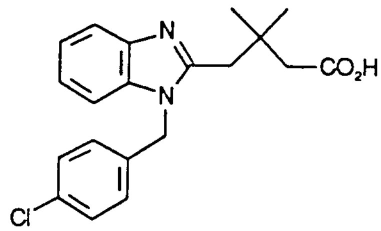

UP 116-58

Exemple 78

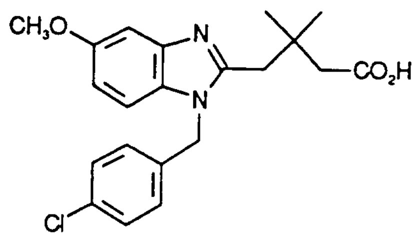

UP 116-64

Exemple 79

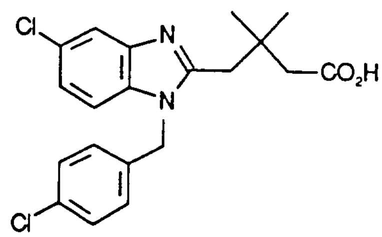

UP 116-60

Exemple 80

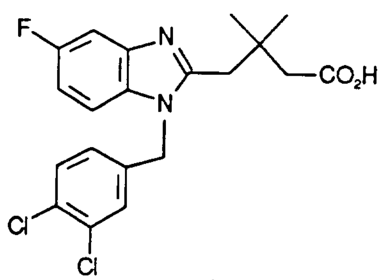

UP 116-62

Exemple 81

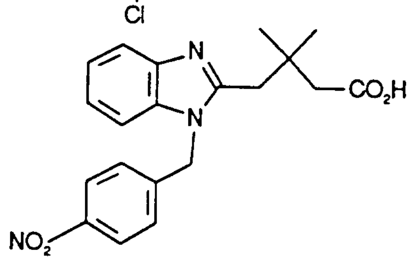

UP 116-66

Exemple 82 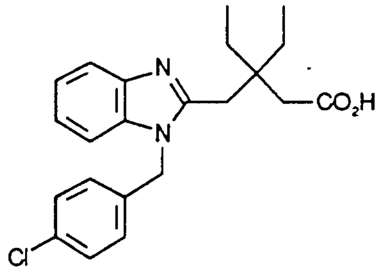 UP 116-68

Exemple 83 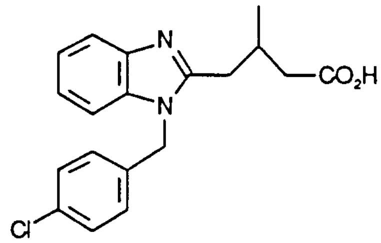 UP 116-70

Exemple 84 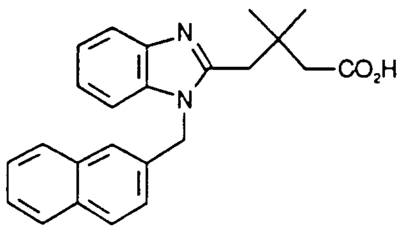 UP 116-71

Exemple 85 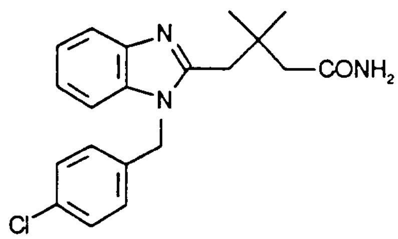 UP 116-72

Exemple 86 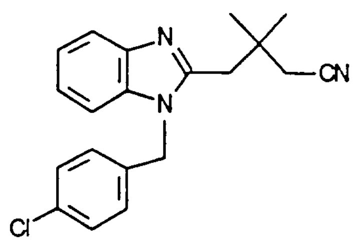 UP 116-73

Exemple 87 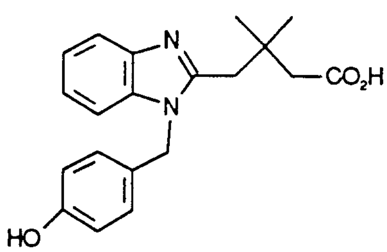 UP 116-61

Exemple 90

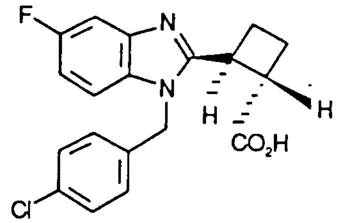

UP 116-74

Exemple 92

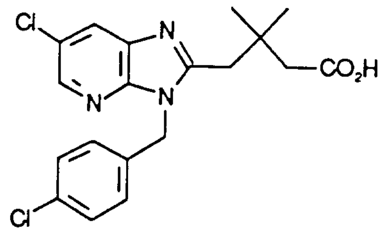

UP 116-77

Exemple 96

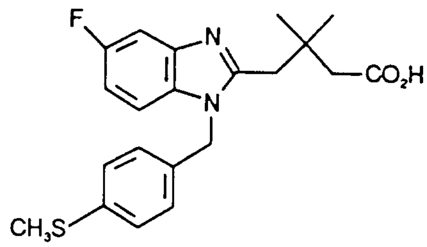

UP 116-78

Exemple 97

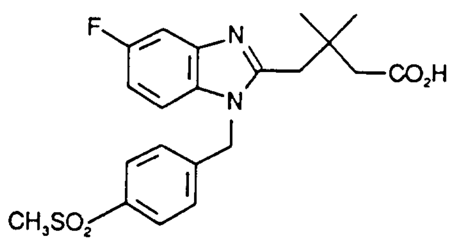

UP 116-79

Exemple 101

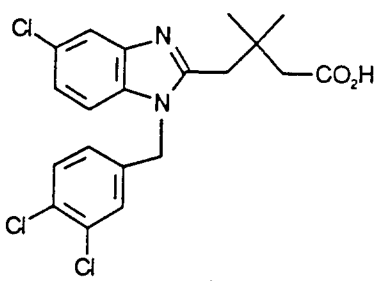

UP 116-81

Exemple 105

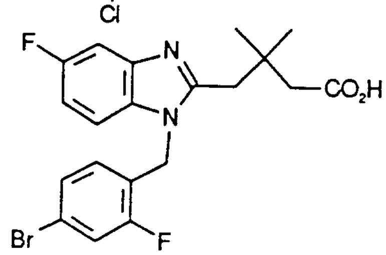

UP 116-83

Exemple 109 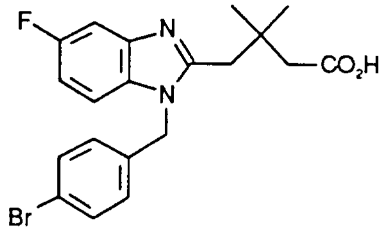 UP 116-84

Exemple 117 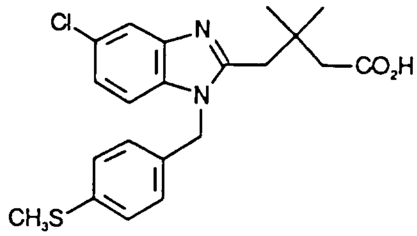 UP 116-87

Exemple 118 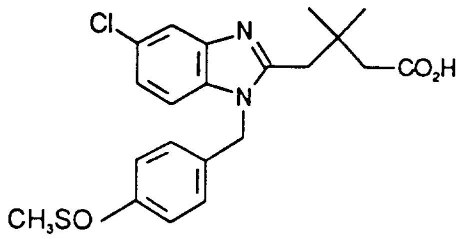 UP 116-86

Exemple 119 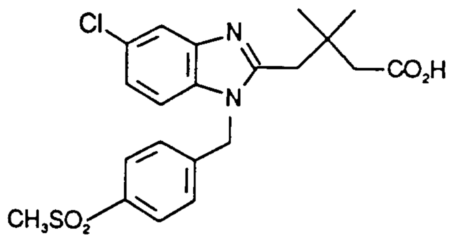 UP 116-89

Exemple 123 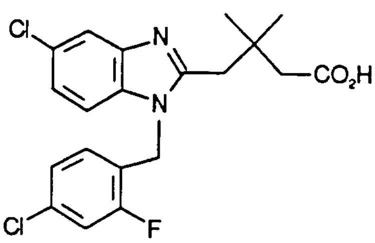 UP 116-88

Exemple 127 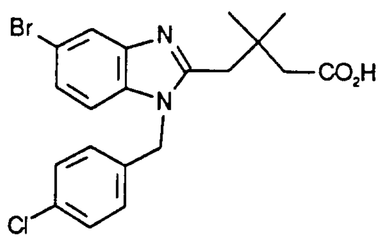 UP 116-90

Exemple 131 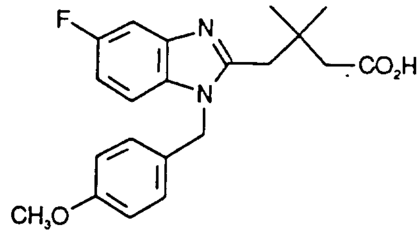 UP 116-91

Exemple 135 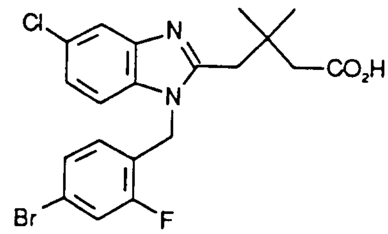 UP 116-93

Exemple 139 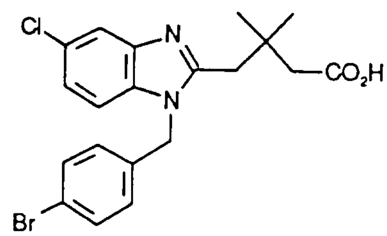 UP 116-95

Exemple 143 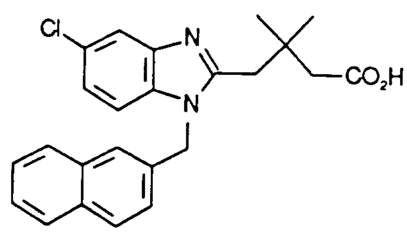 UP 116-97

Exemple 147 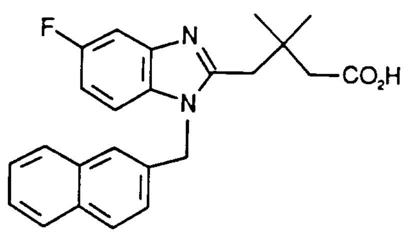 UP 116-96

Exemple 150 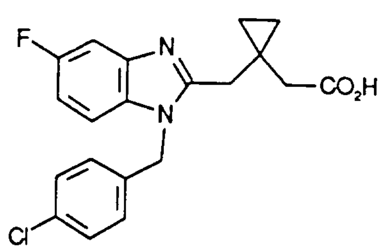 UP 116-75

Exemple 153

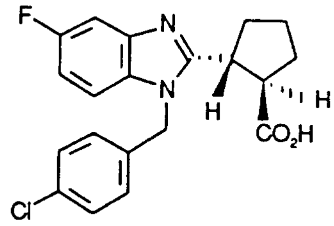

UP 116-80

Exemple 155

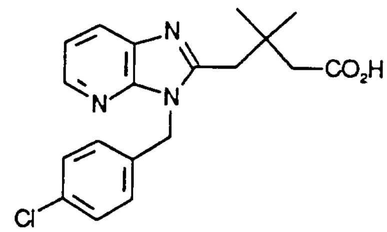

UP 116-82

Exemple 158

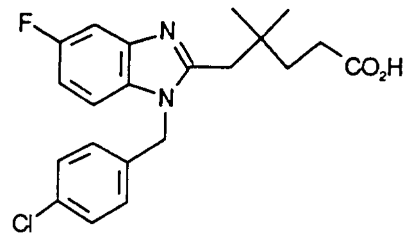

UP 116-92

Exemple 161

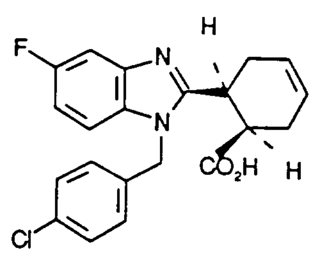

UP 116-76

Exemple 113

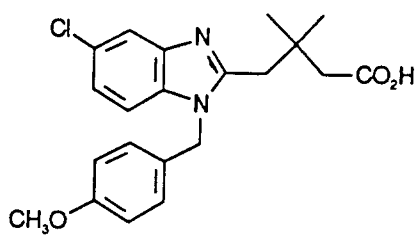

UP 116-85

Exemple 165

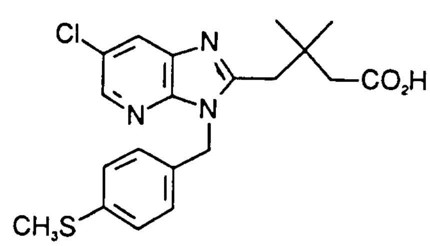

UP 116-99

## PHARMACOLOGIE

### Principe

L'affinité des produits des exemples décrits pour les récepteurs au thromboxane $A_2$ est évaluée par technique de déplacement d'un radioligand spécifiquement fixé sur les récepteurs au $TXA_2$ de plaquettes humaines.

### Technique

Des plaquettes humaines incubent en présence d'une concentration unique de [$^{125}$I] PTA-OH (9,11 - diméthylméthano - 11, 12 - méthano - 16 - (3 [$^{125}$I] iodo -4 - hydroxyphényl)-13, 14 - dihydro - 13 - aza - 15 $\alpha$ - $\omega$ - tétranor - $TXA_2$) antagoniste des récepteurs $TXA_2$ / $PGH_2$ et de deux concentrations d'agents compétiteurs ($10^{-5}$ M, $10^{-7}$ M) durant 30 minutes à 37 ° C.

La réaction est achevée par ajout de tampon, puis rapide filtration à travers des filtres de papier de verre.

La liaison non spécifique est déterminée en présence de U 46619 (9, 11- didéoxy - 11 $\alpha$, 9 $\alpha$ - époxyméthano - prostaglandine F2 $\alpha$; thromboxane $A_2$ mimétique).

### Résultats

Les résultats sont exprimés, pour les doses testées, en pourcentage de déplacement du radioligand spécifiquement fixé sur les récepteurs plaquettaires humains du $TXA_2$. Pour certains produits des exemples, la constante inhibitrice Ki a été déterminée selon la formule de CHENG et PRUSOFF

$$Ki = \frac{CI_{50}}{1 + \frac{L}{K_D}}$$

où CI désigne la concentration inhibitrice 50, L la concentration en ligand radioactif et $K_D$ la constante de dissociation du ligand radioactif.

### Toxicologie

Des études préliminaires de toxicité ont pu montrer que les doses létales 50 déterminées après administration orale chez le rat étaient supérieures à 300 mg/kg, traduisant un index thérapeutique intéressant.

### Conclusion

En conclusion, les molécules décrites dans la présente demande ou leurs sels d'addition non toxiques présentent une importante affinité pour les récepteurs au $TXA_2$ et peuvent être utilisées avec intérêt et profit dans le traitement des pathologies suivantes :

- infarctus du myocarde, angine de poitrine, ictus cérébral, migraine, hémorragie cérébrale, athérosclérose, embolie pulmonaire, asthme bronchique, bronchite, pneumonie, choc circulatoire d'origines diverses (hémorragie, septicémie, défaillance cardiaque, traumatique, pancréatite aiguë, brûlure, bactérienne), néphrite, rejet de greffe, métastases cancéreuses.

par voie orale, sous forme de comprimés ou gélules dosés de 1 à 200 mg ou par voie parentérale sous forme de préparations injectables dosées de 0.01 à 10 mg préférentiellement en plusieurs prises (2 à 4) ou administrations journalières.

| | % de déplacement | |
|---|---|---|
| Exemple | Concentration 1E-7 M | Concentration 1E-5 M |
| 58 | 13 | 95 |
| 59 | 28 | 100 |
| 60 | 24 | 60 |
| 61 | 30 | 100 |
| 62 | 27 | 64 |
| 63 | 37 | 97 |
| 64 | 34 | 97 |
| 65 | 8 | 93 |
| 66 | 52 | 100 |
| 67 | 66 | 100 |
| 68 | 89 | 100 |
| 69 | 86 | 96 |
| 70 | 14 | 100 |
| 71 | 49 | 100 |
| 72 | 71 | 100 |
| 73 | 0 | 98 |
| 74 | 94 | 100 |
| 75 | 57 | 100 |
| 76 | 88 | 99 |
| 77 | 85 | 100 |
| 78 | 44 | 100 |
| 79 | 74 | 99 |
| 80 | 89 | 92 |
| 81 | 83 | 100 |
| 82 | 35 | 97 |
| 83 | 10 | 100 |

| | % de déplacement | |
|---|---|---|
| Exemple | Concentration 1E-7 M | Concentration 1E-5 M |
| 84 | 85 | 98 |
| 85 | 7 | 90 |
| 87 | 50 | 96 |
| 90 | 2 | 97 |
| 92 | 97 | 100 |
| 96 | 100 | 100 |
| 97 | 100 | 100 |
| 101 | 71 | 92 |
| 105 | 100 | 98 |
| 109 | 95 | 92 |
| 117 | 87 | 89 |
| 118 | 54 | 92 |
| 119 | 61 | 82 |
| 123 | 77 | 87 |
| 127 | 38 | 67 |
| 131 | 79 | 100 |
| 135 | 74 | 95 |
| 139 | 98 | 100 |
| 143 | 77 | 89 |
| 147 | 91 | 93 |
| 150 | 80 | 98 |
| 153 | 37 | 100 |
| 155 | 41 | 62 |
| 158 | 56 | 87 |
| 161 | 15 | 96 |

| Produit des exemples | **Ki** (M/l) |
|---|---|
| 68 | $3.10 \times 10^{-8}$ |
| 69 | $1.58 \times 10^{-8}$ |
| 72 | $4.23 \times 10^{-8}$ |
| 73 | $8.07 \times 10^{-7}$ |
| 74 | $6.30 \times 10^{-9}$ |
| 76 | $1.14 \times 10^{-8}$ |
| 77 | $3.10 \times 10^{-8}$ |
| 79 | $2.32 \times 10^{-8}$ |
| 80 | $6.02 \times 10^{-9}$ |
| 81 | $6.01 \times 10^{-8}$ |
| 90 | $1.18 \times 10^{-6}$ |
| 92 | $1.25 \times 10^{-8}$ |
| 96 | $5.00 \times 10^{-9}$ |
| 97 | $3.70 \times 10^{-8}$ |
| 105 | $1.50 \times 10^{-8}$ |
| 117 | $5.80 \times 10^{-9}$ |
| 139 | $1.30 \times 10^{-8}$ |

**Revendications**

**Revendications pour les Etats contractants suivants : AT, BE, CH, DE, DK, FR, GB, IT, LI, LU, NL, SE**

**1.** Dérivés de benzimidazole caractérisés en ce qu'ils répondent à la formule (I)

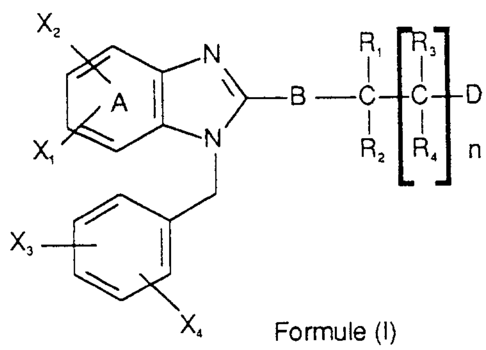

Formule (I)

dans laquelle :

A est un noyau aromatique ou un hétérocycle azoté,

$X_1$, $X_2$, $X_3$ et $X_4$ représentent indépendamment un atome d'hydrogène, un atome d'halogène, un radical alkyle ayant de 1 à 6 atomes de carbone,un radical cyclo alkyle en $C_3$-$C_7$, un radical alkoxy ayant de 1 à 6 atomes de carbone, un radical alkylthio ayant de 1 à 6 atomes de carbone, un groupe sulfone, $SO_2$ alkyle ayant de 1 à 6 atomes de carbone, un groupe sulfoxyde, SO alkyle ayant de 1 à 6 atomes de carbone, un groupe trifluorométhyle, un groupe hydroxyle, un groupe nitro, un radical méthylène alcool ou une fonction COOR' où R' est un hydrogène ou un alkyle ayant de 1 à 6 atomes de carbone, $X_3$ et $X_4$ peuvent également former avec le phényle un naphtalène,

B représente $CR_5R_6$, $R_5$ et $R_6$ étant un atome d'hydrogène ou un radical alkyle ayant de 1 à 6 atomes de carbone, ou un radical cycloalkyle en $C_3$-$C_7$, ou l'atome de soufre,

$R_1$,$R_2$,$R_3$ et $R_4$ représentent indépendamment un atome d'hydrogène ou un radical alkyle ayant de 1 à 6 atomes de carbone ou un radical cycloalkyle en $C_3$-$C_7$. $CR_1R_2$ ou $CR_3R_4$ peuvent former avec B lorsque ce dernier représente $CR_5R_6$ un cycloalkyle ou un cycloalkylène de 3 à 7 atomes de carbone, $R_1R_2$, $R_3$ $R_4$ peuvent également former un cycle de 3 à 7 atomes de carbone,

n est un nombre entier de 1 à 4 et peut être 0 si $R_1$ et $R_2$ sont différents de l'hydrogène,

D représente une fonction chimique qui peut être :

$COOR_7$, $R_7$ étant l'atome d'hydrogène ou un radical alkyle ayant de 1 à 6 atomes de carbone ou un radical cyclo alkyle en $C_3$-$C_7$,

CONH-$R_8$, $R_8$ étant l'atome d'hydrogène ou un radical alkyle ayant de 1 à 6 atomes de carbone ou un radical cyclo alkyle en $C_3$-$C_7$,
CN

ainsi que l'acide [(chloro-4 benzyl)-1 chloro-5 imidazo[4,5-b] pyridine yl-2] mercapto-2 méthyl-2 propanoïque, et leurs sels d'addition en particulier les sels d'addition pharmaceutiquement acceptables.

**2.** Dérivés selon la revendication 1 caractérisés en ce que A est un cycle phényle.

**3.** Dérivés selon la revendication 1 caractérisés en ce que A est un cycle pyridine.

**4.** Dérivés selon l'une des revendications 1 à 3 caractérisés en ce que $X_1$ est l'atome de fluor.

**5.** Dérivés selon l'une des revendications 1 à 3 caractérisés en ce que $X_1$ est l'atome de chlore.

**6.** Dérivés selon l'une des revendications 1 à 5 caractérisés en ce que $X_3$ est l'atome de chlore.

**7.** Dérivés selon l'une des revendications 1 à 5 caractérisés en ce que $X_3$ est le groupement méthoxy.

**8.** Dérivés selon l'une des revendications 1 à 5 caractérisés en ce que $X_3$ est le groupement méthylthio.

**9.** Dérivés selon l'une des revendications 1 à 8 caractérisés en ce que $X_4$ est l'atome de chlore

**10.** Dérivés selon l'une des revendications 1 à 9 caractérisés en ce que D est une fonction acide.

**11.** Dérivés selon l'une des revendications 1 à 10 caractérisés en ce que B est un groupement méthylène, $R_1$ et $R_2$ représentent chacun un méthyle, $R_3$ et $R_4$ l'hydrogène et n étant égal à 1.

**12.** Dérivés selon l'une des revendications 1 à 10 caractérisés en ce que B est l'atome de soufre.

**13.** Dérivés selon l'une des revendications 1 à 10 ou 12 caractérisés en ce que $CR_1$ $R_2$ représente un cyclopentane.

**14.** Dérivés selon l'une quelconque des revendications 1 à 11 caractérisés en ce qu'ils sont choisis parmi les dérivés de formule :

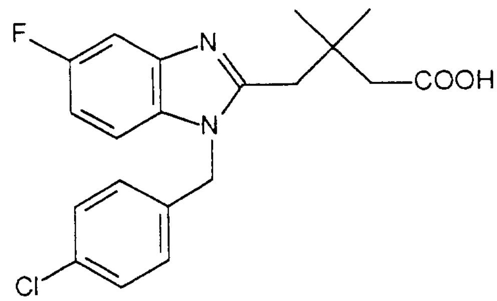

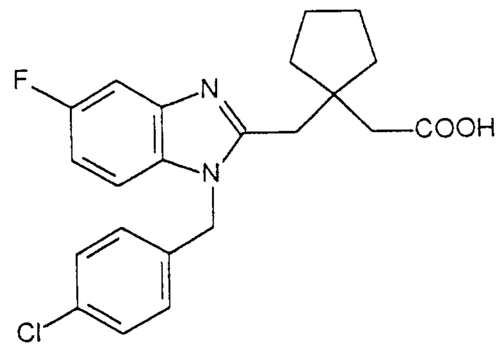

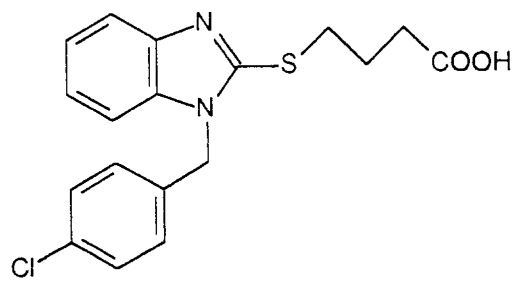

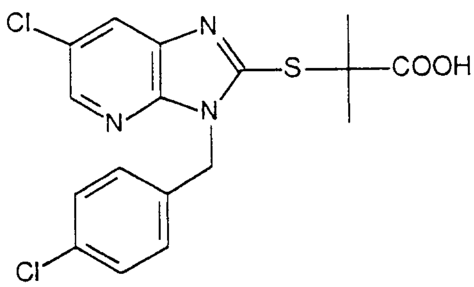

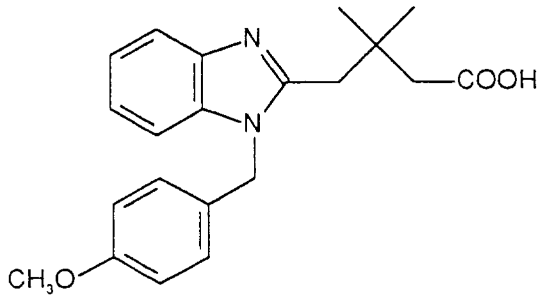

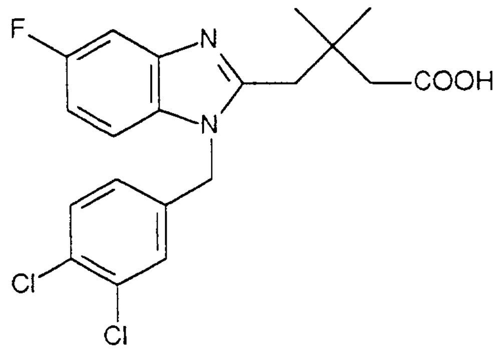

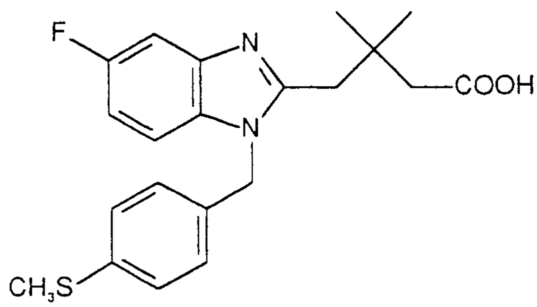

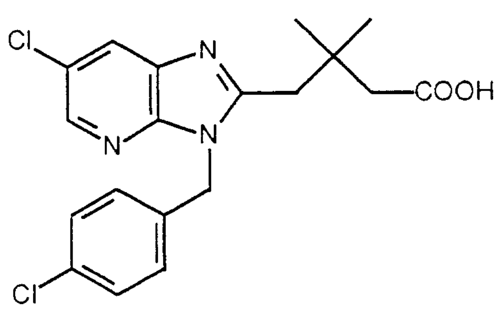

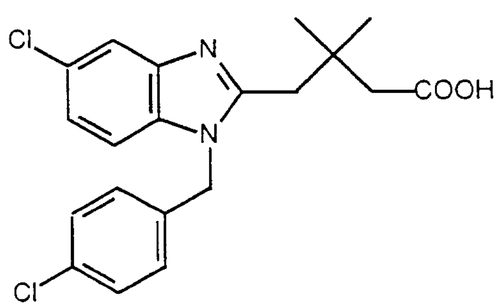

**15.** Procédés de préparation des composés de formule (I) selon l'une quelconque des revendications 1 à 14 caractérisés en ce qu'ils sont préparés :

A/ Lorsque B est un atome de soufre par action d'un halogéno alkanoate d'alkyle sur un mercapto benzimidazole ou azabenzimidazole en présence d'une base telle qu'un alcoolate de sodium ou potassium, d'un hydrure de sodium ou de lithium, de carbonate de potassium dans un alcool, le diméthylformamide, l'acétone ou la butanone-2.

B/ lorsque B est un groupement $CR_5$ $R_6$ en deux étapes : par action d'un chlorure d'acide de formule (VIII)

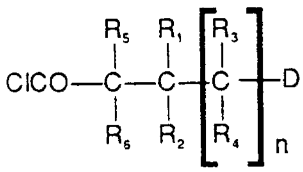

Formule (VIII)

Dans laquelle $R_1$, $R_2$, $R_3$, $R_4$, $R_5$, $R_6$ et n sont définis comme ci-dessus et D est une fonction ester ou nitrile.

sur une diamine de formule (IV)

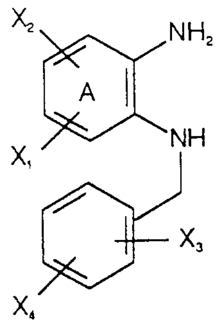

Formule (IV)

dans laquelle A, $X_1$, $X_2$, $X_3$ et $X_4$ sont définis comme ci-dessus suivi dans un deuxième temps d'une cyclisation en milieu acide,

ou bien par réaction de la diamine de formule (IV) sur un aldéhyde de formule (VIII')

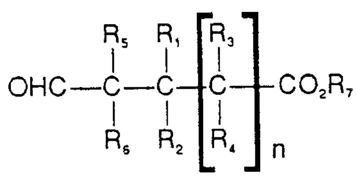

Formule (VIII')

dans laquelle $R_1$, $R_2$, $R_3$, $R_4$, $R_5$, $R_6$ et n sont définis comme ci-dessus et $R_7$ est un radical alkyle ayant de 1 à 6 atomes de carbone dans un milieu acide acétique et alcool, ce dernier pouvant être le méthanol, l'éthanol, le méthoxy éthanol par exemple, le dérivé obtenu étant alors oxydé avec un agent oxydant comme l'iode ou le manganate de barium, l'hydrolyse acide ou basique des dérivés esters ou nitrile conduit aux dérivés acides.

**16.** Composition pharmaceutique, caractérisée en ce qu'elle comprend une quantité pharmaceutiquement efficace d'au moins un composé de formule (I) tel que défini à l'une quelconque des revendications 1 à 14, ou un de ses sels d'addition pharmaceutiquement acceptable, éventuellement incorporé dans un excipient, véhicule ou support pharmaceutiquement acceptable.

**17.** Composition pharmaceutique à activité antagoniste des récepteurs au thromboxane permettant notamment de traiter favorablement l'infarctus du myocarde, l'angine de poitrine, l'ictus cérébral, la migraine, l'hémorragie cérébrale, l'athérosclérose, l'embolie pulmonaire, l'asthme bronchique, la bronchite, une pneumonie, les chocs circulatoires d'origines diverses (hémorragie, septicémie, défaillance cardiaque, traumatique, pancréatique aigüe, brûlure, bactérienne), les néphrites, le rejet de greffe, les métastases cancéreuses, caractérisée en ce qu'elle renferme une quantité pharmaceutiquement efficace d'au moins un composé de formule (I) tel que défini dans l'une quelconque des revendications 1 à 14, ou un de ses sels d'addition pharmaceutiquement acceptable, éventuellement incorporé dans un excipient, véhicule ou support pharmaceutiquement acceptable.

**18.** Procédé de préparation d'une composition pharmaceutique caractérisé en ce qu'on incorpore une quantité pharmaceutiquement efficace d'au moins un composé de formule (I) telle que définie dans l'une quelconque des revendications 1 à 14, ou un de ses sels d'addition pharmaceutiquement acceptable, dans un excipient, véhicule ou support pharmaceutiquement acceptable.

**19.** Procédé selon la revendication 18, caractérisé en ce que la composition pharmaceutique est formulée sous forme de gélules, de comprimés dosés de 1 à 200 mg, ou sous forme de préparations injectables dosées de 0,01 à 10 mg.

**Revendications pour l'Etat contractant suivant : ES**

**1.** Procédé de préparation de dérivés de benzimidazole répondant à la formule (I):

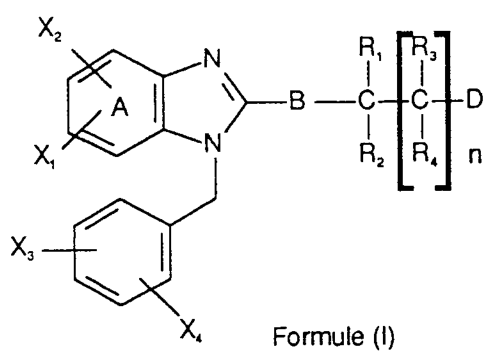

Formule (I)

dans laquelle :

A est un noyau aromatique ou un hétérocycle azoté,

$X_1$, $X_2$, $X_3$ et $X_4$ représentent indépendamment un atome d'hydrogène, un atome d'halogène, un radical alkyle ayant de 1 à 6 atomes de carbone,un radical cyclo alkyle en $C_3$-$C_7$, un radical alkoxy ayant de 1 à 6 atomes de carbone, un radical alkylthio ayant de 1 à 6 atomes de carbone, un groupe sulfone, $SO_2$ alkyle ayant de 1 à 6 atomes de carbone, un groupe sulfoxyde, SO alkyle ayant de 1 à 6 atomes de carbone, un groupe trifluorométhyle, un groupe hydroxyle, un groupe nitro, un radical méthylène alcool ou une fonction COOR' où R' est un hydrogène ou un alkyle ayant de 1 à 6 atomes de carbone, $X_3$ et $X_4$ peuvent également former avec le phényle un naphtalène,

B représente $CR_5R_6$, $R_5$ et $R_6$ étant un atome d'hydrogène ou un radical alkyle ayant de 1 à 6 atomes de carbone, ou un radical cycloalkyle en $C_3$-$C_7$, ou l'atome de soufre,

$R_1$,$R_2$,$R_3$ et $R_4$ représentent indépendamment un atome d'hydrogène ou un radical alkyle ayant de 1 à 6 atomes de carbone ou un radical cycloalkyle en $C_3$-$C_7$. $CR_1R_2$ ou $CR_3R_4$ peuvent former avec B lorsque ce dernier représente $CR_5R_6$ un cycloalkyle ou un cycloalkylène de 3 à 7 atomes de carbone, $R_1R_2$, $R_3$ $R_4$ peuvent également former un cycle de 3 à 7 atomes de carbone,

n est un nombre entier de 1 à 4 et peut être 0 si $R_1$ et $R_2$ sont différents de l'hydrogène,

D représente une fonction chimique qui peut être :

$COOR_7$, $R_7$ étant l'atome d'hydrogène ou un radical alkyle ayant de 1 à 6 atomes de carbone ou un radical cyclo alkyle en $C_3$-$C_7$,

CONH-$R_8$, $R_8$ étant l'atome d'hydrogène ou un radical alkyle ayant de 1 à 6 atomes de carbone ou un radical cyclo alkyle en $C_3$-$C_7$,

CN

ainsi que de l'acide [(chloro-4 benzyl)-1 chloro-5 imidazo[4,5-b] pyridine yl-2] mercapto-2 méthyl-2 propanoïque

ou de leurs sels d'addition en particulier les sels d'addition pharmaceutiquement acceptables, caractérisé en ce qu'il comprend :

A/ Lorsque B est un atome de soufre l'action d'un halogéno alkanoate d'alkyle sur un mercapto benzimidazole ou azabenzimidazole en présence d'une base telle qu'un alcoolate de sodium ou potassium, d'un hydrure de sodium ou de lithium, de carbonate de potassium dans un alcool, le diméthylformamide, l'acétone ou la butanone-2.

B/ lorsque B est un groupement $CR_5$ $R_6$ : dans un premier temps, l'action d'un chlorure d'acide de formule (VIII)

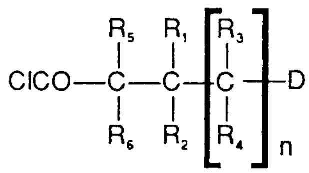

Formule (VIII)

dans laquelle $R_1$, $R_2$, $R_3$, $R_4$, $R_5$, $R_6$ et n sont définis comme ci-dessus et D est une fonction ester ou nitrile.

sur une diamine de formule (IV)

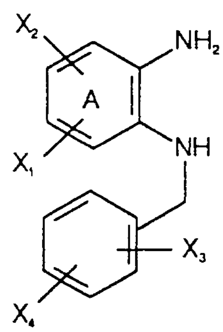

Formule (IV)

dans laquelle A, $X_1$, $X_2$, $X_3$ et $X_4$ sont définis comme ci-dessus suivi dans un deuxième temps d'une cyclisation en milieu acide,

ou bien la réaction de la diamine de formule (IV) sur un aldéhyde de formule (VIII')

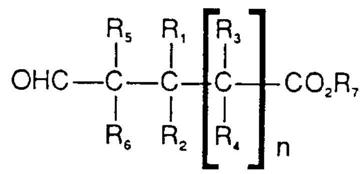

Formule (VIII')

dans laquelle $R_1$, $R_2$, $R_3$, $R_4$, $R_5$, $R_6$ et n sont définis comme ci-dessus et $R_7$ est un radical alkyle ayant de 1 à 6 atomes de carbone dans un milieu acide acétique et alcool, ce dernier pouvant être le méthanol, l'éthanol, le méthoxy éthanol par exemple, le dérivé obtenu étant alors oxydé avec un agent oxydant comme l'iode ou le manganate de barium, l'hydrolyse acide ou basique des dérivés esters ou nitrile conduisant aux dérivés acides; et éventuellement la conversion du produit ainsi obtenu en un sel d'addition, en particulier un sel d'addition pharmaceutiquement acceptable.

**2.** Procédé de préparation selon la revendication 1 des composés de formule (I) dans laquelle A est un cycle phényle.

**3.** Procédé de préparation selon la revendication 1 des composés de formule (I) dans laquelle A est un cycle pyridine.

**4.** Procédé de préparation selon l'une des revendications 1 à 3 des composés de formule (I) dans laquelle $X_1$ est l'atome de fluor.

**5.** Procédé de préparation selon l'une des revendications 1 à 3 des composés de formule (I) dans laquelle $X_1$ est l'atome de chlore.

**6.** Procédé de préparation selon l'une des revendications 1 à 5 des composés de formule (I) dans laquelle $X_3$ est l'atome de chlore.

**7.** Procédé de préparation selon l'une des revendications 1 à 5 des composés de formule (I) dans laquelle $X_3$ est le groupement méthoxy.

**8.** Procédé de préparation selon l'une des revendications 1 à 5 des composés de formule (I) dans laquelle $X_3$ est le groupement méthylthio.

**9.** Procédé de préparation selon l'une des revendications 1 à 8 des composés de formule (I) dans laquelle $X_4$ est l'atome de chlore.

**10.** Procédé de préparation selon l'une des revendications 1 à 9 des composés de formule (I) dans laquelle D est une fonction acide.

**11.** Procédé de préparation selon l'une des revendications 1 à 10 des composés de formule (I) caractérisé en ce que B est un groupement méthylène, $R_1$ et $R_2$ représentent chacun un méthyle, $R_3$ et $R_4$ l'hydrogène et n étant égal à 1.

**12.** Procédé de préparation selon l'une des revendications 1 à 10 des composés de formule (I) dans laquelle B est l'atome de soufre.

**13.** Procédé de préparation selon l'une des revendications 1 à 10 ou 12 des composés de formule (I) dans laquelle $CR_1R_2$ représente un cyclopentane.

**14.** Procédé de préparation selon l'une des revendications 1 à 11 des composés choisis parmi les dérivés de formule :

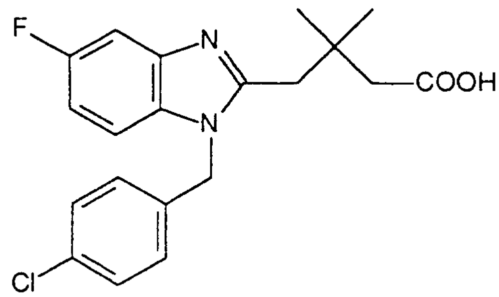

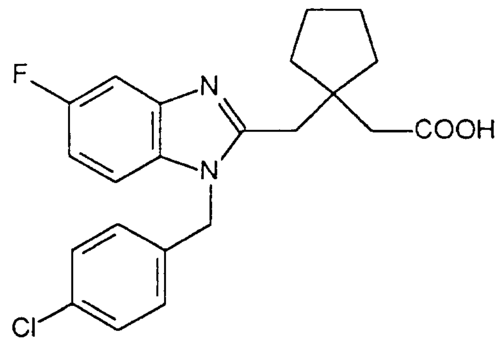

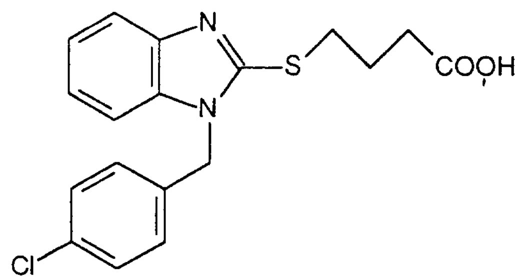

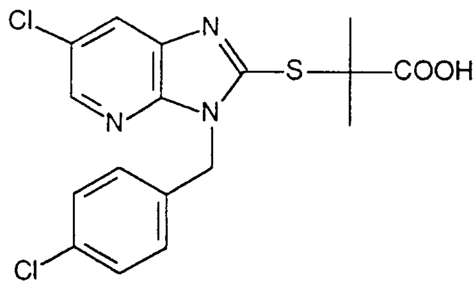

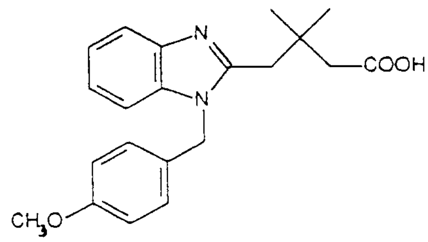

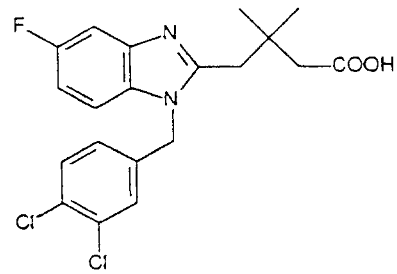

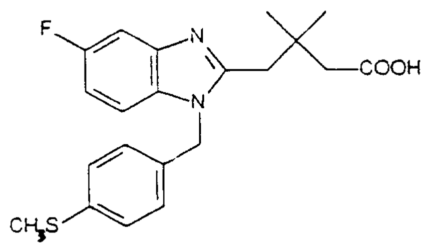

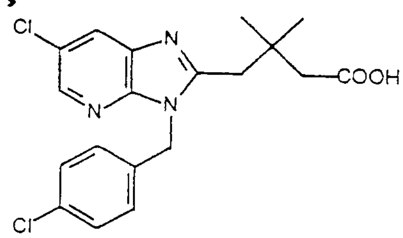

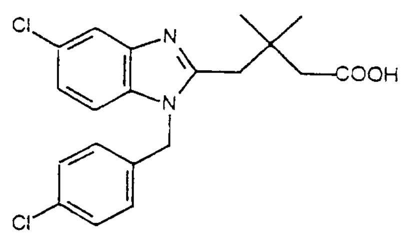

**15.** Procédé de préparation d'une composition pharmaceutique, caractérisé en ce qu'on incorpore une quantité pharmaceutiquement efficace d'au moins un composé de formule (I) préparé selon l'une quelconque des revendications 1 à 14, ou un de ses sels d'addition pharmaceutiquement acceptable, dans un excipient, véhicule ou support pharmaceutiquement acceptable.

**16.** Procédé selon la revendication 15, caractérisé en ce que la composition pharmaceutique est formulée sous forme de gélules, de comprimés dosés de 1 à 200 mg, ou sous forme de préparations injectables dosées de 0,01 à 10 mg.

**Revendications pour l'Etat contractant suivant : GR**

**1.** Dérivés de benzimidazole caractérisés en ce qu'ils répondent à la formule (I)

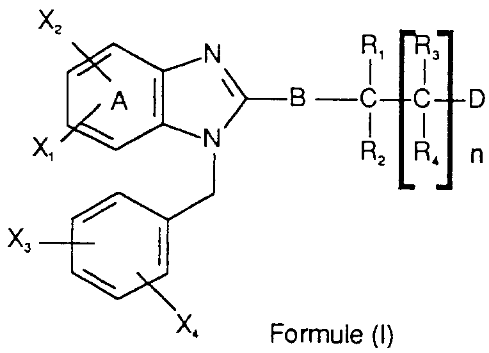

Formule (I)

dans laquelle :

A est un noyau aromatique ou un hétérocycle azoté,

$X_1$, $X_2$, $X_3$ et $X_4$ représentent indépendamment un atome d'hydrogène, un atome d'halogène, un radical alkyle ayant de 1 à 6 atomes de carbone,un radical cyclo alkyle en $C_3$-$C_7$, un radical alkoxy ayant de 1 à 6 atomes de carbone, un radical alkylthio ayant de 1 à 6 atomes de carbone, un groupe sulfone, $SO_2$ alkyle ayant de 1 à 6 atomes de carbone, un groupe sulfoxyde, SO alkyle ayant de 1 à 6 atomes de carbone, un groupe trifluorométhyle, un groupe hydroxyle, un groupe nitro, un radical méthylène alcool ou une fonction COOR' où R' est un hydrogène ou un alkyle ayant de 1 à 6 atomes de carbone, $X_3$ et $X_4$ peuvent également former avec le phényle un naphtalène,

B représente $CR_5R_6$, $R_5$ et $R_6$ étant un atome d'hydrogène ou un radical alkyle ayant de 1 à 6 atomes de carbone, ou un radical cycloalkyle en $C_3$-$C_7$, ou l'atome de soufre,

$R_1$,$R_2$,$R_3$ et $R_4$ représentent indépendamment un atome d'hydrogène ou un radical alkyle ayant de 1 à 6 atomes de carbone ou un radical cycloalkyle en $C_3$-$C_7$. $CR_1R_2$ ou $CR_3R_4$ peuvent former avec B lorsque ce dernier représente $CR_5R_6$ un cycloalkyle ou un cycloalkylène de 3 à 7 atomes de carbone, $R_1R_2$, $R_3$ $R_4$ peuvent également former un cycle de 3 à 7 atomes de carbone,

n est un nombre entier de 1 à 4 et peut être 0 si $R_1$ et $R_2$ sont différents de l'hydrogène,

D représente une fonction chimique qui peut être :

$COOR_7$, $R_7$ étant l'atome d'hydrogène ou un radical alkyle ayant de 1 à 6 atomes de carbone ou un radical cyclo alkyle en $C_3$-$C_7$,

CONH-$R_8$, $R_8$ étant l'atome d'hydrogène ou un radical alkyle ayant de 1 à 6 atomes de carbone ou un radical cyclo alkyle en $C_3$-$C_7$,

CN

ainsi que l'acide [(chloro-4 benzyl)-1 chloro-5 imidazo[4,5-b] pyridine yl-2] mercapto-2 méthyl-2 propanoïque, et leurs sels d'addition en particulier les sels d'addition pharmaceutiquement acceptables.

**2.** Dérivés selon la revendication 1 caractérisés en ce que A est un cycle phényle.

**3.** Dérivés selon la revendication 1 caractérisés en ce que A est un cycle pyridine.

**4.** Dérivés selon l'une des revendications 1 à 3 caractérisés en ce que $X_1$ est l'atome de fluor.

**5.** Dérivés selon l'une des revendications 1 à 3 caractérisés en ce que $X_1$ est l'atome de chlore.

**6.** Dérivés selon l'une des revendications 1 à 5 caractérisés en ce que $X_3$ est l'atome de chlore.

**7.** Dérivés selon l'une des revendications 1 à 5 caractérisés en ce que $X_3$ est le groupement méthoxy.

**8.** Dérivés selon l'une des revendications 1 à 5 caractérisés en ce que $X_3$ est le groupement méthylthio.

**9.** Dérivés selon l'une des revendications 1 à 8 caractérisés en ce que $X_4$ est l'atome de chlore

10. Dérivés selon l'une des revendications 1 à 9 caractérisés en ce que D est une fonction acide.

11. Dérivés selon l'une des revendications 1 à 10 caractérisés en ce que B est un groupement méthylène, $R_1$ et $R_2$ représentent chacun un méthyle, $R_3$ et $R_4$ l'hydrogène et n étant égal à 1.

12. Dérivés selon l'une des revendications 1 à 10 caractérisés en ce que B est l'atome de soufre.

13. Dérivés selon l'une des revendications 1 à 10 ou 12 caractérisés en ce que $CR_1$ $R_2$ représente un cyclopentane.

14. Dérivés selon l'une quelconque des revendications 1 à 11 caractérisés en ce qu'ils sont choisis parmi les dérivés de formule :

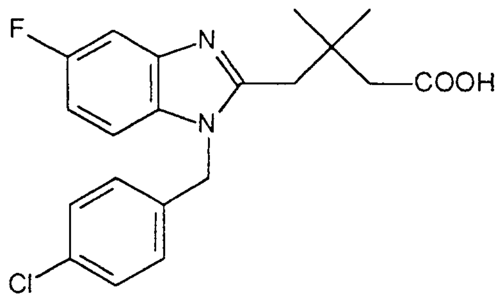

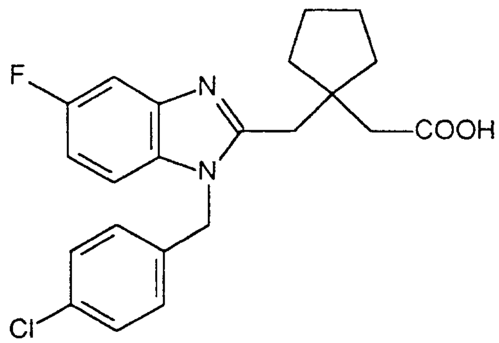

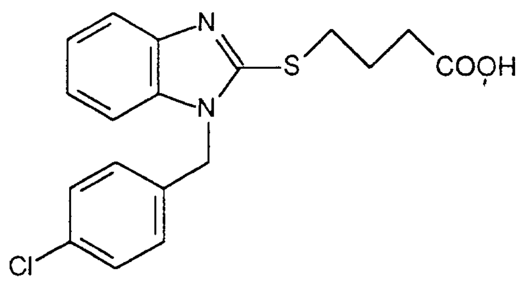

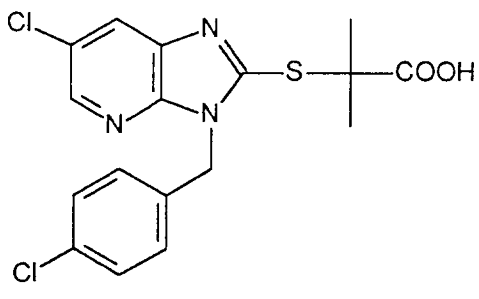

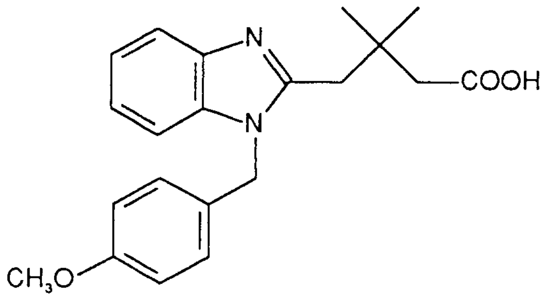

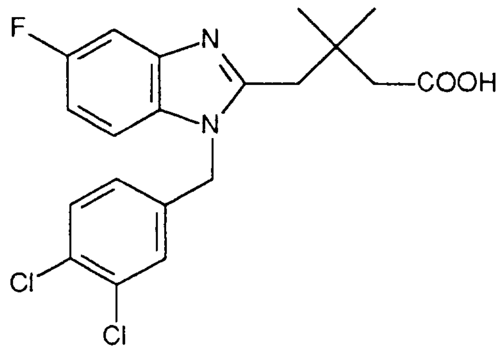

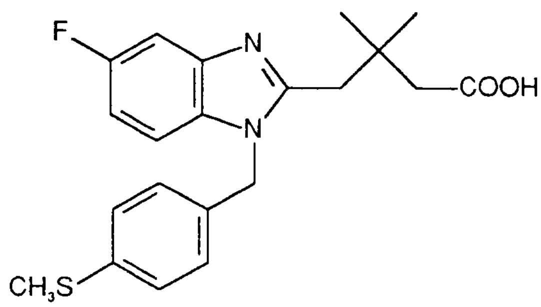

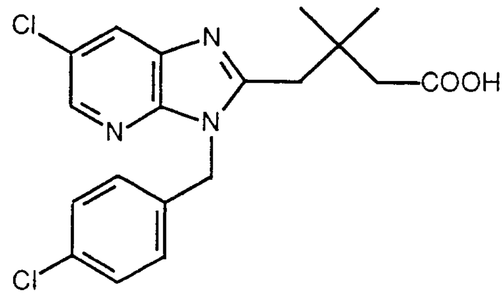

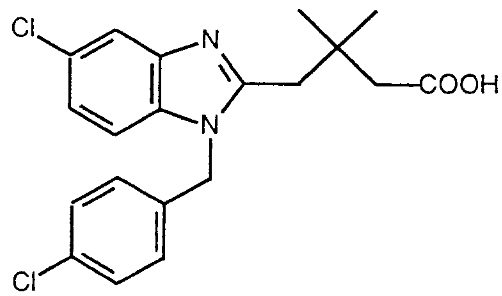

**15.** Procédés de préparation des composés de formule (I) selon l'une quelconque des revendications 1 à 14 caractérisés en ce qu'ils sont préparés :

A/ Lorsque B est un atome de soufre par action d'un halogéno alkanoate d'alkyle sur un mercapto benzimidazole ou azabenzimidazole en présence d'une base telle qu'un alcoolate de sodium ou potassium, d'un hydrure de sodium ou de lithium, de carbonate de potassium dans un alcool, le diméthylformamide, l'acétone ou la butanone-2.

B/ lorsque B est un groupement $CR_5$ $R_6$ en deux étapes : par action d'un chlorure d'acide de formule (VIII)

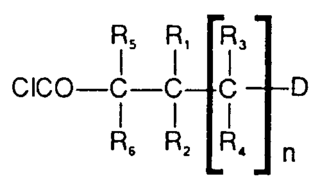

Formule (VIII)

Dans laquelle $R_1$, $R_2$, $R_3$, $R_4$, $R_5$, $R_6$ et n sont définis comme ci-dessus et D est une fonction ester ou nitrile.

sur une diamine de formule (IV)

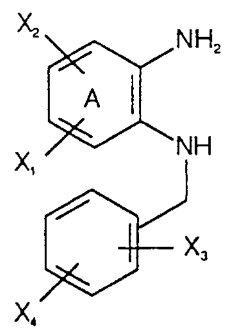

Formule (IV)

dans laquelle A, $X_1$, $X_2$, $X_3$ et $X_4$ sont définis comme ci-dessus suivi dans un deuxième temps d'une cyclisation en milieu acide,

ou bien par réaction de la diamine de formule (IV) sur un aldéhyde de formule (VIII')

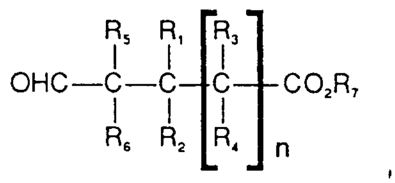

Formule (VIII')

dans laquelle $R_1$, $R_2$, $R_3$, $R_4$, $R_5$, $R_6$ et n sont définis comme ci-dessus et $R_7$ est un radical alkyle ayant de 1 à 6 atomes de carbone dans un milieu acide acétique et alcool, ce dernier pouvant être le méthanol, l'éthanol, le méthoxy éthanol par exemple, le dérivé obtenu étant alors oxydé avec un agent oxydant comme l'iode ou le manganate de barium, l'hydrolyse acide ou basique des dérivés esters ou nitrile conduit aux dérivés acides.

**16.** Procédé de préparation d'une composition pharmaceutique, caractérisé en ce qu'on incorpore une quantité pharmaceutiquement efficace d'au moins un composé de formule (I) telle que définie dans l'une quelconque des revendications 1 à 14, ou un de ses sels d'addition pharmaceutiquement acceptable, dans un excipient, véhicule ou support pharmaceutiquement acceptable.

**17.** Procédé selon la revendication 16, caractérisé en ce que la composition pharmaceutique est formulée sous forme de gélules, de comprimés dosés de 1 à 200 mg, ou sous forme de préparations injectables dosées de 0,01 à 10 mg.

## Claims

### Claims for the following Contracting States : AT, BE, CH, DE, DK, FR, GB, IT, LI, LU, NL, SE

1. Benzimidazole derivatives characterized in that they have the formula (I)

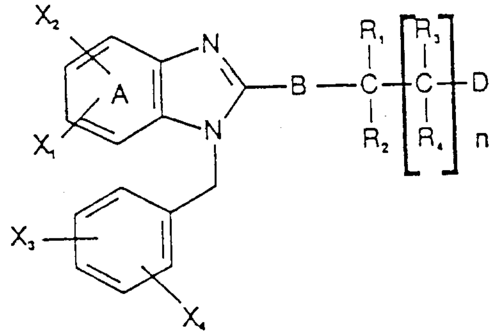

Formula (I)

in which

A is an aromatic ring or a nitrogen heterocycle,

$X_1$, $X_2$, $X_3$ and $X_4$ are independently a hydrogen atom, a halogen atom, an alkyl radical having 1 to 6 carbon atoms, a $C_3$-$C_7$ cycloalkyl radical, an alkoxy radical having 1 to 6 carbon atoms, an alkylthio radical having 1 to 6 carbon atoms, a sulfone group, an $SO_2$ alkyl having 1 to 6 carbon atoms, a sulfoxide group, an SO alkyl having 1 to 6 carbon atoms, a trifluoromethyl group, a hydroxyl group, a nitro group, a methylene alcohol radical or a group COOR' in which R' is a hydrogen or an alkyl having 1 to 6 carbon atoms, $X_3$ and $X_4$ can also form a naphthalene with the phenyl,

B is $CR_5R_6$, $R_5$ and $R_6$ being a hydrogen atom or an alkyl radical having 1 to 6 carbon atoms, or a $C_3$-$C_7$ cycloalkyl radical, or the sulfur atom,

$R_1$, $R_2$, $R_3$ and $R_4$ are independently a hydrogen atom or an alkyl radical having 1 to 6 carbon atoms or a $C_3$-$C_7$ cycloalkyl radical, $CR_1R_2$ or $CR_3R_4$ can form with B, when the latter is $CR_5R_6$, a cycloalkyl or a cycloalkylene having 3 to 7 carbon atoms, $R_1R_2$, $R_3R_4$ can also form a ring having 3 to 7 carbon atoms,

n is an integer from 1 to 4 and can be 0 if $R_1$ and $R_2$ are other than hydrogen,

D is a chemical group which can be:

$COOR_7$, $R_7$ being the hydrogen atom or an alkyl radical having 1 to 6 carbon atoms, or a $C_3$-$C_7$ cycloalkyl radical,

$CONHR_8$, $R_8$ being the hydrogen atom or an alkyl radical having 1 to 6 carbon atoms, or a $C_3$-$C_7$ cycloalkyl radical,

CN,

as well as 2-[1-(4-chlorobenzyl)-5-chloroimidazo[4,5-b]pyridin-2-yl] mercapto-2-methylpropanoic acid, and their addition salts in particular the pharmaceutically acceptable salts.

2. Derivatives according to claim 1, characterized in that A is a phenyl ring.

3. Derivatives according to claim 1, characterized in that A is a pyridine ring.

4. Derivatives according to one of claims 1 to 3, characterized in that $X_1$ is the fluorine atom.

5. Derivatives according to one of claims 1 to 3, characterized in that $X_1$ is the chlorine atom.

6. Derivatives according to one of claims 1 to 5, characterized in that $X_3$ is the chlorine atom.

7. Derivatives according to one of claims 1 to 5, characterized in that $X_3$ is the methoxy group.

8. Derivatives according to one of claims 1 to 5, characterized in that $X_3$ is the methylthio group.

**9.** Derivatives according to one of claims 1 to 8, characterized in that $X_4$ is the chlorine atom.

**10.** Derivatives according to one of claims 1 to 9, characterized in that D is an acid group.

**11.** Derivatives according to one of claims 1 to 10, characterized in that B is a methylene group, $R_1$ and $R_2$ are each a methyl, $R_3$ and $R_4$ are hydrogen and n is equal to 1.

**12.** Derivatives according to one of claims 1 to 10, characterized in that B is the sulfur atom.

**13.** Derivatives according to one of claims 1 to 10 or 12, characterized in that $CR_1R_2$ is a cyclopentane.

**14.** Derivatives according to any one of claims 1 to 11, characterized in that they are selected from the derivatives of formula:

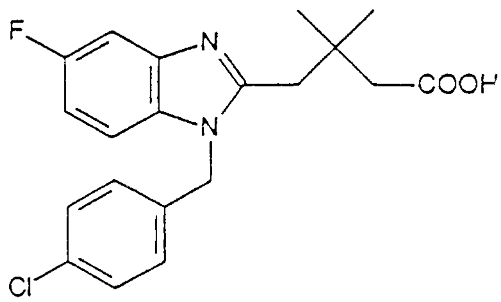

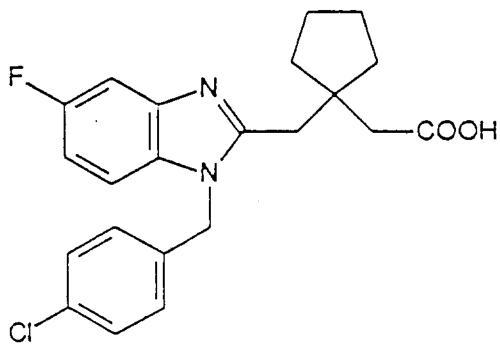

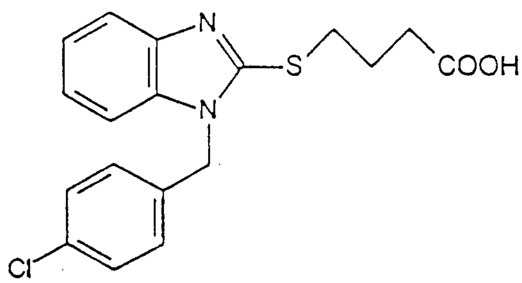

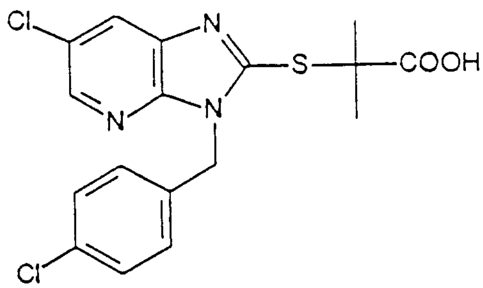

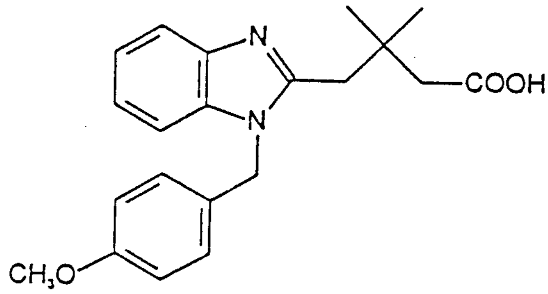

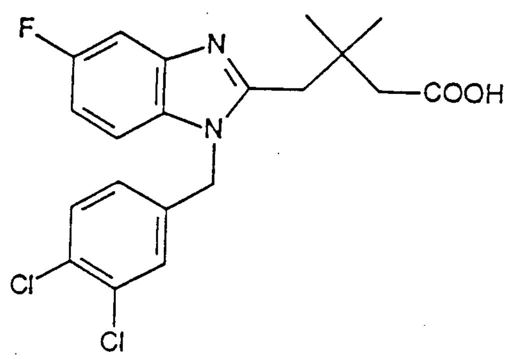

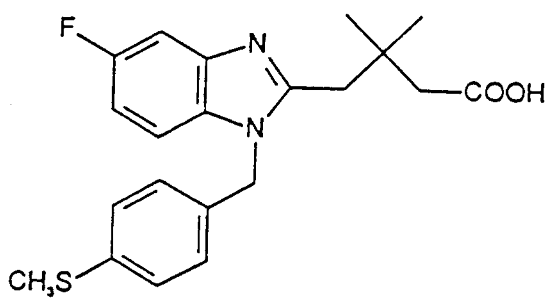

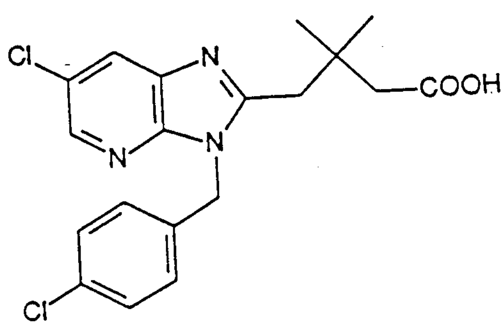

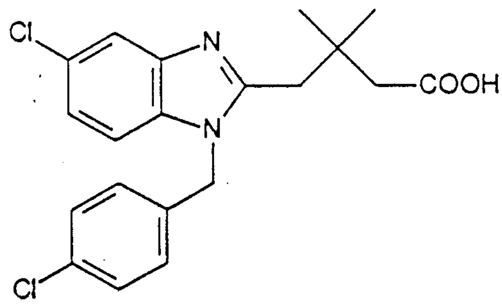

**15.** Methods of preparing the compounds of formula (I) according to any one of claims 1 to 14 characterized in that they are prepared:

A/ if B is a sulfur atom: by reacting an alkyl halogenoalkanoate with a mercaptobenzimidazole or mercaptoazabenzimidazole in the presence of a base such as a sodium or potassium alcoholate, sodium or lithium hydride or potassium carbonate in an alcohol, dimethylformamide, acetone or butan-2-one; or

B/ if B is a group $CR_5R_6$: in two steps by reacting an acid chloride of formula (VIII):

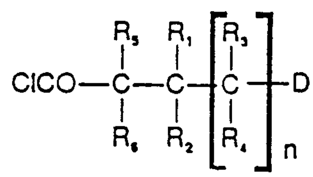

Formula (VIII)

in which $R_1$, $R_2$, $R_3$, $R_4$, $R_5$, $R_6$ and n are as defined above and D is an ester or nitrile group, with a diamine of formula (IV)

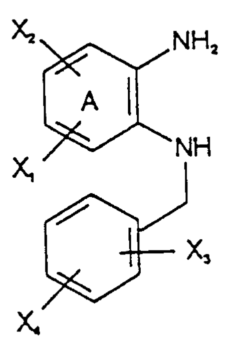

Formula (IV)

in which A, $X_1$, $X_2$, $X_3$ and $X_4$ are as defined above, this being followed in a second stage by cyclization in an acid medium,

or by reacting the diamine of formula (IV) with an aldehyde of formula (VIII'):

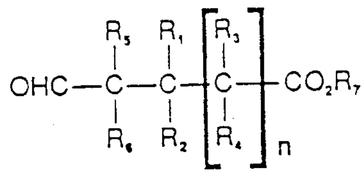

Formula (VIII')

in which $R_1$, $R_2$, $R_3$, $R_4$, $R_5$, $R_6$ and n are as defined above and R7 is an alkyl radical having 1 to 6 carbon atoms, in an acetic acid and alcohol medium, it being possible for said alcohol to be methanol, ethanol or methoxyethanol for example, the derivative obtained then being oxidized with an oxidizing agent such as iodine or barium manganate, acid or basic hydrolysis of the ester or nitrile derivatives yielding the acid derivatives.

**16.** Pharmaceutical composition, characterized in that it comprises a pharmaceutically effective amount of at least one compound of formula (I) as defined in any one of claims 1 to 14, or one of its pharmaceutically acceptable addition salts, which may or may not be incorporated in a pharmaceutically acceptable excipient, vehicle or carrier.

**17.** Pharmaceutical composition active as a thromboxane receptor antagonist, with which the following diseases in particular can be favorably treated: myocardial infarction, angina pectoris, stroke, migraine, cerebral hemorrhage, atherosclerosis, pulmonary embolism, bronchial asthma, bronchitis, pneumonia, circulatory shocks of various origins (hemorrhage, septicemia, heart failure, trauma, acute pancreatitis, burn, bacterial origin), nephritis, graft rejection and cancerous metastases, characterized in that it contains a pharmaceutically effective amount of at least one compound of formula (I) as defined in any one of claims 1 to 14, or one of its pharmaceutically acceptable addition salts, which may or may not be incorporated in a pharmaceutically acceptable excipient, vehicle or carrier.

**18.** Method of preparing a pharmaceutical composition, characterized in that a pharmaceutically effective amount of at least one compound of formula (I) as defined in any one of claims 1 to 14, or one of its pharmaceutically acceptable addition salts, is incorporated into a pharmaceutically acceptable excipient, vehicle or carrier.

**19.** Method according to claim 18, characterized in that the pharmaceutical composition is formulated as gelatin capsules or tablets containing from 1 to 200 mg of active ingredient, or as injectable preparations containing from 0.01 to 10 mg of active ingredient.

**Claims for the following Contracting State : ES**

**1.** Method of preparing benzimidazole derivatives having the formula (I):

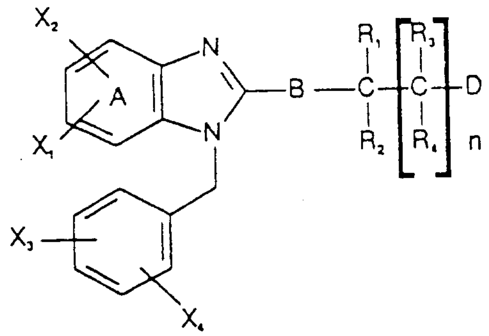

Formula (I)

in which

A is an aromatic ring or a nitrogen heterocycle,

$X_1$, $X_2$, $X_3$ and $X_4$ are independently a hydrogen atom, a halogen atom, an alkyl radical having 1 to 6 carbon atoms, a $C_3$-$C_7$ cycloalkyl radical, an alkoxy radical having 1 to 6 carbon atoms, an alkylthio radical having 1 to 6 carbon atoms, a sulfone group, an $SO_2$ alkyl having 1 to 6 carbon atoms, a sulfoxide group, an SO alkyl having 1 to 6 carbon atoms, a trifluoromethyl group, a hydroxyl group, a nitro group, a methylene alcohol radical or a group COOR' in which R' is a hydrogen or an alkyl having 1 to 6 carbon atoms, $X_3$ and $X_4$ can also form a naphthalene with the phenyl,

B is $CR_5R_6$, $R_5$ and $R_6$ being a hydrogen atom or an alkyl radical having 1 to 6 carbon atoms, or a $C_3$-$C_7$ cycloalkyl radical, or the sulfur atom,

$R_1$, $R_2$, $R_3$ and $R_4$ are independently a hydrogen atom or an alkyl radical having 1 to 6 carbon atoms or a $C_3$-$C_7$ cycloalkyl radical, $CR_1R_2$ or $CR_3R_4$ can form with B, when the latter is $CR_5R_6$, a cycloalkyl or a cycloalkylene having 3 to 7 carbon atoms, $R_1R_2$, $R_3R_4$ can also form a ring having 3 to 7 carbon atoms,

n is an integer from 1 to 4 and can be 0 if $R_1$ and $R_2$ are other than hydrogen,

D is a chemical group which can be:

$COOR_7$, $R_7$ being the hydrogen atom or an alkyl radical having 1 to 6 carbon atoms, or a $C_3$-$C_7$ cycloalkyl radical,

$CONHR_8$, $R_8$ being the hydrogen atom or an alkyl radical having 1 to 6 carbon atoms, or a $C_3$-$C_7$ cycloalkyl radical,

CN,

as well as 2-[1-(4-chlorobenzyl)-5-chloroimidazo[4,5-b]pyridin-2-yl] mercapto-2-methylpropanoic acid,

or their addition salts in particular the pharmaceutically acceptable salts, characterized in that it comprises:

A/ if B is a sulfur atom, reacting an alkyl halogenoalkanoate with a mercaptobenzimidazole or mercaptoazabenzimidazole in the presence of a base such as a sodium or potassium alcoholate, sodium or lithium hydride or potassium carbonate in an alcohol, dimethylformamide, acetone or butan-2-one.

B/ if B is a group $CR_5R_6$, first reacting an acid chloride of formula (VIII):

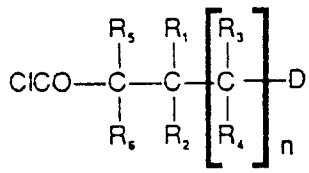

Formula (VIII)

in which $R_1$, $R_2$, $R_3$, $R_4$, $R_5$, $R_6$ and n are as defined above and D is an ester or nitrile group, with a diamine of formula (IV)

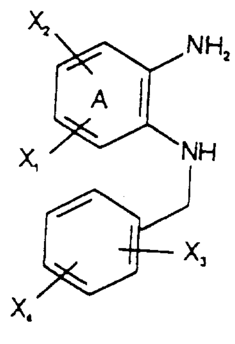

Formula (IV)

in which A, $X_1$, $X_2$, $X_3$ and $X_4$ are as defined above, this being followed in a second stage by cyclization in an acid medium,

or else reacting the diamine of formula (IV) with an aldehyde of formula (VIII'):

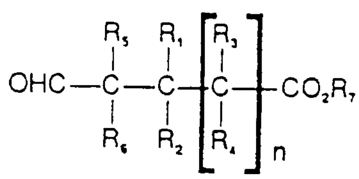

Formula (VIII')

in which $R_1$, $R_2$, $R_3$, $R_4$, $R_5$, $R_6$ and n are as defined above and R7 is an alkyl radical having 1 to 6 carbon atoms, in an acetic acid and alcohol medium, it being possible for said alcohol to be methanol, ethanol or methoxyethanol for example, the derivative obtained then being oxidized with an oxidizing agent such as iodine or barium manganate, acid or basic hydrolysis of the ester or nitrile derivatives yielding the acid derivatives;
and optionally the conversion of the thus obtained product into an addition salt, in particular a pharmaceutically acceptable addition salt.

**2.** Method of preparing according to claim 1 compounds of formula (I) in which A is a phenyl ring.

3. Method of preparing according to claim 1 compounds of formula (I) in which A is a pyridine ring.

4. Method of preparing according to one of claims 1 to 3 compounds of formula (I) in which $X_1$ is the fluorine atom.

5. Method of preparing according to one of claims 1 to 3 compounds of formula (I) in which $X_1$ is the chlorine atom.

6. Method of preparing according to one of claims 1 to 5 compounds of formula (I) in which $X_3$ is the chlorine atom.

7. Method of preparing according to one of claims 1 to 5 compounds of formula (I) in which $X_3$ is the methoxy group.

8. Method of preparing according to one of claims 1 to 5 compounds of formula (I) in which $X_3$ is the methylthio group.

9. Method of preparing according to one of claims 1 to 8 compounds of formula (I) in which $X_4$ is the chlorine atom.

10. Method of preparing according to one of claims 1 to 9 compounds of formula (I) in which D is an acid group.

11. Method of preparing according to one of claims 1 to 10 compounds of formula (I) characterized in that B is a methylene group, $R_1$ and $R_2$ are each a methyl, $R_3$ and $R_4$ are hydrogen and n being equal to 1.

12. Method of preparing according to one of claims 1 to 10 compounds of formula (I) in which B is the sulfur atom.

13. Method of preparing according to one of claims 1 to 10 or 12 compounds of formula (I) in which $CR_1R_2$ is a cyclopentane

14. Method of preparing according to one of claims 1 to 11 compounds selected from the derivatives of formula:

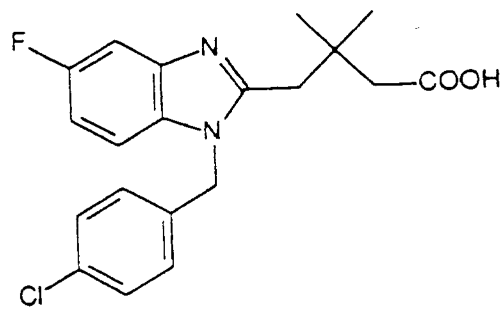

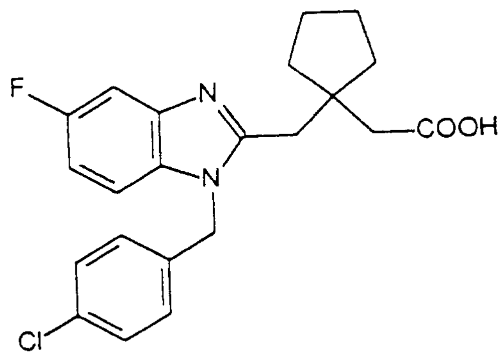

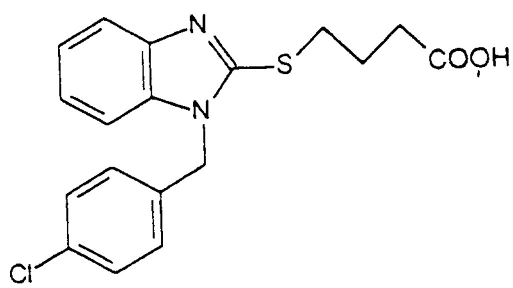

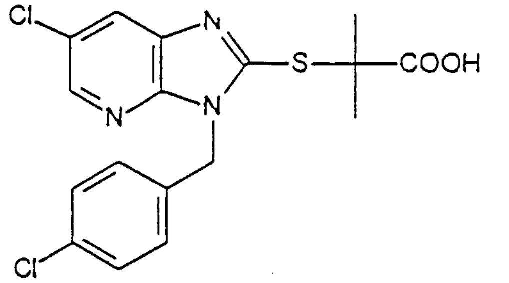

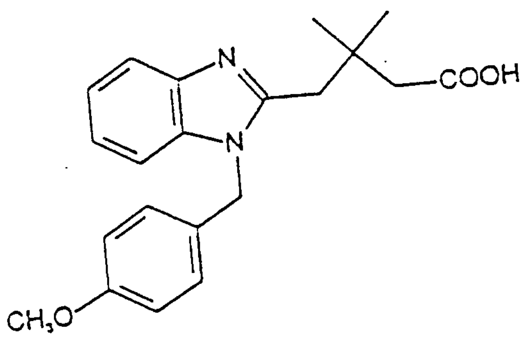

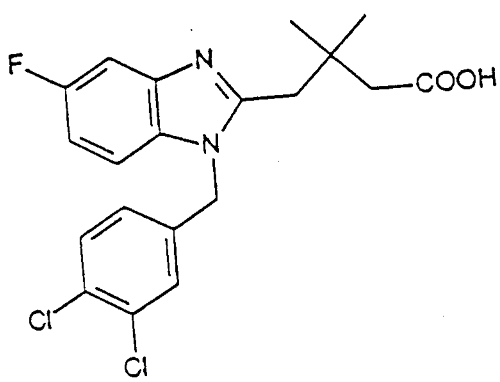

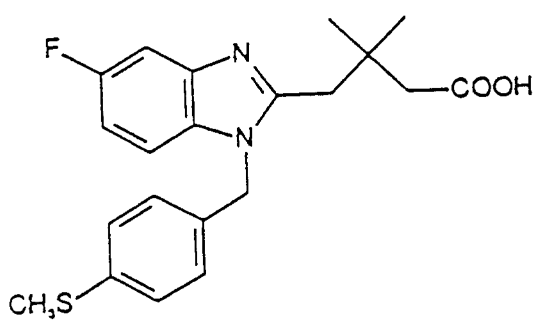

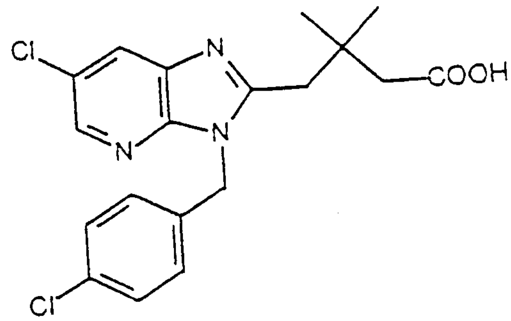

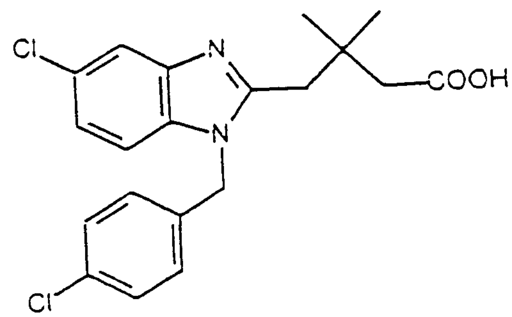

**15.** Method of preparing a pharmaceutical composition, characterized in that a pharmaceutically effective amount of at least one compound of formula (I) prepared according to any one of claims 1 to 14, or one of its pharmaceutically acceptable addition salts, is incorporated in a pharmaceutically acceptable excipient, vehicle or carrier.

**16.** Method according to claim 15, characterized in that the pharmaceutical composition is formulated as gelatin capsules or tablets containing from 1 to 200 mg of active ingredient, or as injectable preparations containing from 0.01 to 10 mg of active ingredient.

**Claims for the following Contracting State : GR**

**1.** Benzimidazole derivatives characterized in that they have the formula (I)

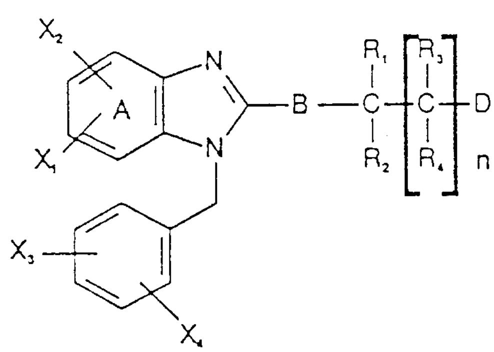

Formula (I)

in which

A is an aromatic ring or a nitrogen heterocycle,

$X_1$, $X_2$, $X_3$ and $X_4$ are independently a hydrogen atom, a halogen atom, an alkyl radical having 1 to 6 carbon atoms, a $C_3$-$C_7$ cycloalkyl radical, an alkoxy radical having 1 to 6 carbon atoms, an alkylthio

radical having 1 to 6 carbon atoms, a sulfone group, an $SO_2$ alkyl having 1 to 6 carbon atoms, a sulfoxide group, an SO alkyl having 1 to 6 carbon atoms, a trifluoromethyl group, a hydroxyl group, a nitro group, a methylene alcohol radical or a group COOR' in which R' is a hydrogen or an alkyl having 1 to 6 carbon atoms, $X_3$ and $X_4$ can also form a naphthalene with the phenyl,

B is $CR_5R_6$, $R_5$ and $R_6$ being a hydrogen atom or an alkyl radical having 1 to 6 carbon atoms, or a $C_3$-$C_7$ cycloalkyl radical, or the sulfur atom,

$R_1$, $R_2$, $R_3$ and $R_4$ are independently a hydrogen atom or an alkyl radical having 1 to 6 carbon atoms or a $C_3$-$C_7$ cycloalkyl radical, $CR_1R_2$ or $CR_3R_4$ can form with B, when the latter is $CR_5R_6$, a cycloalkyl or a cycloalkylene having 3 to 7 carbon atoms, $R_1R_2$, $R_3R_4$ can also form a ring having 3 to 7 carbon atoms,

n is an integer from 1 to 4 and can be 0 if $R_1$ and $R_2$ are other than hydrogen,

D is a chemical group which can be:

$COOR_7$, $R_7$ being the hydrogen atom or an alkyl radical having 1 to 6 carbon atoms, or a $C_3$-$C_7$ cycloalkyl radical,

$CONHR_8$, $R_8$ being the hydrogen atom or an alkyl radical having 1 to 6 carbon atoms, or a $C_3$-$C_7$ cycloalkyl radical,

CN,

and their addition salts in particular the pharmaceutically acceptable salts.

as well as 2-[1-(4-chlorobenzyl)-5-chloroimidazo[4,5-b]pyridin-2-yl] mercapto-2-methylpropanoic acid, and their addition salts in particular the pharmaceutically acceptable salts.

**2.** Derivatives according to claim 1, characterized in that A is a phenyl ring.

**3.** Derivatives according to claim 1, characterized in that A is a pyridine ring.

**4.** Derivatives according to one of claims 1 to 3, characterized in that $X_1$ is the fluorine atom.

**5.** Derivatives according to one of claims 1 to 3, characterized in that $X_1$ is the chlorine atom.

**6.** Derivatives according to one of claims 1 to 5, characterized in that $X_3$ is the chlorine atom.

**7.** Derivatives according to one of claims 1 to 5, characterized in that $X_3$ is the methoxy group.

**8.** Derivatives according to one of claims 1 to 5, characterized in that $X_3$ is the methylthio group.

**9.** Derivatives according to one of claims 1 to 8, characterized in that $X_4$ is the chlorine atom.

**10.** Derivatives according to one of claims 1 to 9, characterized in that D is an acid group.

**11.** Derivatives according to one of claims 1 to 10, characterized in that B is a methylene group, $R_1$ and $R_2$ are each a methyl, $R_3$ and $R_4$ are hydrogen and n is equal to 1.

**12.** Derivatives according to one of claims 1 to 10, characterized in that B is the sulfur atom.

**13.** Derivatives according to one of claims 1 to 10 or 12, characterized in that $CR_1R_2$ is a cyclopentane.

**14.** Derivatives according to any one of claims 1 to 11, characterized in that they are selected from the derivatives of formula:

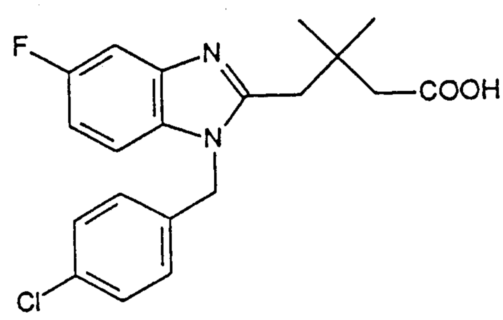

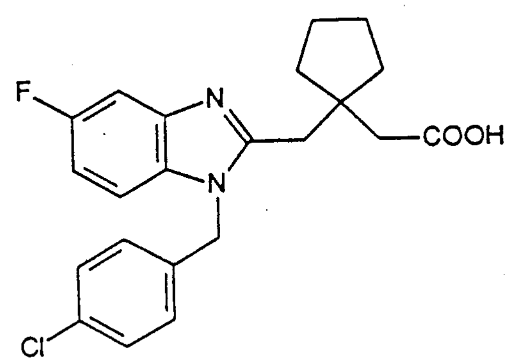

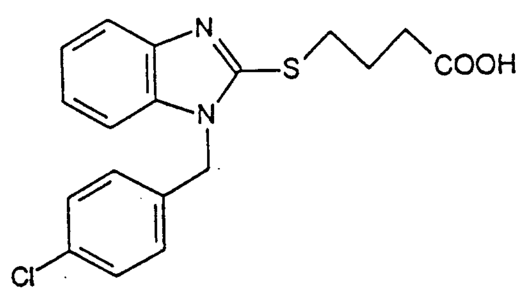

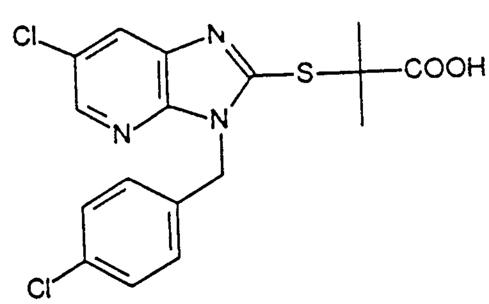

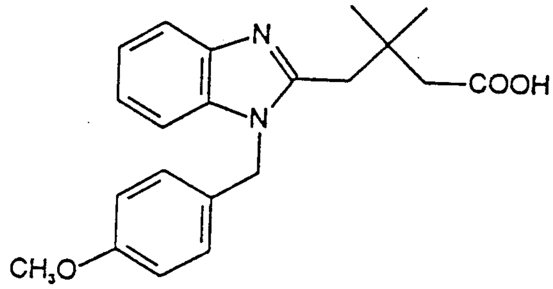

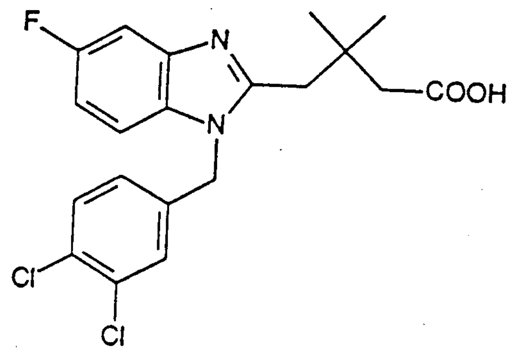

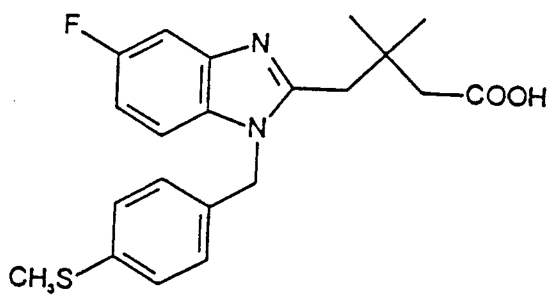

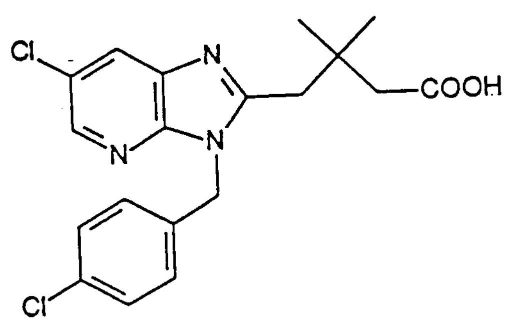

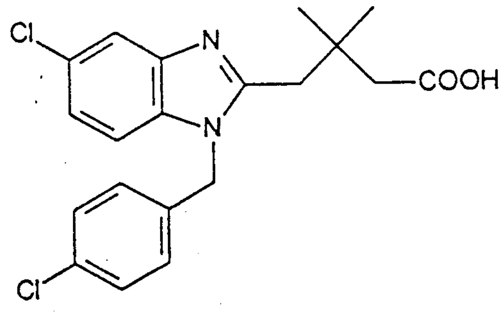

**15.** Methods of preparing the compounds of formula (I) according to any one of claims 1 to 14 characterized in that they are prepared:

A/ if B is a sulfur atom: by reacting an alkyl halogenoalkanoate with a mercaptobenzimidazole or mercaptoazabenzimidazole in the presence of a base such as a sodium or potassium alcoholate, sodium or lithium hydride or potassium carbonate in an alcohol, dimethylformamide, acetone or butan-2-one; or

B/ if B is a group $CR_5R_6$: in two steps by reacting an acid chloride of formula (VIII):

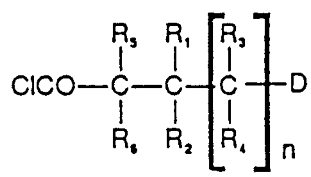

Formula (VIII)

in which $R_1$, $R_2$, $R_3$, $R_4$, $R_5$, $R_6$ and n are as defined above and D is an ester or nitrile group, with a diamine of formula (IV)

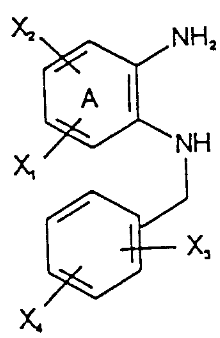

Formula (IV)

in which A, $X_1$, $X_2$, $X_3$ and $X_4$ are as defined above, this being followed in a second stage by cyclization in an acid medium,

or by reacting the diamine of formula (IV) with an aldehyde of formula (VIII'):

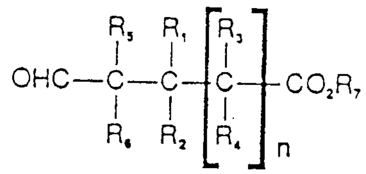

Formula (VIII')

in which $R_1$, $R_2$, $R_3$, $R_4$, $R_5$, $R_6$ and n are as defined above and R7 is an alkyl radical having 1 to 6 carbon atoms, in an acetic acid and alcohol medium, it being possible for said alcohol to be methanol, ethanol or methoxyethanol for example, the derivative obtained then being oxidized with an oxidizing agent such as iodine or barium manganate, acid or basic hydrolysis of the ester or nitrile derivatives yielding the acid derivatives.

**16.** Method of preparing a pharmaceutical composition, characterized in that a pharmaceutically effective amount of at least one compound of formula (I) as defined in any one of claims 1 to 14, or one of its pharmaceutically acceptable addition salts, is incorporated in a pharmaceutically acceptable excipient, vehicle or carrier.

**17.** Method according to claim 16, characterized in that the pharmaceutical composition is formulated as gelatin capsules or tablets containing from 1 to 200 mg of active ingredient, or as injectable preparations containing from 0.01 to 10 mg of active ingredient.

**Patentansprüche**
**Patentansprüche für folgende Vertragsstaaten : AT, BE, CH, DE, DK, FR, GB, IT, LI, LU, NL, SE**

**1.** Benzimidazolderivate, dadurch gekennzeichnet, daß sie der Formel (I) entsprechen:

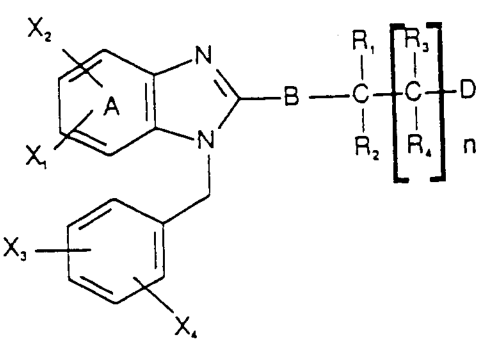

Formel (I)

worin
A ein aromatischer Kern oder ein stickstoffhaltiger Heterocyclus ist,
$X_1$, $X_2$, $X_3$ und $X_4$ unabhängig voneinander ein Wasserstoffatom, ein Halogenatom, einen Alkylrest mit 1 bis 6 Kohlenstoffatomen, einen $C_3$-$C_7$ Cycloalkylrest, einen Alkoxyrest mit 1 bis 6 Kohlenstoffatomen, einen Alkylthiorest mit 1 bis 6 Kohlenstoffatomen, eine Sulfongruppe, $SO_2$-Alkyl mit 1 bis 6 Kohlenstoffatomen, eine Sulfoxidgruppe, SO-Alkyl mit 1 bis 6 Kohlenstoffatomen, eine Trifluormethylgruppe, eine Hydroxylgruppe, eine Nitrogruppe, einen Methylenalkoholrest oder eine COOR'-Funktion, worin R' ein Wasserstoff oder ein Alkyl mit 1 bis 6 Kohlenstoffatomen ist, darstellen, wobei $X_3$ und $X_4$ gleichermaßen mit dem Phenyl ein Naphthalin bilden können,
B $CR_5R_6$ darstellt, wobei $R_5$ und $R_6$ ein Wasserstoffatom oder ein Alkylrest mit 1 bis 6 Kohlenstoffatomen oder ein $C_3$-$C_7$ Cycloalkylrest oder ein Schwefelatom sind,

$R_1$, $R_2$, $R_3$ und $R_4$ unabhängig ein Wasserstoffatom oder einen Alkylrest mit 1 bis 6 Kohlenstoffatomen oder einen $C_3$-$C_7$ Cycloalkylrest darstellen, wobei $CR_1R_2$ oder $CR_3R_4$ mit B, wenn letzteres für $CR_5R_6$ steht, ein Cycloalkyl oder ein Cycloalkylen mit 3 bis 7 Kohlenstoffatomen bilden können und $R_1R_2$, $R_3R_4$ gleichermaßen einen Cyclus mit 3 bis 7 Kohlenstoffatomen bilden können,
n eine ganze Zahl von 1 bis 4 ist und 0 sein kann, wenn $R_1$ und $R_2$ von Wasserstoff verschieden sind,
D eine chemische Funktion darstellt, die sein kann:
$COOR_7$, wobei $R_7$ ein Wasserstoffatom oder ein Alkylrest mit 1 bis 6 Kohlenstoffatomen oder ein $C_3$-$C_7$-Cycloalkylrest ist,
CONH-$R_8$, wobei $R_8$ ein Wasserstoffatom oder ein Alkylrest mit 1 bis 6 Kohlenstoffatomen oder ein $C_3$-$C_7$-Cycloalkylrest ist,
CN,
sowie 2-[1-(4-Chlorbenzyl)-5-chlorimidazo[4,5-b]pyridin-2-yl]mercapto-2-methylpropionsäure sowie deren Additionssalze, insbesondere pharmazeutisch annehmbare Additionssalze.

**2.** Derivate nach Anspruch 1, dadurch gekennzeichnet, daß A ein Phenylring ist.

**3.** Derivate nach Anspruch 1, dadurch gekennzeichnet, daß A ein Pyridinring ist.

**4.** Derivate nach einem der Ansprüche 1 bis 3, dadurch gekennzeichnet, daß $X_1$ ein Fluoratom ist.

**5.** Derivate nach einem der Ansprüche 1 bis 3, dadurch gekennzeichnet, daß $X_1$ ein Chloratom ist.

**6.** Derivate nach einem der Ansprüche 1 bis 5, dadurch gekennzeichnet, daß $X_3$ ein Chloratom ist.

**7.** Derivate nach einem der Ansprüche 1 bis 5, dadurch gekennzeichnet, daß $X_3$ eine Methoxygruppe ist.

**8.** Derivate nach einem der Ansprüche 1 bis 5, dadurch gekennzeichnet, daß $X_3$ eine Methylthiogruppe ist.

**9.** Derivate nach einem der Ansprüche 1 bis 8, dadurch gekennzeichnet, daß $X_4$ ein Chloratom ist.

**10.** Derivate nach einem der Ansprüche 1 bis 9, dadurch gekennzeichent, daß D ein Säurefunktion ist.

**11.** Derivate nach einem der Ansprüche 1 bis 10, dadurch gekennzeichnet, daß B eine Methylengruppe ist, $R_1$ und $R_2$ jeweils Methyl darstellen, $R_3$ und $R_4$ Wasserstoff und n gleich 1 ist.

**12.** Derivate nach einem der Ansprüche 1 bis 10, dadurch gekennzeichnet, daß B ein Schwefelatom ist.

**13.** Derivate nach einem der Ansprüche 1 bis 10 oder 12, dadurch gekennzeichnet, daß $CR_1R_2$ ein Cyclopentan darstellt.

**14.** Derivate nach einem beliebigen der Ansprüche 1 bis 11, dadurch gekennzeichnet, daß sie aus den Derivaten der Formel ausgewählt sind:

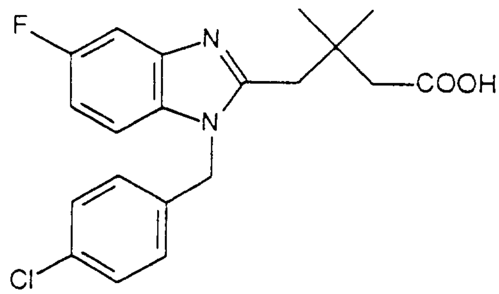

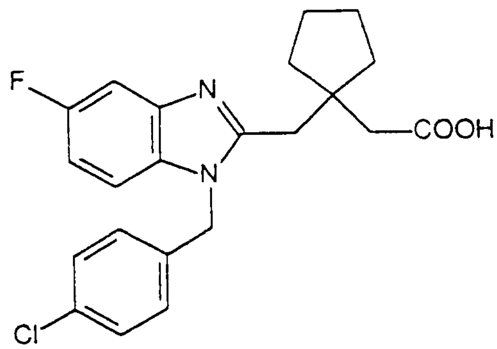

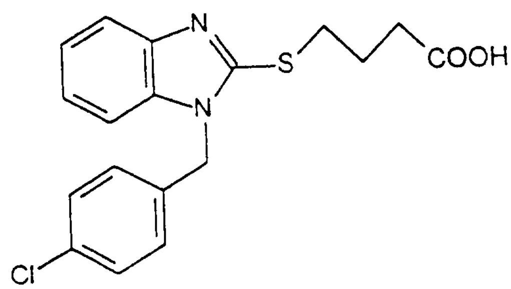

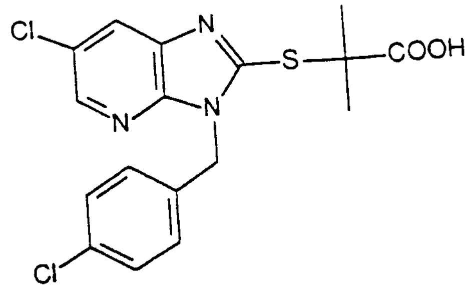

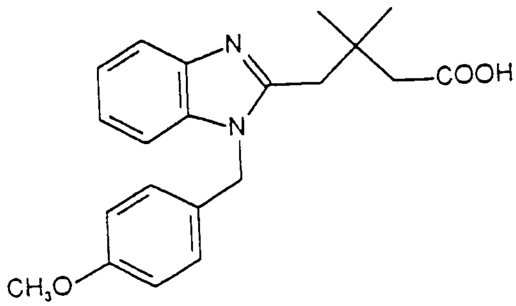

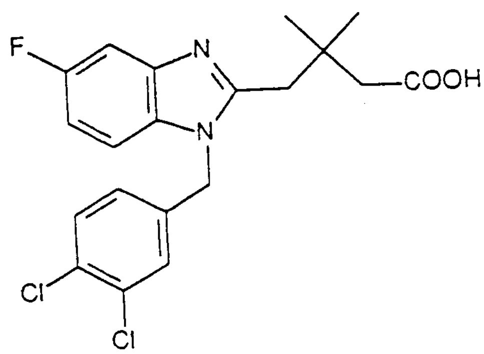

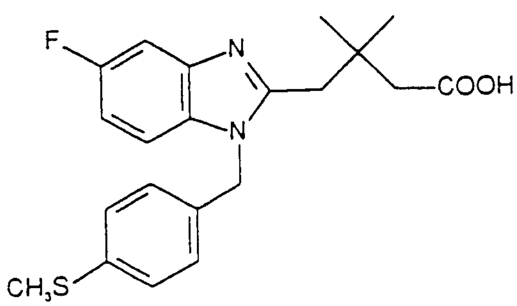

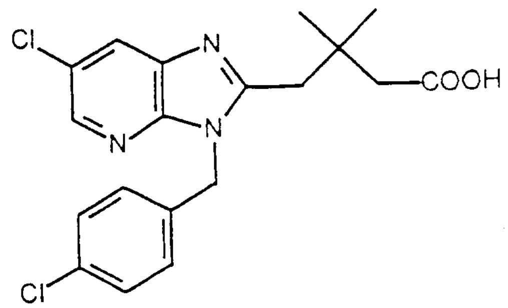

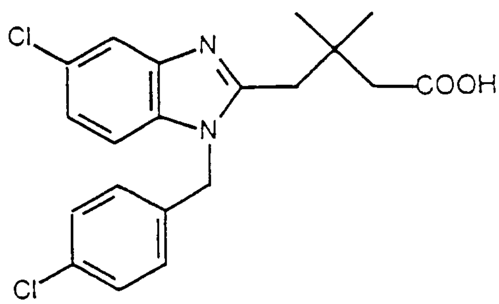

**15.** Verfahren zur Herstellung der Verbindungen der Formel (I) nach irgendeinem der Ansprüche 1 bis 14, dadurch gekennzeichnet, daß sie hergestellt sind:

A/wenn B ein Schwefelatom ist, durch Einwirken eines Alkylhalogenalkanoats auf ein Mercaptobenzimidazol oder Azabenzimidazol in Gegenwart einer Base, wie einem Natrium- oder Kaliumalkoholat, einem Natrium- oder Lithiumhydrid, Kaliumcarbonat, in einem Alkohol, Dimethylformamid, Aceton oder 2-Butanon;

B/wenn B eine $CR_5R_6$-Gruppe ist, in zwei Schritten: durch Einwirken eines Säurechlorids der Formel (VIII)

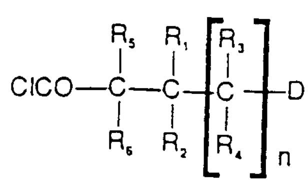

Formel (VIII)

worin $R_1$, $R_2$, $R_3$, $R_4$, $R_5$, $R_6$ und n wie vorstehend definiert sind und D eine Ester- oder Nitrilfunktion ist,

auf ein Diamin der Formel (IV)

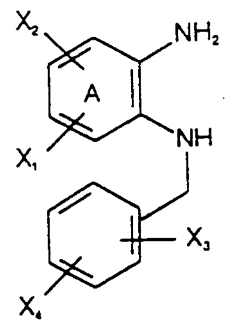

Formel (IV)

worin A, $X_1$, $X_2$, $X_3$ und $X_4$ wie vorstehend definiert sind, gefolgt in einem zweiten Schritt von der Cyclisierung in saurem Medium, oder auch durch Reaktion des Diamins der Formel (IV) mit einem Aldehyd der Formel (VIII')

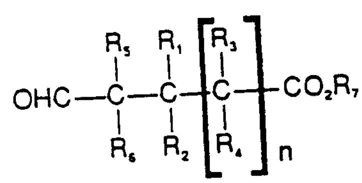

Formel (VIII')

worin $R_1$, $R_2$, $R_3$, $R_4$, $R_5$, $R_6$ und n wie vorstehend definiert sind und $R_7$ ein Alkylrest mit 1 bis 6 Kohlenstoffatomen ist, in einem essigsauren und alkoholischen Mileu, wobei letzteres beispielsweise Methanol, Ethanol oder Methoxyethanol sein kann, und danach Oxidieren des erhaltenen Derivats mit einem Oxidationsmittel, wie Jod oder Bariummanganat, wobei saure oder basische Hydrolyse der Ester- oder Nitrilderivate zu den Säurederivaten führt.

**16.** Pharmazeutische Zusammensetzung, dadurch gekennzeichnet, daß sie eine pharmazeutisch wirksame Menge wenigstens einer Verbindung der Formel (I), wie einem der Ansprüche 1 bis 14 definiert, oder ihrer pharmazeutisch annehmbaren Additionssalze, ggf. eingebracht in einen pharmazeutisch annehmbaren Excipienten, Transportstoff oder Träger, enthält.

**17.** Pharmazeutische Zusammensetzung mit antagonistischer Wirkung auf Thromboxanrezeptoren, die insbesondere die günstige Beeinflussung eines Myokardinfarkts, von Agina pectoris, zerebralem Ictus, Migräne, Hirnblutungen, Arteriosklerose, Lungenembolie, Bronchialasthma, Bronchitis, Lungenentzündung, Kreislaufschock verschiedenen Ursprungs (Blutungen, Sepsis, Kreislaufversagen, Trauma, akute Pankreatitis, Verbrennungen, bakteriellen Ursprungs), Nephritis, Abstoßungsreaktionen, Krebsmetastasen erlaubt, dadurch gekennzeichnet, daß sie eine pharmazeutisch wirksame Menge wenigstens einer Verbindung der Formel (I), wie in irgendeinem der Ansprüche 1 bis 14 definiert, oder eines ihr pharmazeutisch annehmbaren Additionssalze enthält, ggf. eingebracht in einen pharmazeutisch annehmbaren Excipienten, Transportstoff oder Träger.

**18.** Verfahren zur Herstellung einer pharmazeutischen Zusammensetzung, dadurch gekennzeichnet, daß man eine pharmazeutisch wirksame Menge wenigstens einer Verbindung der Formel (I), wie in

irgendeinem der Ansprüche 1 bis 14 definiert, oder eines ihrer pharmazeutisch annehmbaren Additionssalze, in einen pharmazeutisch annehmbaren Excipienten, Transportstoff oder Träger einbringt.

**19.** Verfahren nach Anspruch 18, dadurch gekennzeichnet, daß die pharmazeutische Zusammensetzung in Form von Gelatinekapseln, von Tabletten mit Dosen von 1 bis 200 mg oder in Form von injizierbaren Zubreitungen mit Dosen von 0,01 bis 10 mg formuliert wird.

**Patentansprüche für folgenden Vertragsstaat : ES**

**1.** Verfahren zur Herstellung von Benzimidazolderivaten, der Formel (I):

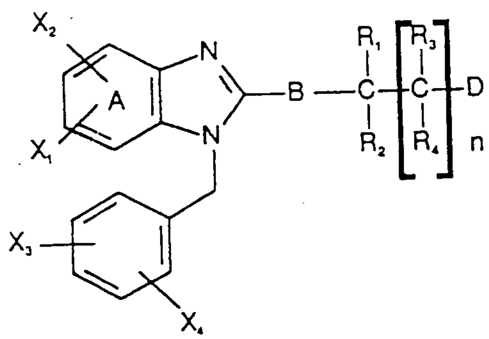

Formel (I)

worin

A ein aromatischer Kern oder ein stickstoffhaltiger Heterocyclus ist,

$X_1$, $X_2$, $X_3$ und $X_4$ unabhängig voneinander ein Wasserstoffatom, ein Halogenatom, einen Alkylrest mit 1 bis 6 Kohlenstoffatomen, einen $C_3$-$C_7$ Cycloalkylrest, einen Alkoxyrest mit 1 bis 6 Kohlenstoffatomen, einen Alkylthiorest mit 1 bis 6 Kohlenstoffatomen, eine Sulfongruppe, $SO_2$-Alkyl mit 1 bis 6 Kohlenstoffatomen, eine Sulfoxidgruppe, SO-Alkyl mit 1 bis 6 Kohlenstoffatomen, eine Trifluormethylgruppe, eine Hydroxylgruppe, eine Nitrogruppe, einen Methylenalkoholrest oder eine COOR'-Funktion, worin R' ein Wasserstoff oder ein Alkyl mit 1 bis 6 Kohlenstoffatomen ist, darstellen, wobei $X_3$ und $X_4$ gleichermaßen mit dem Phenyl ein Naphthalin bilden können,

B $CR_5R_6$ darstellt, wobei $R_5$ und $R_6$ ein Wasserstoffatom oder ein Alkylrest mit 1 bis 6 Kohlenstoffatomen oder ein $C_3$-$C_7$ Cycloalkylrest oder ein Schwefelatom sind,

$R_1$, $R_2$, $R_3$ und $R_4$ unabhängig ein Wasserstoffatom oder einen Alkylrest mit 1 bis 6 Kohlenstoffatomen oder einen $C_3$-$C_7$ Cycloalkylrest darstellen, wobei $CR_1R_2$ oder $CR_3R_4$ mit B, wenn letzteres für $CR_5R_6$ steht, ein Cycloalkyl oder ein Cycloalkylen mit 3 bis 7 Kohlenstoffatomen bilden können und $R_1R_2$, $R_3R_4$ gleichermaßen einen Cyclus mit 3 bis 7 Kohlenstoffatomen bilden können,

n eine ganze Zahl von 1 bis 4 ist und 0 sein kann, wenn $R_1$ und $R_2$ von Wasserstoff verschieden sind,

D eine chemische Funktion darstellt, die sein kann:

$COOR_7$, wobei $R_7$ ein Wasserstoffatom oder ein Alkylrest mit 1 bis 6 Kohlenstoffatomen oder ein $C_3$-$C_7$-Cycloalkylrest ist,

CONH-$R_8$, wobei $R_8$ ein Wasserstoffatom oder ein Alkylrest mit 1 bis 6 Kohlenstoffatomen oder ein $C_3$-$C_7$-Cycloalkylrest ist,

CN,

sowie 2-[1-(4-Chlorbenzyl)-5-chlorimidazo[4,5-b]pyridin-2-yl]mercapto-2-methylpropionsäure, oder deren Additionssalzen, insbesondere den pharmazeutisch annehmbaren Additionssalzen,

dadurch gekennzeichnet, daß es umfaßt:

A/ wenn B ein Schwefelatom ist, das Einwirken eines Alkylhalogenalkanoats auf ein Mercaptobenzimidazol oder Azabenzimidazol in Gegenwart einer Base, wie einem Natrium- oder Kaliumalkoholat, einem Natrium- oder Lithiumhydrid, Kaliumcarbonat, in einem Alkohol, Dimethylformamid, Aceton oder 2-Butanon;

B/wenn B eine $CR_5R_6$-Gruppe ist, in einem ersten Schritt das Einwirken eines Säurechlorids der Formel (VIII)

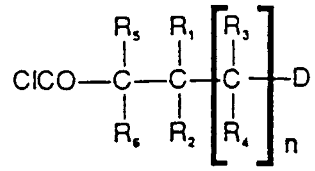

Formel (VIII)

worin $R_1$, $R_2$, $R_3$, $R_4$, $R_5$, $R_6$ und n wie vorstehend definiert sind und D eine Ester- oder Nitrilfunktion ist,
worin $R_1$, $R_2$, $R_3$, $R_4$, $R_5$, $R_6$ und n wie vorstehend definiert sind und D eine Ester- oder Nitrilfunktion ist,
auf ein Diamin der Formel (IV)

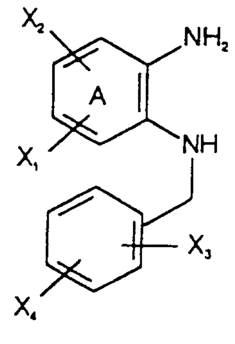

Formel (IV)

worin A, $X_1$, $X_2$, $X_3$ und $X_4$ wie vorstehend definiert sind, gefolgt in einem zweiten Schritt von der Cyclisierung in saurem Medium,
oder auch durch Reaktion des Diamins der Formel (IV) mit einem Aldehyd der Formel (VIII')

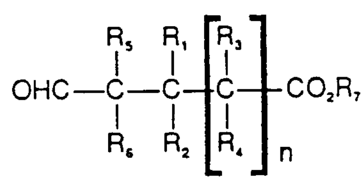

Formel (VIII')

worin $R_1$, $R_2$, $R_3$, $R_4$, $R_5$, $R_6$ und n wie vorstehend definiert sind und $R_7$ ein Alkylrest mit 1 bis 6 Kohlenstoffatomen ist, in einem essigsauren und alkoholischen Mileu, wobei letzteres beispielsweise Methanol, Ethanol oder Methoxyethanol sein kann, und danach Oxidieren des erhaltenen Derivats mit einem Oxidationsmittel, wie Jod oder Bariummanganat, wobei saure oder basische Hydrolyse der Ester- oder Nitrilderivate zu den Säurederivaten führt;
und ggfs. Umwandlung des so erhaltenen Produkts in ein Additionssalz, insbesondere ein pharmazeutisch annehmbares Additionssalz.

**2.** Herstellungsverfahren nach Anspruch 1 der Verbindungen von Formel (I), worin A ein Phenylring ist.

3. Herstellungsverfahren nach Anspruch 1 der Verbindungen von Formel (I), worin A ein Pyridinring ist.

4. Herstellungsverfahren nach einem der Ansprüche 1 bis 3 von Verbindungen der Formel (I), worin $X_1$ ein Fluoratom ist.

5. Herstellungsverfahren nach einem der Ansprüche 1 bis 3 von Verbindungen der Formel (I), worin $X_1$ ein Chloratom ist.

6. Herstellungsverfahren nach einem der Ansprüche 1 bis 5 von Verbindungen der Formel (I), worin $X_3$ ein Chloratom ist.

7. Herstellungsverfahren nach einem der Ansprüche 1 bis 5 von Verbindungen der Formel (I), worin $X_3$ eine Methoxygruppe ist.

8. Herstellungsverfahren nach einem der Ansprüche 1 bis 5 von Verbindungen der Formel (I), worin $X_3$ eine Methylthiogruppe ist.

9. Herstellungsverfahren nach einem der Ansprüche 1 bis 8 von Verbindungen der Formel (I), worin $X_4$ ein Chloratom ist.

10. Herstellungsverfahren nach einem der Ansprüche 1 bis 9 von Verbindungen der Formel (I), worin D eine Säurefunktion ist.

11. Herstellungsverfahren nach einem der Ansprüche 1 bis 10 von Verbindungen der Formel (I), worin B eine Methylengruppe ist, $R_1$ und $R_2$ jeweils Methyl darstellen, $R_3$ und $R_4$ Wasserstoff und n gleich 1 ist.

12. Herstellungsverfahren nach einem der Ansprüche 1 bis 10 von Verbindungen der Formel (I), worin B ein Schwefelatom ist.

13. Herstellungsverfahren nach einem der Ansprüche 1 bis 10 oder 12 von Verbindungen der Formel(I), worin $CR_1R_2$ ein Cyclopentan darstellt.

14. Herstellungsverfahren nach einem beliebigen der Ansprüche 1 bis 11 von Verbindungen, die aus Derivaten der Formeln ausgewählt sind:

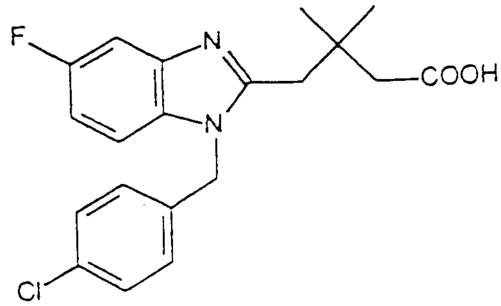

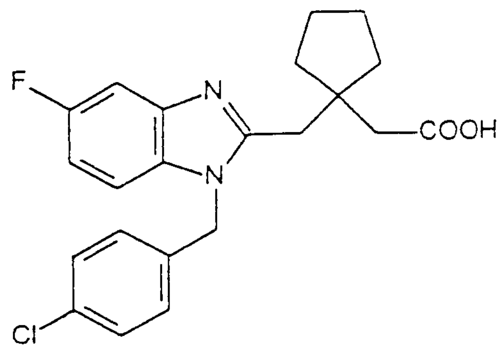

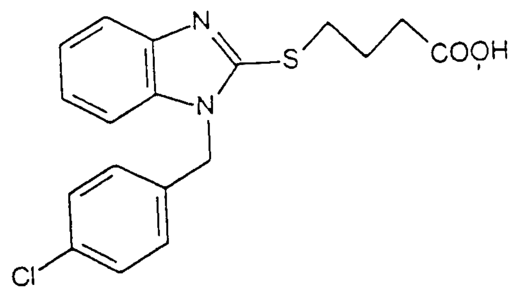

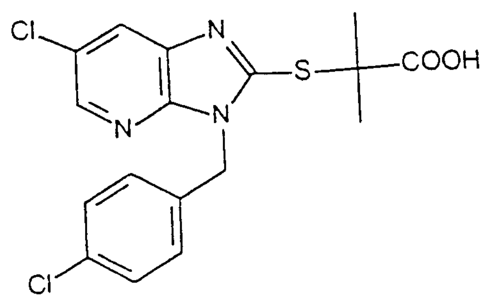

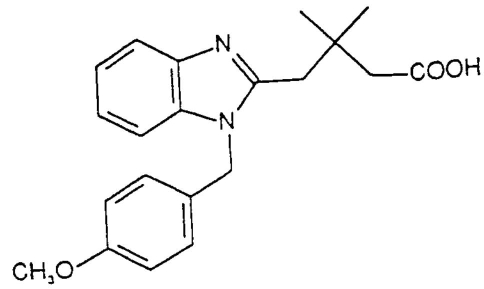

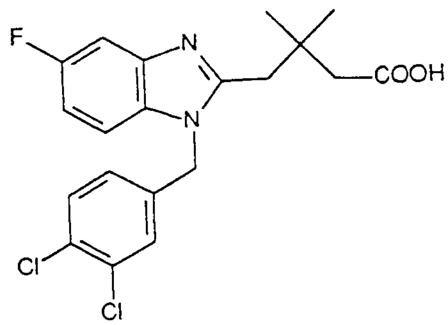

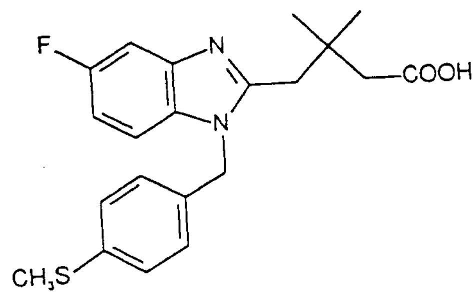

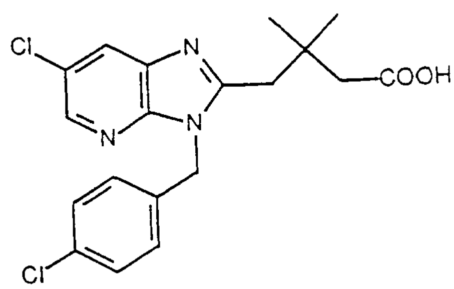

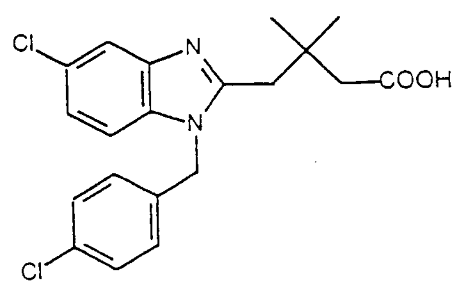

**15.** Verfahren zur Herstellung einer pharmazeutischen Zusammensetzung, dadurch gekennzeichnet, daß man eine pharmazeutisch wirksame Menge wenigstens einer Verbindung der Formel (I), hergestellt nach einem der Ansprüche 1 bis 14, oder eines ihrer pharmazeutisch annehmbaren Additiossalze, in einen pharmazeutisch annehmbaren Excipienten, Transportstoff oder Träger einbringt.

**16.** Verfahren nach Anspruch 15, dadurch gekennzeichnet, daß die pharmazeutische Zusammensetzung in Form von Gelatinekapsein, von Tabletten mit Dosen von 1 bis 200 mg oder in Form von injizierbaren Zubereitungen mit Dosen von 0,01 bis 10 mg formuliert wird.

**Patentansprüche für folgenden Vertragsstaat : GR**

**1.** Benzimidazolderivate, dadurch gekennzeichnet, daß sie der Formel (I) entsprechen:

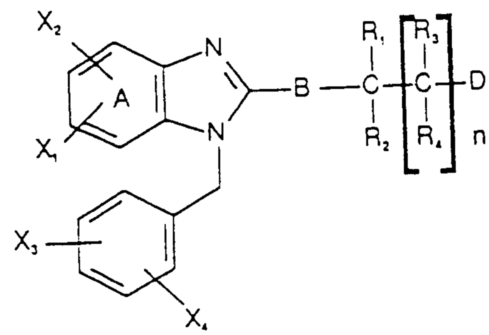

Formel (I)

worin
A ein aromatischer Kern oder ein stickstoffhaltiger Heterocyclus ist,
$X_1$, $X_2$, $X_3$ und $X_4$ unabhängig voneinander ein Wasserstoffatom, ein Halogenatom, einen Alkylrest mit 1 bis 6 Kohlenstoffatomen, einen $C_3$-$C_7$ Cycloalkylrest, einen Alkoxyrest mit 1 bis 6 Kohlenstoffatomen, einen Alkylthiorest mit 1 bis 6 Kohlenstoffatomen, eine Sulfongruppe, $SO_2$-Alkyl mit 1 bis 6 Kohlenstoffatomen, eine Sulfoxidgruppe, SO-Alkyl mit 1 bis 6 Kohlenstoffatomen, eine Trifluormethylgruppe, eine Hydroxylgruppe, eine Nitrogruppe, einen Methylenalkoholrest oder eine COOR'-Funktion, worin R' ein Wasserstoff oder ein Alkyl mit 1 bis 6 Kohlenstoffatomen ist, darstellen, wobei $X_3$ und $X_4$ gleichermaßen mit dem Phenyl ein Naphthalin bilden können,
B $CR_5R_6$ darstellt, wobei $R_5$ und $R_6$ ein Wasserstoffatom oder ein Alkylrest mit 1 bis 6 Kohlenstoffatomen oder ein $C_3$-$C_7$ Cycloalkylrest oder ein Schwefelatom sind,
$R_1$, $R_2$, $R_3$ und $R_4$ unabhängig ein Wasserstoffatom oder einen Alkylrest mit 1 bis 6 Kohlenstoffatomen oder einen $C_3$-$C_7$ Cycloalkylrest darstellen, wobei $CR_1R_2$ oder $CR_3R_4$ mit B, wenn letzteres für $CR_5R_6$ steht, ein Cycloalkyl oder ein Cycloalkylen mit 3 bis 7 Kohlenstoffatomen bilden können und $R_1R_2$, $R_3R_4$ gleichermaßen einen Cyclus mit 3 bis 7 Kohlenstoffatomen bilden können,
n eine ganze Zahl von 1 bis 4 ist und 0 sein kann, wenn $R_1$ und $R_2$ von Wasserstoff verschieden sind,
D eine chemische Funktion darstellt, die sein kann:
$COOR_7$, wobei $R_7$ ein Wasserstoffatom oder ein Alkylrest mit 1 bis 6 Kohlenstoffatomen oder ein $C_3$-$C_7$-Cycloalkylrest ist,
CONH-$R_8$, wobei $R_8$ ein Wasserstoffatom oder ein Alkylrest mit 1 bis 6 Kohlenstoffatomen oder ein $C_3$-$C_7$-Cycloalkylrest ist,
CN,
sowie 2-[1-(4-Chlorbenzyl)-5-chlorimidazo[4,5-b]pyridin-2-yl]mercapto-2-methylpropionsäure sowie deren Additionssalze, insbesondere pharmazeutisch annehmbare Additionssalze.

**2.** Derivate nach Anspruch 1, dadurch gekennzeichnet, daß A ein Phenylring ist.

**3.** Derivate nach Anspruch 1, dadurch gekennzeichnet, daß A ein Pyridinring ist.

**4.** Derivate nach einem der Ansprüche 1 bis 3, dadurch gekennzeichnet, daß $X_1$ ein Fluoratom ist.

**5.** Derivate nach einem der Ansprüche 1 bis 3, dadurch gekennzeichnet, daß $X_1$ ein Chloratom ist.

**6.** Derivate nach einem der Ansprüche 1 bis 5, dadurch gekennzeichnet, daß $X_3$ ein Chloratom ist.

7. Derivate nach einem der Ansprüche 1 bis 5, dadurch gekennzeichnet, daß $X_3$ eine Methoxygruppe ist.

8. Derivate nach einem der Ansprüche 1 bis 5, dadurch gekennzeichnet, daß $X_3$ eine Methylthiogruppe ist.

9. Derivate nach einem der Ansprüche 1 bis 8, dadurch gekennzeichnet, daß $X_4$ ein Chloratom ist.

10. Derivate nach einem der Ansprüche 1 bis 9, dadurch gekennzeichent, daß D ein Säurefunktion ist.

11. Derivate nach einem der Ansprüche 1 bis 10, dadurch gekennzeichnet, daß B eine Methylengruppe ist, $R_1$ und $R_2$ jeweils Methyl darstellen, $R_3$ und $R_4$ Wasserstoff und n gleich 1 ist.

12. Derivate nach einem der Ansprüche 1 bis 10, dadurch gekennzeichnet, daß B ein Schwefelatom ist.

13. Derivate nach einem der Ansprüche 1 bis 10 oder 12, dadurch gekennzeichnet, daß $CR_1R_2$ ein Cyclopentan darstellt.

14. Derivate nach einem beliebigen der Ansprüche 1 bis 11, dadurch gekennzeichnet, daß sie aus den Derivaten der Formel ausgewählt sind:

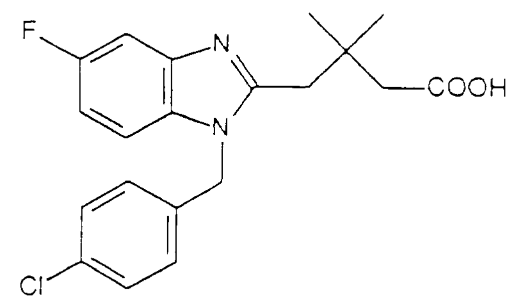

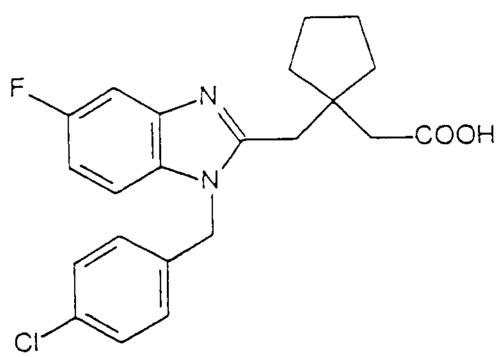

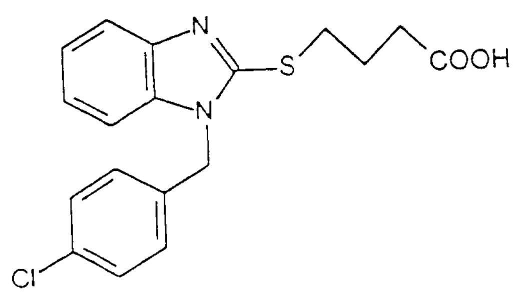

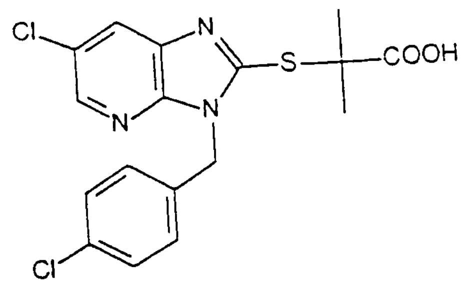

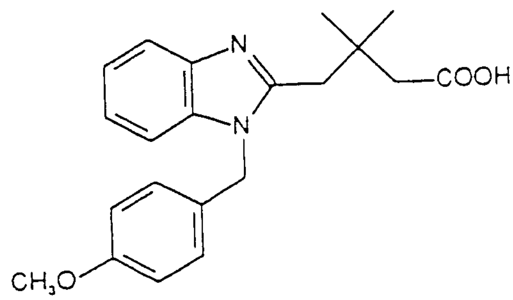

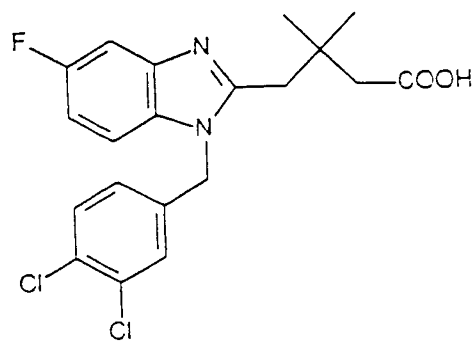

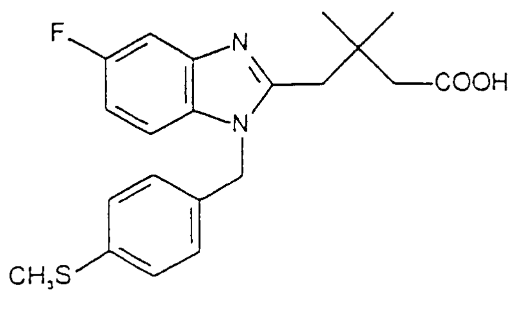

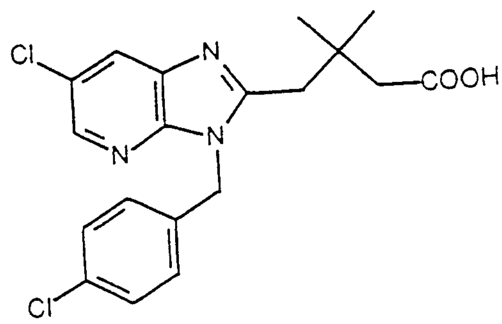

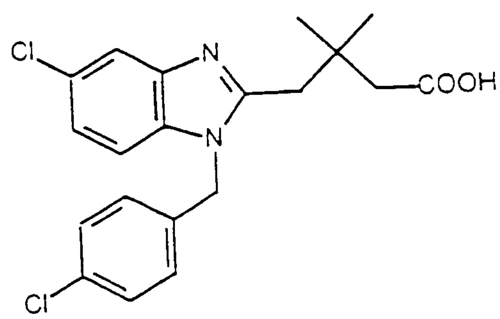

**15.** Verfahren zur Herstellung der Verbindungen der Formel (I) nach irgendeinem der Ansprüche 1 bis 14, dadurch gekennzeichnet, daß sie hergestellt sind:

A/wenn B ein Schwefelatom ist, durch Einwirken eines Alkylhalogenalkanoats auf ein Mercaptobenzimidazol oder Azabenzimidazol in Gegenwart einer Base, wie einem Natrium- oder Kaliumalkoholat, einem Natrium- oder Lithiumhydrid, Kaliumcarbonat, in einem Alkohol, Dimethylformamid, Aceton oder 2-Butanon;

B/wenn B eine $CR_5R_6$-Gruppe ist, in zwei Schritten: durch Einwirken eines Säurechlorids der Formel (VIII)

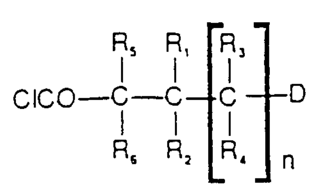

Formel (VIII)

worin $R_1$, $R_2$, $R_3$, $R_4$, $R_5$, $R_6$ und n wie vorstehend definiert sind und D eine Ester- oder Nitrilfunktion ist,

auf ein Diamin der Formel (IV)

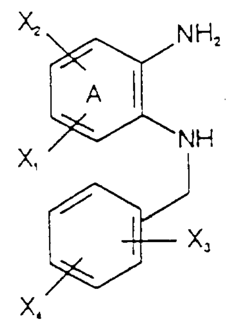

Formel (IV)

worin A, $X_1$, $X_2$, $X_3$ und $X_4$ wie vorstehend definiert sind, gefolgt in einem zweiten Schritt von der Cyclisierung in saurem Medium, oder auch durch Reaktion des Diamins der Formel (IV) mit einem Aldehyd der Formel (VIII')

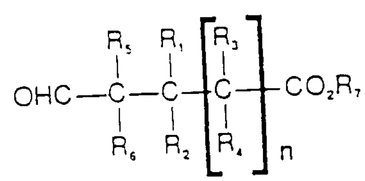

Formel (VIII')

worin $R_1$, $R_2$, $R_3$, $R_4$, $R_5$, $R_6$ und n wie vorstehend definiert sind und $R_7$ ein Alkylrest mit 1 bis 6 Kohlenstoffatomen ist, in einem essigsauren und alkoholischen Mileu, wobei letzteres beispielsweise Methanol, Ethanol oder Methoxyethanol sein kann, und danach Oxidieren des erhaltenen Derivats mit einem Oxidationsmittel, wie Jod oder Bariummanganat, wobei saure oder basische Hydrolyse der Ester- oder Nitrilderivate zu den Säurederivaten führt.

**16.** Verfahren zur Herstellung einer pharmazeutischen Zusammensetzung, dadurch gekennzeichnet, daß man eine pharmazeutisch wirksame Menge wenigstens einer Verbindung der Formel (I), wie in einem der Ansprüche 1 bis 14 definiert, oder eines ihrer pharmazeutisch annehmbaren Additionssalze, in einen pharmazeutisch annehmbaren Excipienten, Transportstoff oder Träger, einbringt.

**17.** Verfahren nach Anspruch 16, dadurch gekennzeichnet, daß die pharmazeutische Zusammensetzung in Form von Gelatinekapseln, von Tabletten mit Dosen von 1 bis 200 mg oder in Form von injizierbaren Zubereitungen mit Dosen von 0,01 bis 10 mg formuliert wird.